(19) **Europäisches Patentamt**
**European Patent Office**
**Office européen des brevets**

(11) **EP 4 710 885 A1**

(12) **EUROPEAN PATENT APPLICATION**
published in accordance with Art. 153(4) EPC

(43) Date of publication:
**18.03.2026 Bulletin 2026/12**

(21) Application number: **24822751.4**

(22) Date of filing: **13.06.2024**

(51) International Patent Classification (IPC):
**A61B 34/20** (2016.01)   **A61B 17/34** (2006.01)

(52) Cooperative Patent Classification (CPC):
**A61B 17/34; A61B 34/20**

(86) International application number:
**PCT/CN2024/099054**

(87) International publication number:
**WO 2024/255803 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **14.06.2023   CN 202310707754
31.07.2023   CN 202310964477
27.10.2023   CN 202311421935**

(71) Applicant: **Wuhan United Imaging Surgical Co.,
Ltd.**
**Wuhan, Hubei 430073 (CN)**

(72) Inventors:
• **HE, Shaowen**
**Wuhan, Hubei 430073 (CN)**

• **ZHAN, Xuzheng**
**Wuhan, Hubei 430073 (CN)**
• **YANG, Jie**
**Wuhan, Hubei 430073 (CN)**
• **ZHANG, Jing**
**Wuhan, Hubei 430073 (CN)**
• **YANG, Fei**
**Wuhan, Hubei 430073 (CN)**
• **SONG, Weifan**
**Wuhan, Hubei 430073 (CN)**
• **SHENG, Menghan**
**Wuhan, Hubei 430073 (CN)**
• **WAN, Mengyu**
**Wuhan, Hubei 430073 (CN)**

(74) Representative: **Wang, Bo
Panovision IP
Ebersberger Straße 3
85570 Markt Schwaben (DE)**

(54) **PUNCTURE ASSISTANCE SYSTEM AND DEVICE, AND CORRECTION SYSTEM AND
SURGICAL ROBOT**

(57)   Provided is a puncture assistance system, a puncture assistance device, a puncture needle position correction system, and a surgical robot. The system includes a processor configured to: obtain a scan image of a target object; the scan image including at least one of a preoperative image, a first image from a first time period, and a second image from a second time period; the first time period is earlier than the second time period; generate puncture guidance information based on puncture status information and the scan image; guide a puncture operation based on the puncture guidance information; and display the puncture guidance information.

EP 4 710 885 A1

S401

| Target organ segmentation | Tumor site extraction | Dangerous tissue identification |

Perform preoperative image scanning.

Perform initial puncture path planning. — S402

Perform guiding and positioning. — S403

Perform puncture preparing. — S404

S405

| Needle tract posture adjustment | Needle tract insertion | Real-time exposure |

Perform intraoperative puncture.

Complete puncture. — S406

**FIG. 4**

## Description

**CROSS-REFERENCE TO RELATED APPLICATIONS**

This application claims priority to the Chinese Patent Application No. 202310964477.0, filed on July 31, 2023, the Chinese Patent Application No. 202310707754.X, filed on June 14, 2023, the Chinese Patent Application No. 202311421935.2, filed on October 27, 2023, the contents of each of which are hereby incorporated by reference.

**TECHNICAL FIELD**

The present disclosure relates to the field of medical technology, and in particular to a puncture assistance system and device, a puncture needle position correction system, and a surgical robot.

**BACKGROUND**

Percutaneous puncture of a lesion or soft tissue with a surgical needle is a very important portion of modern clinical medicine and has attracted the attention of many researchers in recent years. Similar minimally invasive techniques are utilized in numerous clinical applications, such as biopsies, injections, neurosurgery, cancer treatment procedures, etc.

Current puncture surgery solutions mainly include manual blind puncture, real-time image-guided puncture, master-slave real-time guided puncture, etc. The master-slave real-time guided puncture refers to a solution that a physician, located outside the operating room (i.e., the computed tomography (CT) shielding room), controls a surgical robot to perform remote puncture procedures and operates a CT device to perform real-time image scanning and display of images of a patient. Using this solution can prevent the doctor from being harmed by radiation during the surgery.

However, since the current master-slave real-time guided puncture solution relies too much on the subjective medical experience of the physician, it is prone to causing the problem of inaccurate puncture, thereby prolonging the surgery time. Moreover, multiple posture adjustments, scans, and punctures also increase the pain of the patient and the radiation dose.

Therefore, it is necessary to provide a puncture assistance system, a puncture assistance device, a puncture needle position correction system, and a surgical robot, to reduce the radiation dose during surgery, shorten the imaging time, and improve the puncture accuracy.

**SUMMARY**

One or more embodiments of the present disclosure provide a puncture assistance system, the system includes a processor configured to: obtain a scan image of a target object; the scan image including at least one of a preoperative image, a first image from a first time period, and a second image from a second time period; the first time period is earlier than the second time period; generate puncture guidance information based on puncture status information and the scan image; guide a puncture operation based on the puncture guidance information; and display the puncture guidance information.

One or more embodiments of the present disclosure provide a puncture needle position correction system, and the system includes a positioning correction module configured to: determine first position information of a puncture needle in an image coordinate system based on positioning position information of the puncture needle in a robot coordinate system, the first position information including a needle tip position and a needle tail position of the puncture needle; identify the needle tip position in a puncture image based on the first position information to obtain second position information of the puncture needle in the puncture image, the puncture image including a second image and/or a target guidance image; verify the first position information based on the second position information to obtain a verification result; and determine target position information based on the verification result, the target position information including position information of a first needle tip point.

One or more embodiments of the present disclosure provide a puncture assistance device. The puncture assistance device includes: a scanning module, a puncture assistance module, and a display module. The scanning module is configured to obtain a scan image of a target object, the scan image including at least one of a preoperative image, a first image from a first time period, and a second image from a second time period, and the first time period is earlier than the second time period. The puncture assistance module is configured to generate puncture guidance information based on puncture status information and the scan image, and guide a puncture operation based on the puncture guidance information, and the display module is configured to display the puncture guidance information.

One or more embodiments of the present disclosure provide a surgical robot, and the surgical robot includes: a surgical robot master hand, a first processor, a surgical robot slave hand, a puncture needle being disposed on the surgical robot slave hand. The surgical robot master hand is communicatively connected to a puncture assistance device. The surgical robot master hand is configured to, after receiving a first puncture operation instruction, send the first puncture operation instruction to the first processor, and send a movement instruction for performing a puncture operation to the puncture assistance device. The first processor is configured to, after receiving the first puncture operation instruction, send a fifth movement instruction corresponding to the first puncture operation instruction to the surgical robot slave hand. The puncture assistance device is configured to, after receiving the movement instruction, send a scan instruction to a medical imaging device to cause the medical imaging device to perform medical imaging scanning on a target object. And the surgical robot slave hand is configured to move the puncture needle according to the fifth movement instruction to perform a puncture operation corresponding to the first puncture operation instruction, the puncture operation including a posture adjustment operation or a needle insertion operation, the posture adjustment operation being used to adjust a posture of the puncture needle, the needle insertion operation being used to adjust a puncture depth of the puncture needle.

**BRIEF DESCRIPTION OF THE DRAWINGS**

The present disclosure is further described by way of exemplary embodiments. These exemplary embodiments are described in detail with reference to the accompanying drawings. These embodiments are not limiting. In these embodiments, the same reference numerals denote the same structures.

FIG. 1 is a schematic diagram illustrating an exemplary puncture assistance system according to some embodiments of the present disclosure;

FIG. 2 is a schematic diagram illustrating an exemplary puncture assistance device according to some embodiments of the present disclosure;

FIG. 3 is an exemplary diagram illustrating an interaction view of a puncture method according to some embodiments of the present disclosure;

FIG. 4 is an exemplary schematic diagram illustrating a puncture process according to some embodiments of the present disclosure;

FIG. 5 is an exemplary schematic diagram illustrating a tissue segmentation obtained during a preoperative image scanning stage according to some embodiments of the present disclosure;

FIG. 6 is an exemplary schematic diagram illustrating an initial puncture path displayed on an image obtained by a scanning device according to some embodiments of the present disclosure;

FIG. 7 is an exemplary schematic diagram illustrating an extracted needle tract contour according to some embodiments of the present disclosure;

FIG. 8 is a schematic diagram illustrating an interface where a needle tip point and a predicted target point are located in a same target image according to some embodiments of the present disclosure;

FIG. 9 is a schematic diagram illustrating an interface where a needle tip point and a predicted target point are located in different target images according to some embodiments of the present disclosure;

FIG. 10 is a schematic diagram illustrating an interface of same-slice posture adjustment according to some embodiments of the present disclosure;

FIG. 11 is a schematic diagram illustrating an interface for cross-slice posture adjustment according to some embodiments of the present disclosure;

FIG. 12 is an exemplary flowchart illustrating a process of determining a target image according to some embodiments of the present disclosure;

FIG. 13 is an exemplary schematic diagram illustrating a target respiratory amplitude point according to some embodiments of the present disclosure;

FIG. 14 is an exemplary schematic diagram illustrating a target respiratory amplitude range according to some embodiments of the present disclosure;

FIG. 15 is an exemplary schematic diagram illustrating real-time image updating according to some embodiments of the present disclosure;

FIG. 16 is an exemplary schematic diagram illustrating an ideal respiratory amplitude point of a target object during a surgical procedure according to some embodiments of the present disclosure;

FIG. 17 is an exemplary flowchart illustrating a process of determining a first guidance image according to some embodiments of the present disclosure;

FIG. 18 is an exemplary flowchart illustrating a process of determining an updated target guidance image according to some embodiments of the present disclosure;

FIG. 19 is an exemplary schematic diagram illustrating a puncture needle in a second image according to some embodiments of the present disclosure;

FIG. 20 is an exemplary schematic diagram illustrating a first guidance image according to some embodiments of the present disclosure;

FIG. 21 is an exemplary schematic diagram illustrating a second guidance image according to some embodiments of the present disclosure;

FIG. 22 is an exemplary flowchart illustrating a process of a puncture needle position correction according to some embodiments of the present disclosure;

FIG. 23 is an exemplary flowchart illustrating a process of a puncture needle position correction according to another embodiment of the present disclosure;

FIG. 24 is an exemplary flowchart illustrating a process of a puncture needle position correction according to another embodiment of the present disclosure;

FIG. 25 is an exemplary flowchart illustrating a process of a puncture needle position correction according to another embodiment of the present disclosure;

FIG. 26 is an exemplary flowchart illustrating a process of a puncture needle position correction according to another embodiment of the present disclosure;

FIG. 27 is an exemplary flowchart illustrating a process of a puncture needle position correction according to another embodiment of the present disclosure;

FIG. 28 is a schematic diagram illustrating another exemplary puncture assistance device according to some other embodiments of the present disclosure;

FIG. 29 is a schematic diagram illustrating an exemplary surgical robot according to some embodiments of the present disclosure;

FIG. 30 is a schematic diagram illustrating an exemplary puncture needle position correction system according to some embodiments of the present disclosure;

FIG. 31 is a schematic diagram illustrating an exemplary puncture needle position correction device according to some embodiments of the present disclosure;

FIG. 32 is a schematic diagram illustrating another exemplary puncture needle position correction device according to some other embodiments of the present disclosure;

FIG. 33 is a schematic diagram illustrating another exemplary puncture needle position correction device according to some other embodiments of the present disclosure;

FIG. 34 is a schematic diagram illustrating another exemplary a puncture needle position correction device according to some other embodiments of the present disclosure; and

FIG. 35 is an internal diagram illustrating an exemplary computer device according to some embodiments of the present disclosure.

## DETAILED DESCRIPTION

To more clearly illustrate the technical solutions in the embodiments of the present disclosure, the accompanying drawings used in the description of the embodiments will be briefly introduced below. Obviously, the accompanying drawings in the following description are merely some examples or embodiments of the present disclosure. For those of ordinary skill in the art, the present disclosure can be applied to other similar scenarios based on these accompanying drawings without creative efforts. Unless obviously obtained from the context or the context illustrates otherwise, the same numeral in the drawings refers to the same structure or operation.

It should be understood that the terms "system," "device," "unit," and/or "module" used herein are methods for distinguishing components, elements, parts, sections, or assemblies of different levels. However, if other words can achieve the same purpose, the words can be replaced by other expressions.

Unless the context clearly indicates an exception, the terms "a," "an," "one," and/or "the" are not limited to the singular and may include the plural. Generally, the terms "include" and "comprise" only indicate that clearly identified steps and elements are included, and these steps and elements do not constitute an exclusive list. A method or device may also include other steps or elements.

The present disclosure uses flowcharts to illustrate operations performed by a system according to embodiments of the present disclosure. It should be understood that preceding or following operations are not necessarily performed precisely in order. Instead, various steps can be processed in reverse order or simultaneously. Meanwhile, other operations can be added to these processes, or one or more operations can be removed from these processes.

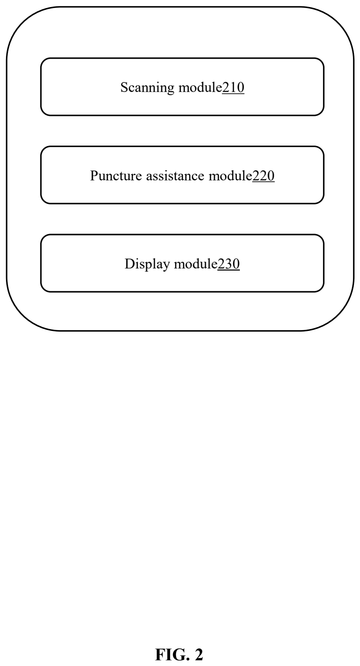

In some embodiments of the present disclosure, a puncture assistance system, the puncture assistance system includes a processor configured to: obtain a scan image of a target object; the scan image including at least one of a preoperative image, a first image from a first time period, and a second image from a second time period; the first time period is earlier than the second time period; generate puncture guidance information based on puncture status information and the scan image; guide a puncture operation based on the puncture guidance information; and display the puncture guidance information. More descriptions regarding the puncture assistance system may be found in FIG. 2 and the related descriptions. The processor involved in various embodiments provided in the present disclosure may be a general-purpose processor, a central processing unit, a graphics processing unit, a digital signal processor, a programmable logic device, a data processing logic device based on quantum computing, etc., and is not limited thereto.

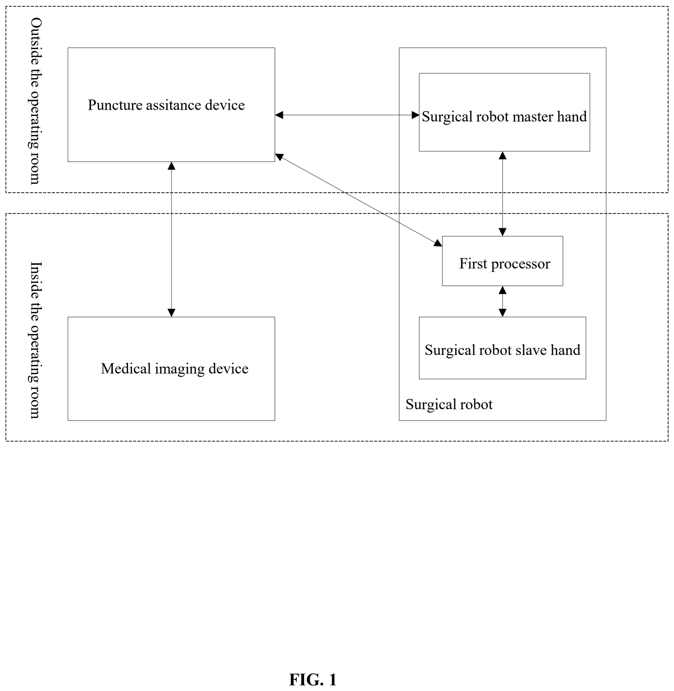

FIG. 1 is a schematic diagram illustrating an exemplary puncture assistance system according to some embodiments of the present disclosure.

As shown in FIG. 1, the puncture assistance system of the embodiments of the present disclosure includes a puncture assistance device, a surgical robot, and a medical imaging device. The puncture assistance device is communicatively connected to the surgical robot and the medical imaging device, respectively.

In some embodiments, the puncture assistance device may be configured to: obtain a scan image of a target object; generate puncture guidance information based on puncture status information and the scan image; guide a puncture operation based on the puncture guidance information; and display the puncture guidance information. In some embodiments, the puncture assistance device may include a scanning module 210, a puncture assistance module 220, and a display module 230. More descriptions regarding the puncture assistance device may be found in FIG. 2 and the related descriptions.

In some embodiments, the surgical robot is configured to perform the puncture operation. For example, the surgical robot is configured to send the puncture status information of a puncture needle in the surgical robot to the puncture assistance device.

In the puncture assistance system of the embodiments of the present disclosure, an annotated image is generated by annotating a needle tip point and a predicted target point onto a target image, and the annotated image is displayed. This enables a doctor to clearly understand a position of the needle tip point of the puncture needle and a position that the needle tip point is able to reach (i.e., a position of the predicted target point), and assists the doctor in judging the needle tip point of the puncture needle and a puncture point that the puncture needle is able to reach, thereby reducing reliance on experiences of the doctor and improving the accuracy of the puncture.

As shown in FIG. 1, the surgical robot in the embodiments of the present disclosure may include a surgical robot master hand, a first processor, and a surgical robot slave hand being communicatively connected in sequence. It can be understood that in the surgical robot, the surgical robot master hand is an operation end, and the surgical robot slave hand is an execution end. A user remotely controls the surgical robot slave hand to perform the puncture operation by operating the surgical robot master hand. In some embodiments, the puncture needle is disposed on the surgical robot slave hand. The surgical robot master hand is communicatively connected to the puncture assistance device.

As shown in FIG. 1, to protect the doctor from the effects of radiation during medical imaging scanning, the puncture assistance system may include two portions: a portion outside an operating room and a portion inside the operating room. The puncture assistance device and the surgical robot master hand, which are operated by the doctor, are disposed outside the operating room, a medical imaging device for scanning the target object and the surgical robot slave hand for puncturing the target object are disposed inside the operating room.

In some embodiments, the surgical robot slave hand may include a robotic arm, a puncture tip connected to an end of the robotic arm, and the puncture needle installed on the puncture tip. During the puncture operation, the robotic arm of the surgical robot slave hand drives the puncture needle to move through the puncture tip, thereby implementing the puncture operation of the puncture needle on the target object. Generally, the puncture operation of the puncture needle may include a posture adjustment operation and a needle insertion operation. The posture adjustment operation includes adjusting a posture of the puncture needle, and the needle insertion operation includes adjusting a puncture depth of the puncture needle.

For example, the first processor is configured to resolve an operation instruction applied by the doctor to the surgical robot master hand into a movement instruction executable by the surgical robot slave hand, and send the movement instruction to the surgical robot slave hand, the surgical robot slave hand executes the movement instruction to drive the puncture needle to move, thereby completing the puncture operation.

It is able to be understood that in the embodiment shown in FIG. 1, the first processor is disposed inside the operating room. In other embodiments, the first processor may also be disposed outside the operating room. Alternatively, the first processor may also be integrated with the surgical robot slave hand or the surgical robot master hand. The specific disposition position and form of the first processor in the present disclosure are not specifically limited.

It is able to be understood that the communicative connection in the embodiments of the present disclosure may be a wireless communicative connection. For example, the wireless communicative connection may be implemented through wireless communication techniques such as a Bluetooth (BT) technique, a Wireless-Fidelity (WiFi) technique, or a Near Field Communication (NFC) technique, etc. Certainly, the communicative connection in the embodiments of the present disclosure may also be a wired communicative connection. The present disclosure does not limit or elaborate on this.

The medical imaging device may be configured to perform a scanning operation on the target object to obtain the scan image.

The medical imaging device refers to a medical scanning device capable of obtaining the scan image. For example, the medical imaging device may be a CT scanning device, an Magnetic Resonance (MR) scanning device, a Positron Emission Tomography/Magnetic Resonance (PET/MR) scanning device, a Positron Emission Tomography-Computed Tomography (PET-CT) scanning device, or an ultrasonic scanning device, etc. The present disclosure does not limit the specific type of the medical imaging device, as long as the medical imaging device is able to obtain the scan image of the target object.

In some embodiments, the scanning operation of the medical imaging device on the target object may be manually controlled by the doctor. For example, when the doctor operates the surgical robot master hand to perform the puncture operation, the doctor controls the medical imaging device to perform the scanning operation by stepping on a control pedal of the medical imaging device.

In other embodiments, the scanning operation of the medical imaging device may be automatically controlled by the puncture assistance device.

For example, when the doctor operates the surgical robot to perform the puncture operation, the surgical robot master hand is configured to send a first puncture operation instruction to the first processor and send a movement instruction for performing the puncture operation to the puncture assistance device after receiving the first puncture operation instruction. The first processor is configured to send a fifth movement instruction corresponding to the first puncture operation instruction to the surgical robot slave hand after receiving the first puncture operation instruction. The puncture assistance device is configured to send a scan instruction to the medical imaging device after receiving the movement instruction, causing the medical imaging device to perform medical imaging scanning (i.e., a real-time exposure scanning operation) on the target object. The medical imaging device performs the scanning operation on the target object after receiving the scan instruction. The surgical robot slave hand is configured to move the puncture needle according to the fifth movement instruction to perform a puncture operation corresponding to the first puncture operation instruction. The puncture operation includes a posture adjustment operation or a needle insertion operation, the posture adjustment operation is used to adjust a posture of the puncture needle, the needle insertion operation is used to adjust a puncture depth of the puncture needle. It is able to be understood that the first puncture operation instruction may indicate an operation of the doctor on the surgical robot master hand. The movement instruction may be a notification sent by the surgical robot master hand to the puncture assistance device that the puncture operation is about to be performed. The target object may be a patient. In the embodiment above, when the surgical robot master hand receives the first puncture operation instruction, the puncture assistance device is prompted to control the medical imaging device to perform the scanning operation. This can implement automatic control of the medical imaging device to perform real-time exposure scanning during the puncture operation, thereby ensuring that the medical imaging device performs the scanning operation every time when the surgical robot master hand receives a puncture operation instruction and ensuring the timeliness of the medical imaging scanning. In the embodiment above, the medical imaging scanning is performed before the puncture operation is executed during a time interval between the surgical robot master hand receiving the puncture operation instruction and the surgical robot slave hand executing the puncture operation instruction, thereby avoiding blind puncture caused by the puncture operation having been executed but the medical imaging scanning not having been performed.

It is able to be understood that a specific function of the fifth movement instruction is related to the puncture operation corresponding to the first puncture operation instruction. When the puncture operation corresponding to the first puncture operation instruction is the posture adjustment operation, the fifth movement instruction is used to control the surgical robot to move the puncture needle to perform posture adjustment. When the puncture operation corresponding to the first puncture operation instruction is the needle insertion operation, the fifth movement instruction is used to control the surgical robot to move the puncture needle for needle insertion.

As another example, when the doctor operates the surgical robot to perform the puncture operation, the surgical robot master hand sends a second puncture operation instruction to the first processor after receiving the second puncture operation instruction. The first processor is configured to determine a sixth movement instruction corresponding to the second puncture operation instruction. Then, the first processor is configured to send the sixth movement instruction to the surgical robot slave hand, and send a movement instruction for performing the puncture operation to the puncture assistance device. The puncture assistance device is configured to send a scan instruction to the medical imaging device after receiving the movement instruction, causing the medical imaging device to perform medical imaging scanning on the target object. The medical imaging device performs the scanning operation on the target object after receiving the scan instruction. The surgical robot slave hand is configured to move the puncture needle according to the sixth movement instruction to perform a puncture operation corresponding to the second puncture operation instruction. The puncture operation includes a posture adjustment operation or a needle insertion operation. The posture adjustment operation is used to adjust a posture of the puncture needle. The needle insertion operation is used to adjust a puncture depth of the puncture needle. It is able to be understood that the second puncture operation instruction may indicate an operation performed by a doctor on the surgical robot master hand, the movement instruction may be a notification from the first processor to the puncture assistance device that the surgical robot is about to perform a puncture operation, and the target object may be a patient. In the embodiment above, when the surgical robot master hand receives the second puncture operation instruction, the surgical robot master hand sends the second puncture operation instruction to the first processor. When the first processor receives the second puncture operation instruction, the first processor prompts the puncture assistance device to control the medical imaging device to perform the scanning operation. This enables automatic control of the medical imaging device for real-time exposure during the puncture operation process, thereby ensuring that the medical imaging device performs the scanning operation every time when the surgical robot master hand receives the puncture operation instruction, and ensuring the timeliness of medical imaging scanning. In the embodiment above, the medical imaging scanning is performed before the puncture operation is executed during a time interval between sending the sixth movement instruction corresponding to the second puncture operation instruction to the surgical robot slave hand using the first processor and the surgical robot slave hand executing the sixth movement instruction, thereby avoiding the blind puncture caused by the puncture operation having been executed but medical image scanning not having been performed. In addition, using the first processor to send instruction information to the puncture assistance device, compared with using the surgical robot master hand to send instruction information to the puncture assistance device, the scanning time will be shortened to a certain extent, and thus a radiation dose to the target object can be reduced.

It is able to be understood that a specific function of the sixth movement instruction is related to the puncture operation corresponding to the second puncture operation instruction. When the puncture operation corresponding to the second puncture operation instruction is the posture adjustment operation, the sixth movement instruction is used to control the surgical robot to move the puncture needle for posture adjustment. When the puncture operation corresponding to the second puncture operation instruction is the needle insertion operation, the sixth movement instruction is used to control the surgical robot to move the puncture needle for needle insertion.

It should be understood that the first puncture operation instruction and the second puncture operation instruction may be the same or different, and the present disclosure does not impose limitations on this.

FIG. 2 is a schematic diagram illustrating an exemplary puncture assistance device according to some embodiments of the present disclosure.

As shown in FIG. 2, the puncture assistance device in the embodiments of the present disclosure includes a scanning module 210, a puncture assistance module 220, and a display module 230.

In some embodiments, the scanning module 210 may be configured to obtain a scan image of a target object. More descriptions regarding a medical imaging device may be found in FIG. 1 and the related descriptions.

The target object refers to an object requiring a puncture interventional surgery. In some embodiments, the puncture interventional surgery includes, but is not limited to, at least one of percutaneous intervention, transvascular intervention, puncture biopsy, etc.

In some embodiments, the scanning operation may be a scanning operation performed before an intraoperative puncture. In some embodiments, the scanning operation may be a scanning operation performed during a preoperative image scanning phase.

In some embodiments, the scanning operation may also be a scanning operation performed during a posture adjustment process or during a needle insertion process, the posture adjustment process includes adjusting a posture of a puncture needle, and the needle insertion process includes adjusting a puncture depth of the puncture needle. Performing the scanning operation during the posture adjustment process or needle insertion process can ensure that scanning is performed during the main stages of the puncture and ensure that the user can monitor the puncture process in real-time through the scan image, thereby reducing the difficulty of the puncture and improving the accuracy of the puncture.

In some embodiments, during the posture adjustment process, the medical imaging device performs the scanning operation before a surgical robot executes a posture adjustment operation. During the needle insertion process, the medical imaging device performs the scanning operation before the surgical robot executes a needle insertion operation. By performing scanning before the surgical robot executes the posture adjustment operation or before the surgical robot executes the needle insertion operation, a guiding role of the scanning for the posture adjustment operation or the needle insertion operation can be ensured, thereby avoiding blind posture adjustment or needle insertion. More description regarding the surgical robot may be found in FIG. 1 and the related descriptions.

The scan image refers to an image acquired by the medical imaging device performing a scan. In some embodiments, the scan image may include at least one of a preoperative image, a first image from a first time period, and a second image from a second time period, etc. The first time period is earlier than the second time period.

The preoperative image refers to an image of a target portion (e.g., a lesion, a target organ, etc.) of the target object acquired before the surgical procedure is performed when a user (e.g., a doctor performing the surgery) performs surgical planning (e.g., determining a surgical path, a needle entry point, etc.). The preoperative image may include images of different slices of the target portion of the target object. A pre-surgical phase before the surgical procedure is performed may include any time point before the commencement of the surgical procedure on the target object. For example, the pre-surgical phase may include several days before the surgical procedure, tens of minutes before the surgical procedure, etc. The user may scan the target object before the surgical procedure is performed (e.g., several days before the surgical procedure, tens of minutes before the surgical procedure, etc.) to obtain the preoperative image, thereby facilitating surgical planning for the target object.

The scanning module 210 may perform a plain scan on the target portion of the target object before the surgical procedure is performed via the medical imaging device to obtain the preoperative image. To determine a specific position of the lesion and a distribution position of surrounding organ tissues, the preoperative image may be obtained by scanning the target object based on a relatively large scanning range (e.g., 200 mm) and performing image reconstruction, and the relatively large scanning range includes the target portion and the surrounding organ tissues.

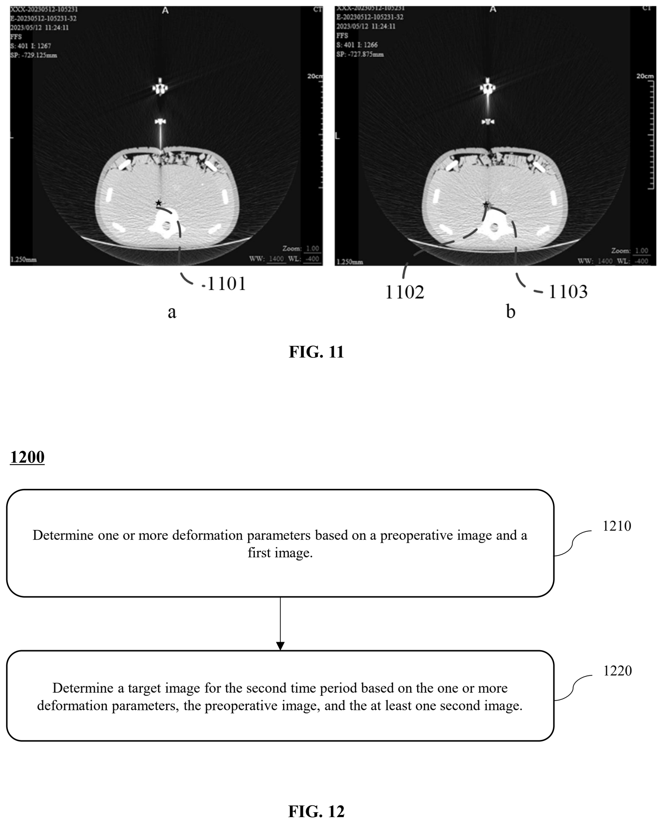

After obtaining the preoperative image, the scanning module 210 also needs to obtain one or more first images in the first time period to register each of the one or more first images with the preoperative image, thereby obtaining one or more deformation parameters. Each of the one or more deformation parameters corresponds to a respiratory amplitude parameter. More descriptions regarding the respiratory amplitude parameter and the deformation parameter may be found in FIG. 12 of the present disclosure and the related descriptions. The first time period refers to a registration preparation time period close to a surgery time period before the surgical procedure is performed, which may also be referred to as an intraoperative preparation time period. For example, the first time period may be 3~7 seconds immediately before the surgery time period. The surgery time period refers to a time period from the commencement of the surgical procedure on the target object to an end of the surgical procedure. For example, the surgery time period refers to the time period from the time when the user formally starts to place a surgical instrument (e.g., the puncture needle, etc.) used in the interventional surgery into the body of the target object to the time when the aforementioned surgical instrument is removed from the body of the target object. The surgical instrument refers to an instrument used in the interventional surgery or the interventional procedure. For example, the surgical instrument may include, but is not limited to, the puncture needle, a drainage tube, etc. The puncture needle may include various types of puncture needles.

The first image refers to an image of the target portion acquired in real-time proximate to the surgery time period. For example, the first image may be a real-time scan image obtained from the target portion within a complete respiratory cycle (e.g., 3~4 s) of the target object proximate to the surgery time period. As another example, the first image may be a real-time scan image obtained from the target portion when the target object performs a respiratory training proximate to the surgery time period. As another example, the first image may be a real-time scan image obtained from the target portion at any time when the target object has a respiratory change proximate to the surgery time period. In some embodiments, the first image is obtained after obtaining the preoperative image. For example, the preoperative image is obtained 2~3 days, 15 minutes, etc., before the surgical procedure, and the first image is obtained 3~7 seconds proximate to the surgery time period.

The scanning module 210 may perform continuous scanning on the target object via the medical imaging device within the first time period, perform real-time reconstruction, and obtain the first image. To accurately reflect a situation of the lesion area, the first image may be an image obtained by scanning the target portion based on a relatively small scanning range (e.g., 20 mm) and performing image reconstruction. In some embodiments, to subsequently determine a plurality of target guidance images to better guide the interventional surgery, the scanning module may obtain a plurality of first images within the first time period. More descriptions regarding the target guidance image may be found in FIG. 12 and the related descriptions.

In some embodiments, the scanning module 210 may also continuously obtain a plurality of scan images of the target object via the medical imaging device within the first time period, divide the plurality of scan images into a plurality of intervals, and select one scan image from each of the intervals as the first image. A deformation value of the target portion of the target object in each of the aforementioned intervals is less than a preset deformation threshold. For example, the scanning module 210 continuously obtains 30 scan images within the first time period of a complete respiratory range of the target object at a preset frequency. The scanning module 210 may evenly divide the aforementioned 30 scan images into 10 intervals. The scanning module 210 selects a middle scan image in each of the intervals as the first image. For example, the scanning module 210 sequentially selects a second scan image, a fifth scan image, a seventh scan image, etc., as the first images.

The second time period refers to a time period in a surgery time period . The second time period may also be referred to as the intraoperative time period.

In some embodiments, the second image refers to an image of a target portion acquired in real time during the surgery time period.

In some embodiments, the scanning module 210 performs continuous scanning on the target portion of the target object through the medical imaging device within the second time period. The scanning module 210 performs image reconstruction to obtain at least one second image. In some embodiments, the first image and the second image may be tomographic images.

To accurately reflect deformation of the lesion region during the surgery execution and reduce the radiation dose of the target object during the surgery, the second image may be an image obtained by scanning the target portion based on the small scanning range (e.g., 20 mm) and performing the image reconstruction. It should be understood that, to determine a plurality of target guidance images subsequently to better guide the interventional surgery, the scanning module 210 may obtain a plurality of second images within the second time period. It should be understood that the second image includes not only the target portion but also the puncture needle. However, because the second image is an image obtained based on the small scanning range, the puncture needle may not be completely visible in the second image. The second image is unable to reflect distribution of the surrounding tissues and organs of the lesion area. Therefore, the second image is unable to be directly used for interventional guidance of the surgical procedure.

It should be understood that, to perform real-time interventional guidance during the interventional surgery, a rate at which the scanning module 210 performs continuous scanning and real-time reconstruction on the target portion through the medical imaging device needs to be greater than a rate threshold. The rate threshold may be a default value (e.g., 10 fps).

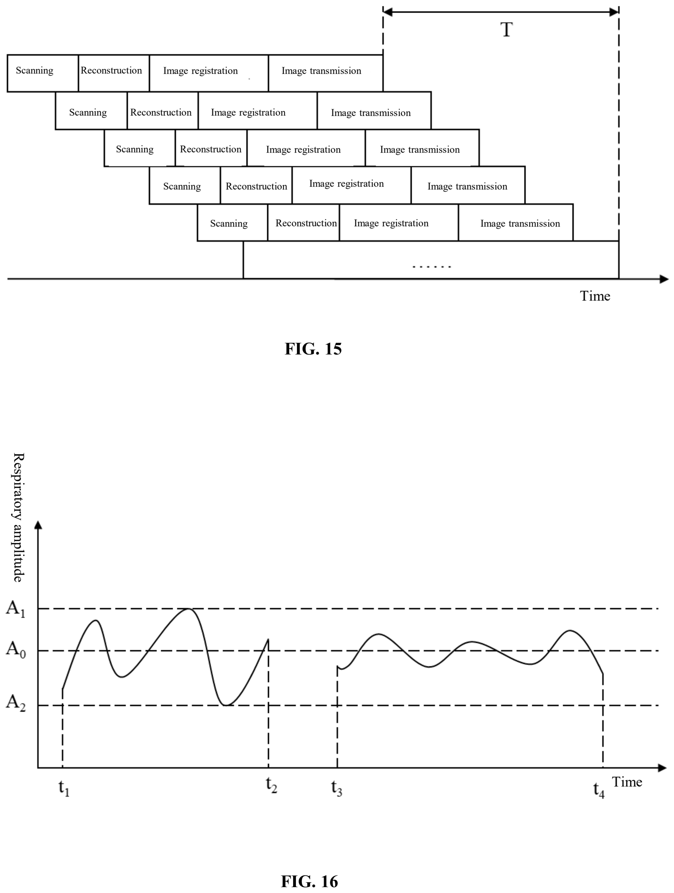

FIG. 15 is an exemplary schematic diagram illustrating real-time image updating according to some embodiments of the present disclosure. Merely by way of example, as shown in FIG. 15, to perform real-time interventional guidance within a time period T, a second image needs to be updated at a rate greater than the rate threshold. The image updating includes scanning the target portion, image reconstruction, image registration between the second image and a preoperative image, and image transmission.

More descriptions regarding acquiring a scan image may be found in FIG. 12 and the related descriptions.

In some embodiments, the puncture assistance module 220 is configured to generate puncture guidance information based on puncture status information and the scan image, and guide a puncture operation based on the puncture guidance information.

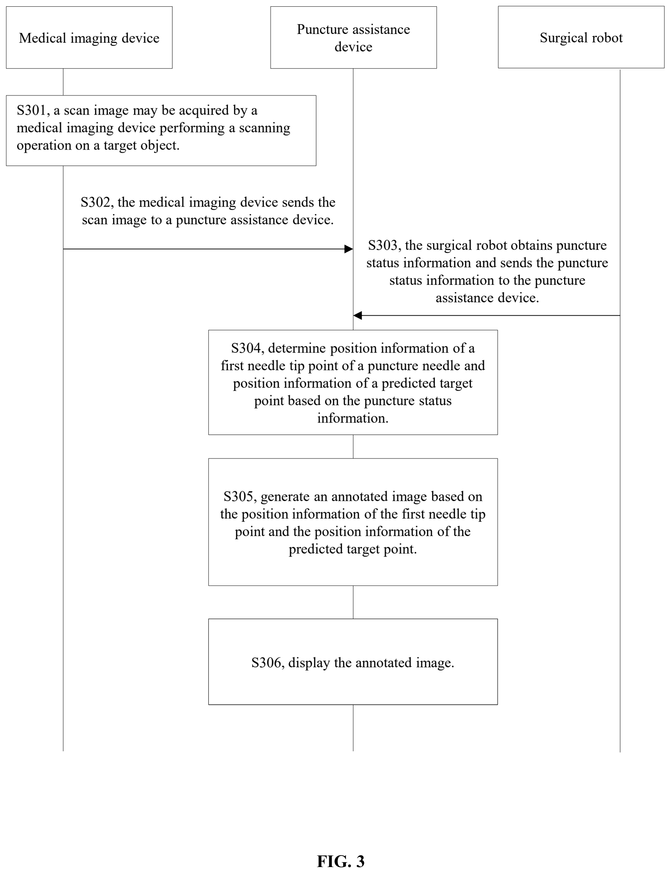

The puncture status information refers to status information of a puncture needle when the scanning operation is performed on a target object. In some embodiments, the puncture status information may include a puncture depth, a puncture rate, a posture angle, etc. More descriptions regarding the puncture status information herein may be found in FIG. 3 and the related descriptions.

In some embodiments, a processor may obtain the puncture status information through a surgical robot. In some embodiments, the puncture assistance module 220 may directly obtain the puncture status information from the surgical robot. Alternatively, the puncture assistance module 220 may indirectly obtain the puncture status information from the surgical robot. The present disclosure does not limit this. For example, the surgical robot may directly report the puncture status information to the puncture assistance module 220. Alternatively, the puncture assistance module 220 may actively obtain the puncture status information from the surgical robot. Alternatively, the surgical robot may send the puncture status information to a relay device. The puncture assistance module 220 may obtain the puncture status information from the relay device. More descriptions regarding the surgical robot may be found in FIG. 1 and the related descriptions.

The puncture guidance information refers to guidance information that facilitates the puncture operation on the target object.

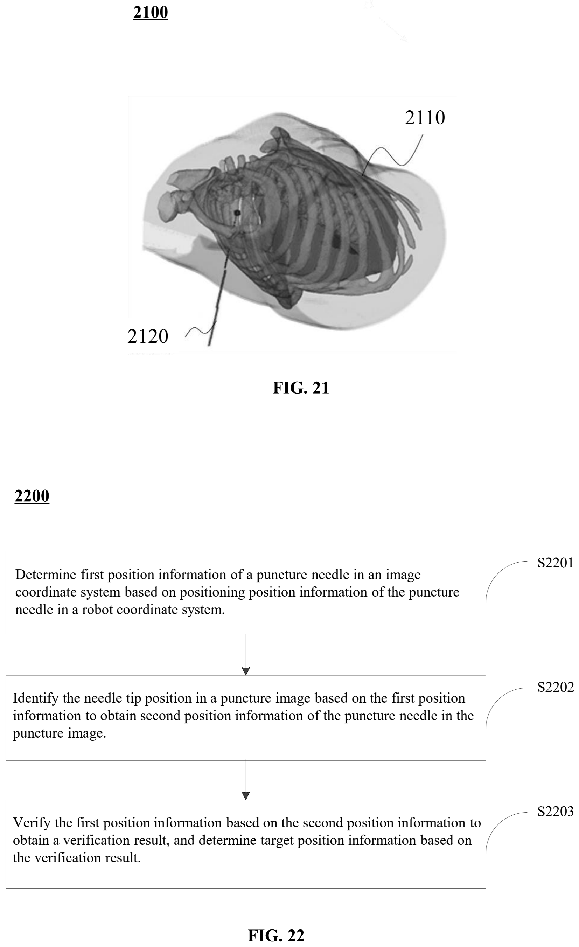

In some embodiments, the puncture guidance information may include positioning information, such as positioning position information, first position information, second position information, third position information, target position information. The puncture guidance information may further include alarm information that prompts needle withdrawal when the puncture needle is deformed. More descriptions regarding the puncture guidance information may be found in FIG. 22 to FIG. 27 and the related descriptions.

In some embodiments, the puncture guidance information further includes information of a body surface puncture point, a real-time needle tip position, a predicted target point, a lesion position, and a posture adjustment process, various annotated images, etc. More descriptions regarding the puncture guidance information may be found in FIG. 3 to FIG. 11 and the related descriptions.

In some embodiments, the puncture guidance information may further include one or more target guidance images, etc. More descriptions regarding the puncture guidance information may be found in FIG. 12 to FIG. 21 and the related descriptions.

The puncture operation refers to a related operation during a puncture surgery.

In some embodiments, the display module 230 is configured to display the puncture guidance information.

In some embodiments, the display module 230 may display the puncture guidance information in a plurality of ways. For example, the display module 230 may control the medical imaging device to display the puncture guidance information.

In some embodiments, the puncture assistance device may further include a positioning correction module. More descriptions regarding the positioning correction module may be found in FIG. 22 and the related descriptions.

In some embodiments of the present disclosure, the puncture guidance information is generated based on the puncture status information and the scan image to guide the puncture operation. This can more intuitively guide the doctor to perform the puncture operation and improve accuracy and safety of the puncture surgery.

FIG. 3 is an exemplary diagram illustrating an interaction view of a puncture method according to some embodiments of the present disclosure. As shown in FIG. 3, the puncture method includes the following operations.

In S301, a scan image may be acquired by a medical imaging device performing a scanning operation on a target object. More descriptions regarding image acquisition may be found in FIG. 2 and the related descriptions.

In S302, the medical imaging device sends the scan image to a puncture assistance device

In S303, the surgical robot obtains puncture status information and sends the puncture status information to the puncture assistance device.

More description about the surgical robot obtaining the puncture status information may be found in FIG. 2 and the related descriptions.

In S304, position information of a first needle tip point of a puncture needle and position information of a predicted target point are determined based on the puncture status information.

The position information of the first needle tip point is determined by calculation based on a relative positional relationship between the puncture needle and the surgical robot. That is, the position information of the first needle tip point is a theoretical value determined by calculation. When the puncture needle is in a normal state, the position information of the first needle tip point obtained by calculation is accurate. In this case, the puncture assistance device may directly obtain the position information of the first needle tip point from the puncture status information. It is able to be understood that the puncture needle being in the normal state refers to the puncture needle not having an abnormality, such as bending or breakage.

Exemplarily, the position information of the first needle tip point may include coordinates of the needle tip point. The coordinates are obtained by solving joint angles of the robotic arm. A specific calculation process is not elaborated herein.

In some embodiments, in addition to directly obtaining the coordinates of the needle tip point from the surgical robot, the puncture assistance device may obtain related parameters from the surgical robot and solve the joint angles of the robotic arm based on the related parameters to obtain the coordinates of the needle tip point. In some embodiments, when the robotic arm is a 5-axis joint robotic arm, a specific calculation process of the coordinates of the needle tip point may refer to the following formula (1):

$$T = \prod_{i=1}^{5} \exp\left(\hat{\xi}_i, \theta_i\right) \cdot T_{\text{Conrol}}^{Base}(0) \ i = 1, \dots, 5 \ (1)$$

$\hat{\xi}_i$, $i = 1, \dots, 5$ denotes the screw coordinates of each joint of the robotic arm, $\theta_i$, $i = 1, \dots, 5$ denotes the joint angle of each axis of the axis joint robotic arm of the surgical robot, and $T_{\text{Conrol}}^{Base}(0)$ denotes a homogeneous transformation matrix of the control point coordinate system in a robot coordinate system when all joints of the robotic arm are in a zero position. T denotes a posture matrix of the multi-axis surgical robot, which includes posture information and position information of the multi-axis surgical robot, where the position information is $_{tip0}^{RB}T$, $_{tip0}^{RB}T$ denotes initial coordinates of the needle tip point in the robot coordinate system.

In some embodiments, during needle insertion, real-time coordinates of the needle tip point may be obtained by the following formula (2):

$$_{Tip}^{RB}T = {}_{tip0}^{RB}T \cdot transl(0,0,-depth) \ (2)$$

$_{Tip}^{RB}T$ denotes the real-time coordinates of the needle tip point in the robot coordinate system, and *(0,0, -depth)* denotes movement distances of real-time needle tip point in X, Y, and Z directions of the robot coordinate system, respectively.

In some embodiments, the position information of the first needle tip point may also be the position information of the needle tip point in the target position information obtained after a correction. More descriptions regarding the content herein may be found in FIG. 22 and the descriptions.

In some embodiments, the predicted target point refers to an estimated spatial point reached by the needle tip point of the puncture needle after completing the needle insertion according to a posture in the puncture status information.

Merely by way of example, the position information of the predicted target point may include coordinates of the predicted target point. The puncture assistance device may obtain the coordinates of the predicted target point based on the initial coordinates of the needle tip point in the robot coordinate system and a length of the puncture needle. The specific calculation process of the coordinates of the predicted target point may be found in the following formula (3):

$$_{target}^{RB}T = {}_{tip0}^{RB}T \cdot transl(0,0,-length) \ (3)$$

$_{tip0}^{RB}T$ denotes the initial coordinates of the needle tip point in the robot coordinate system, *length* denotes the length of the puncture needle, and $_{target}^{RB}T$ denotes the coordinates of the predicted target point in the robot coordinate system.

In S305, an annotated image may be generated based on the position information of the first needle tip point and the position information of the predicted target point.

In some embodiments, the annotated image refers to an image obtained after annotating the needle tip point, the predicted target point, etc., onto a target image.

In some embodiments, the target image may include a preoperative image, a first image, one or two images among a plurality of second images, a target guidance image, etc. More descriptions regarding the second image may be found in FIG. 2 and the related descriptions.

In some embodiments, the target image may be determined based on the scan images.

In some embodiments, the puncture assistance device may be further configured to determine each of the preoperative image and the first image in the scan images as the target image.

In some embodiments, the puncture assistance device may be further configured to determine a second image in the scan image as the target image.

In some embodiments, the puncture assistance device may be further configured to determine the target image of the second time period (i.e., the target guidance image) based on the preoperative image, the first image, and the second image. More content herein may be found in FIG. 12 and the related descriptions.

In some embodiments, the puncture assistance device may generate the annotated image based on the position information of the first needle tip point and the position information of the predicted target point. For example, the puncture assistance device may determine a corresponding second image based on the position information of the first needle tip point and determine the corresponding second image as the target image. The puncture assistance device may annotate the needle tip point on the target image to generate the annotated image. Merely by way of example, after the puncture assistance device determines the target image where the needle tip point is located, the puncture assistance device may convert the coordinates of the needle tip point in the robot coordinate system to the image coordinate system of the second image obtained by the medical imaging device, and then annotate the needle tip point on the corresponding target image to generate the annotated image. Similarly, in some embodiments, the puncture assistance device may annotate the predicted target point onto the target image based on the position information of the predicted target point to generate the annotated image.

In some embodiments, the puncture assistance device may annotate the needle tip point and the predicted target point onto the same target image or on two different target images. The needle tip point and the predicted target point each occupy a certain three-dimensional space. The needle tip point may be located in one or more target images of the target object, so the target images involving the needle tip point may include one or more images. Similarly, the predicted target point may also be located in one or more slices of the target object, so the target images involving the predicted target point may include one or more images. In the present disclosure, quantifiers such as one or a plurality regarding the description of images may represent one image or a plurality of images, or one slice or a plurality of slices of images, etc. The present disclosure does not impose limitations.

For example, when the needle tip point and the predicted target point are located in the same slice of the target object, the needle tip point and the predicted target point may be annotated on the same target image. In this case, the target image includes one second image from the plurality of second images, the annotated target image is the annotated image, and the quantity of the annotated image is one. When the needle tip point and the predicted target point are located in different slices of the target object, the needle tip point and the predicted target point are annotated onto two different second images. In this case, the target image includes at least two second images from the plurality of second images, the annotated target images are the annotated images, and the quantity of the annotated images is two.

In S306, the annotated image is displayed.

It is able to be understood that the displayed annotated image (i.e., the annotated target image) may be a portion of the tomographic images of the target image, or all tomographic images of the target image. The present disclosure does not impose limitations on this.

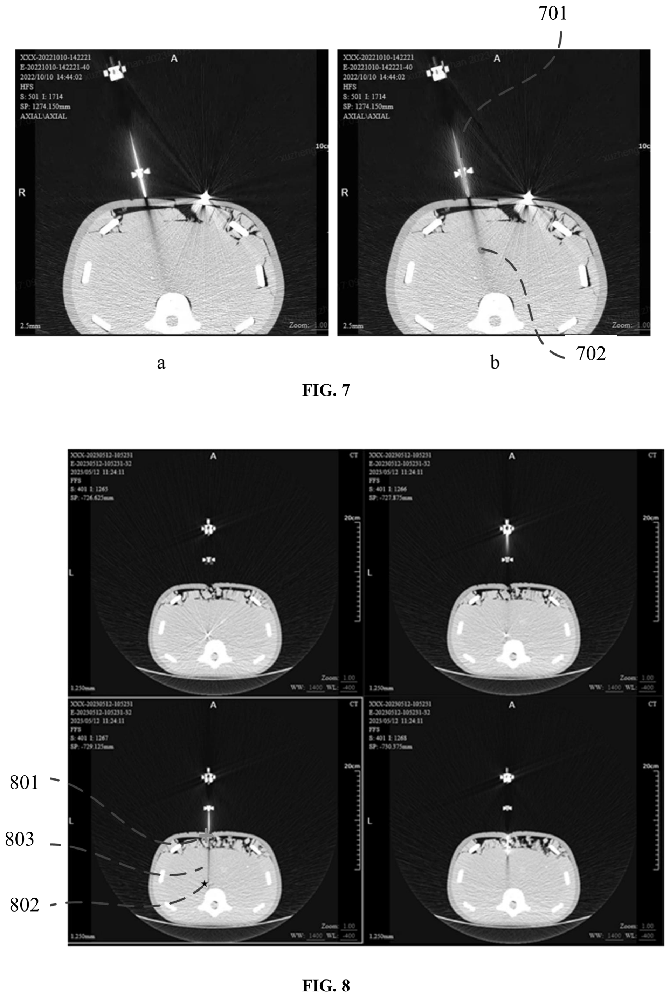

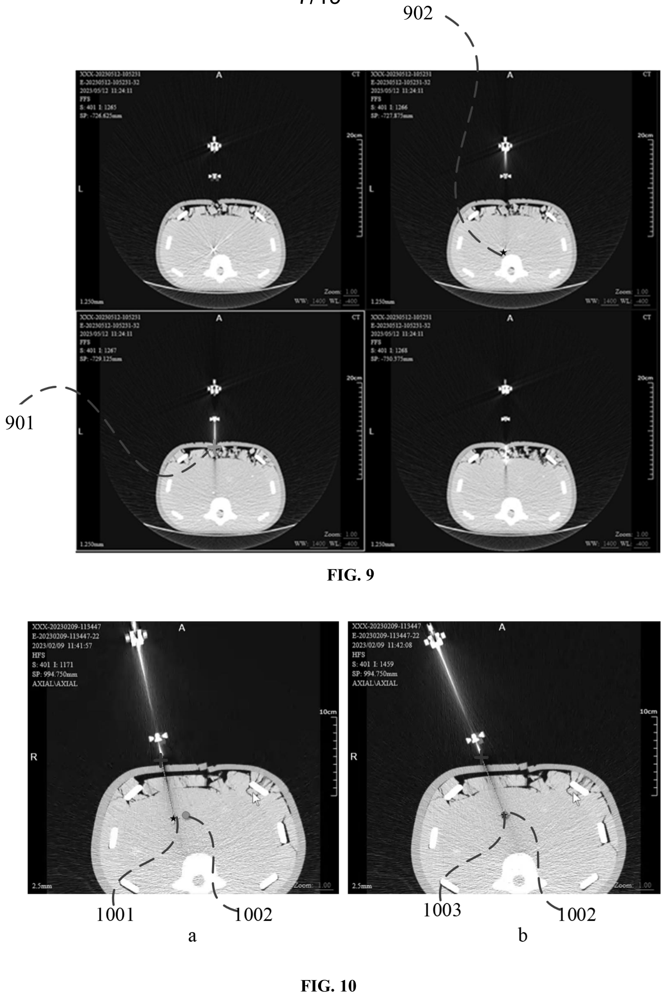

In some embodiments, the needle tip point and the predicted target point may be located on target images of the same slice or on target images of different slices. When the needle tip point and the predicted target point are annotated onto the target images of the same slice, the annotated image also displays a connection line between the needle tip point and the predicted target point. More content herein may be found in FIG. 8 and FIG. 9 and the related descriptions.

In some embodiments, the puncture assistance device displays the puncture status information through a display module. The displayed puncture status information may include at least one of the puncture depth, the puncture rate, and the posture angle. The puncture depth refers to a depth to which the needle tip point of the puncture needle has entered the skin. The posture angle refers to an angle of the puncture needle relative to the target object. The puncture rate refers to a moving rate of the puncture needle along a needle insertion direction.

In some embodiments, when the puncture assistance device determines that the puncture rate is greater than a certain threshold, the puncture assistance device displays warning information indicating an excessive puncture rate through the display module. By reminding about the excessive puncture rate, a probability of overly fast puncture can be reduced, and the safety of the puncture operation can be improved. For example, when the puncture rate is greater than 30 mm/s, the warning information indicating an excessive puncture rate may be displayed. The specific form of the warning information may be a pop-up prompt window, or a display method of the puncture rate may change, for example, displaying a warning icon simultaneously with the displayed puncture rate. The present disclosure does not impose limitations on the form of the warning information.

In some embodiments, the puncture assistance device may also annotate a target lesion of the target object. In some embodiments, the puncture assistance device annotates the target lesion of the target object onto the second image among the plurality of tomographic images to obtain the annotated second image. The second image includes a tomographic image among the plurality of tomographic images where the target lesion is scanned. An annotated second image is displayed through the display module. In the embodiment, by annotating the target lesion, the user can see the lesion of the target object more clearly, thereby allowing the user to control the puncture process more accurately and improving the user experience.

It is able to be understood that the lesion is generally a three-dimensional structure, so the target lesion of the target object may exist in a plurality of slices, i.e., the target lesion may be identified through a plurality of tomographic images obtained by scanning.

In some embodiments, if the second image includes only one tomographic image, then the second image may be a tomographic image where a center of the target lesion is scanned, or the second image may be a tomographic image with the largest lesion area, or the second image may also be a tomographic image satisfying other conditions. The present disclosure does not impose limitations on this.

In other embodiments, the second image may include each of multiple tomographic images, or the second image may include each of the all tomographic images where the target lesion is scanned. The present disclosure does not impose limitations on this.

In some embodiments, the target lesion is annotated on a tomographic image annotated with the predicted target point, i.e., the first image annotated with the predicted target point and the second image annotated with the target lesion may be the same tomographic image. By annotating the target lesion on the tomographic image where the predicted target point is located, it is convenient for the user to determine whether the puncture needle is able to reach the target lesion by performing a puncture with a current puncture state. For example, when the predicted target point coincides with the target lesion, or the predicted target point is located within a contour range of the target lesion, it can be determined that the puncture needle is able to reach the target lesion by performing the puncture under the current puncture state. Otherwise, it is determined that the puncture needle is unable to reach the target lesion by performing the puncture under the current puncture state.

In other embodiments, the needle tip point, the predicted target point, and the target lesion may be annotated onto the same tomographic image, the first image annotated with the needle tip point and the predicted target point includes one tomographic image, the second image annotated with the target lesion includes one tomographic image, and the first image and the second image are the same tomographic image.

Merely by way of example, while displaying the annotated second image through the display module, the puncture assistance device also displays adjacent scan images of the second image through the display module. In the embodiment above, the display of the adjacent scan images of the second image is substantially the same in manner and technical effect as the display of the adjacent scan images of the first image described above, and is not repeated here.

In some embodiments, the puncture assistance device also displays a three-dimensional image of the puncture needle and a three-dimensional image of the target lesion through the display module. Each of the three-dimensional image of the puncture needle and the three-dimensional image of the target lesion is obtained by using the plurality of tomographic images. Each of the three-dimensional image of the puncture needle and the three-dimensional image of the target lesion may be obtained by performing three-dimensional reconstruction using the plurality of tomographic images, or may be generated by an image recognition algorithm, the present disclosure does not limit this. In the embodiment above, by displaying the three-dimensional images, the presentation of the puncture needle and the target lesion is more three-dimensional and vivid.

Merely by way of example, the puncture needle and the target lesion are displayed in the same three-dimensional image, a relative positional relationship between the puncture needle and the target lesion in the three-dimensional image is the same as a relative positional relationship between the puncture needle and the target lesion in the image coordinate system, and the image coordinate system refers to a coordinate system where the plurality of tomographic images are located. By displaying the puncture needle and the target lesion in the same three-dimensional image, and faithfully presenting the relative positional relationship between the puncture needle and the target lesion in the image coordinate system, the puncture progress can be seen more intuitively, thereby facilitating the user to determine whether the puncture is completed.

Merely by way of example, in the same three-dimensional image, the puncture needle and the target lesion are displayed in different colors. By displaying the puncture needle and the target lesion in different colors, it is convenient for the user to distinguish the puncture needle and the target lesion, and the user can better determine the relative positional relationship between the puncture needle and the target lesion.

In some embodiments, the processor can flexibly set and display an exposure range and an exposure interval time of the annotated image.

The exposure range refers to data reflecting an exposure degree of the annotated image. For example, the exposure range may include a center of the annotated image, a slice thickness of the annotated image, and a count of slices radiating outward, etc.

The exposure interval time refers to a time interval between two adjacent exposures.

In some embodiments, the processor may flexibly set the exposure range and the exposure interval time of the annotated image in a plurality of ways. For example, the processor may obtain historical settings of the doctor within a preset historical time period from memory, and set an exposure range and an exposure interval time used most frequently in the historical settings as the exposure range and the exposure interval time of the current annotated image.

In some embodiments, the processor may display the exposure range of the annotated image in a plurality of ways. For example, the processor may control the medical imaging device to display the exposure range and the exposure interval time of the annotated image while displaying the annotated image.

In some embodiments, the processor may determine the exposure range based on a puncture range and lesion characteristics.

The puncture range refers to a range of a travel path of the puncture needle. The puncture range may be preset in advance.

The lesion characteristics refer to relevant features that characterize the lesion. For example, the lesion characteristics may include the size of the lesion, the shape of the lesion, an organ where the lesion is located, etc.

In some embodiments, the processor may determine the exposure range based on a first preset relationship among the puncture range, the lesion characteristics, and the exposure range. The first preset relationship may be preset according to actual situations and historical experiences. For example, the first preset relationship may include a correlation between the puncture range and the exposure range. Merely by way of example, if a current exposure range is unable to cover the puncture range, the exposure range is increased. As another example, the first preset relationship may include a correlation between the lesion characteristics and the exposure range. Merely by way of example, if the current exposure range is unable to cover the lesion that needs to be punctured, the exposure range is increased. If the current exposure range is much larger than the lesion, so that the lesion is unable to be clearly displayed, the exposure range is reduced.

In some embodiments, the processor may determine the exposure range based on the puncture range and the lesion characteristics through a first vector database. For example, the processor may construct the first vector database based on a historical puncture range, historical lesion characteristics, and an actual exposure range in historical records, construct a first feature vector based on the current puncture range and the lesion characteristics, perform matching in the first vector database, and determine the actual exposure range corresponding to a first reference vector in the first vector database with the highest similarity as the exposure range corresponding to the current situation.

The first vector database includes the first reference vectors and corresponding actual exposure ranges each of which corresponds to one of the first reference vectors. A first reference vector is constructed based on the actual historical puncture range and the historical lesion characteristics in the historical records, and the actual exposure range corresponding to the first reference vector may be determined based on an actual exposure range with the highest lesion coverage rate in historical surgeries. The similarity may be calculated based on a cosine distance, a Euclidean distance, etc.

In some embodiments, the processor may determine the exposure range based on the puncture range and the lesion characteristics through an exposure range prediction model.

In some embodiments, the exposure range prediction model may be a machine learning model. For example, the exposure range prediction model may be a neural network model, a deep neural network model, etc., or any combination thereof.

In some embodiments, the exposure range prediction model may be obtained by training based on first training samples and first labels. A first training sample may be a sample puncture range and sample lesion characteristics in the historical records, and the first label of the first training sample may be constructed based on the first training sample. For example, the processor may construct the first label of the first training sample based on a distance between a center point of the puncture range and a center point of the exposure range corresponding to the first training sample in a historical surgery, e.g., using the exposure range with the shortest distance as the first label. As another example, the processor may construct the first label of the first training sample based on a coverage rate of the lesion in the historical surgery corresponding to the first training sample, e.g., using the exposure range corresponding to the historical surgery with the highest coverage rate as the first label.

In some embodiments, the processor may input a large count of first training samples into an initial exposure range prediction model, construct a loss function based on an output of the initial exposure range prediction model and the first labels, and iteratively update the initial exposure range prediction model based on the loss function; when a value of the loss function satisfies an iteration termination condition, the training is completed, and a trained exposure range prediction model is obtained. The iteration termination condition may include the loss function converging, a count of iterations reaching a threshold, etc.

In some embodiments of the present disclosure, by determining the exposure range based on the puncture range and the lesion characteristics, a more reasonable exposure range can be determined to obtain more accurate scan images, so as to provide the doctor with better real-time image reference during the puncture process.

In some embodiments, the processor may determine the exposure range based on an adjustment amplitude of the posture of the puncture needle.

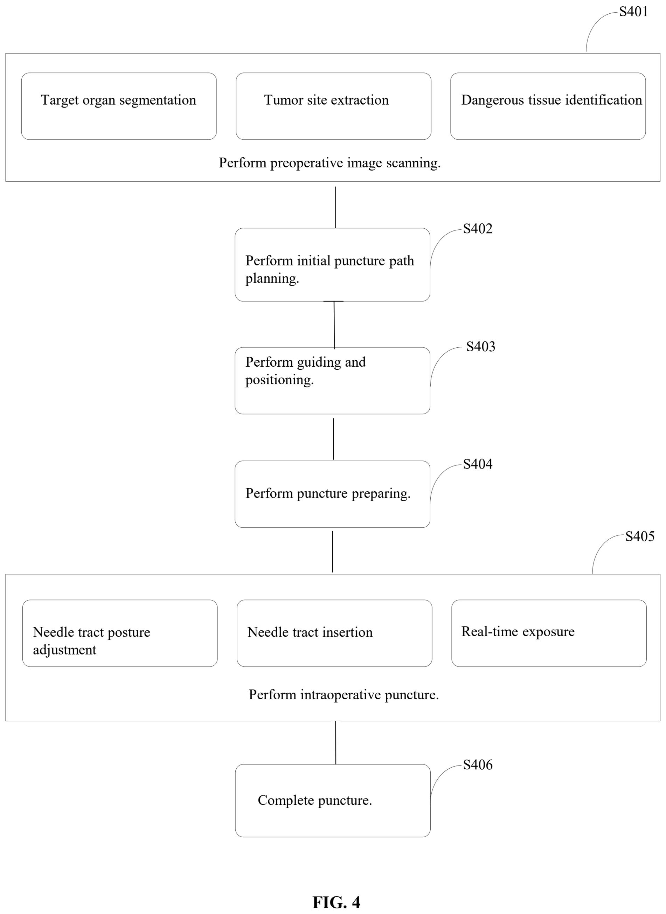

The adjustment amplitude of the posture of the puncture needle refers to an indicator reflecting an adjustment condition of the posture of the puncture needle. For example, the adjustment amplitude may include an adjustment angle of the posture of the puncture needle, an adjustment distance, etc. The adjustment angle of the posture of the puncture needle may include a relevant angle of an intra-slice posture adjustment and a relevant angle of an inter-slice posture adjustment. More descriptions regarding the posture adjustment of the puncture needle may be found in FIG. 4 or FIG. 9-FIG. 11 and the related descriptions.

In some embodiments, the processor may determine the exposure range based on a second preset relationship of the adjustment amplitude of the posture of the puncture needle and of the exposure range. The second preset relationship may be preset according to actual conditions and historical experiences. For example, the second preset relationship may include a correlation between the adjustment angle of the posture of the puncture needle and the exposure range. Merely by way of example, when the adjustment angle of the posture of the puncture needle is an angle of the inter-slice posture adjustment, a center of the scan image is adjusted so that the predicted target point is able to be annotated on the scan image after the posture adjustment of the puncture needle.

In some embodiments, the processor may determine the exposure range through a second vector database based on the adjustment amplitude of the posture of the puncture needle. For example, the processor may construct the second vector database based on historical adjustment amplitudes of the posture of the puncture needle and actual exposure ranges in the historical records, construct a second feature vector based on a current adjustment amplitude of the posture of the puncture needle, perform matching in the second vector database, and determine an actual exposure range corresponding to a second reference vector with a highest similarity as a current corresponding exposure range.

The second vector database includes second reference vectors and corresponding actual exposure ranges each of which corresponds to one of the second reference vectors. A second reference vector is constructed from an actual historical adjustment amplitude of the posture of the puncture needle in the historical records. The corresponding actual exposure range may be determined in a manner similar to determining the first label. The similarity may be calculated based on a cosine distance, a Euclidean distance, etc.

In some embodiments, an input of the exposure range prediction model may further include the adjustment amplitude of the posture of the puncture needle. In some embodiments, when the input of the exposure range prediction model includes the adjustment amplitude of the posture of the puncture needle, the first training sample may further include the historical adjustment amplitude of the posture of the puncture needle in historical data.

In some embodiments of the present disclosure, by considering the adjustment amplitude of the posture of the puncture needle when determining the exposure range, the determined exposure range can better conform to actual conditions, a more referential scan image can be obtained, and a more precise puncture operation can be facilitated for the doctor.

In some embodiments, the processor may further determine an exposure interval time between two adjacent exposures based on a risk level of a current puncture needle area, the lesion characteristics, and the adjustment amplitude of the posture of the puncture needle.

The risk level of the puncture needle area refers to an indicator reflecting a probability and a danger degree of harm to the target object from the puncture needle.

In some embodiments, the processor may construct a first preset table based on a distance between a tip of the puncture needle and a blood vessel or a bone and the risk level of the puncture needle area, and determine the risk level of the current puncture needle area by querying the first preset table. The first preset table includes a correspondence between the distance between the tip of the puncture needle and the blood vessel or the bone and the risk level of the puncture needle area. The first preset table may be constructed based on historical data or preset manually based on experience. For example, the correspondence in the first preset table may be that a smaller distance between the tip of the puncture needle and the blood vessel or the bone corresponds to a higher risk level of the puncture needle area.

In some embodiments, the processor may determine the exposure interval time by matching a third vector database based on the risk level of the current puncture needle area, the lesion characteristics, and the adjustment amplitude of the posture of the puncture needle. For example, the processor may construct the third vector database based on historical risk levels of the puncture needle area, historical lesion characteristics, historical adjustment amplitudes of the posture of the puncture needle, and actual exposure interval times in the historical records, construct a third feature vector based on the risk level of the current puncture needle area, the lesion characteristics, and the adjustment amplitude of the posture of the puncture needle, perform matching in the third vector database, and determine an actual exposure interval time corresponding to a third reference vector with a highest similarity as a current corresponding exposure interval time.

The third vector database includes third reference vectors and corresponding actual exposure interval times each of which corresponds to one of the third reference vectors. A third reference vector is constructed from an actual historical risk level of the puncture needle area, historical lesion characteristics, and a historical adjustment amplitude of the posture of the puncture needle in the historical records. The corresponding actual exposure interval time may be determined in a manner similar to determining a second label described later. The similarity may be calculated based on a cosine distance, a Euclidean distance, etc.

In some embodiments, the processor may determine the exposure interval time by an exposure interval prediction model based on the risk level of the current puncture needle area, the lesion characteristics, and the adjustment amplitude of the posture of the puncture needle. In some embodiments, the exposure interval prediction model may be a machine learning model. For example, the exposure interval prediction model may be the neural network model, the deep neural network model, etc., or a combination thereof.

In some embodiments, the exposure interval prediction model may be obtained by training based on second training samples with second labels. A second training sample may be a sample risk level of the puncture needle area, sample lesion characteristics, and a sample adjustment amplitude of the posture of the puncture needle in the historical records. The second label of the second training sample may be constructed based on the second training sample. For example, the processor may construct the second label based on an overlap rate between the puncture needle travel path captured by the medical imaging device after the posture adjustment of the puncture needle and a planned puncture needle travel path in historical surgeries corresponding to the second training sample. For example, an exposure interval time corresponding to the historical surgery with the highest overlap rate described above is used as the second label.

A specific training process of the exposure interval prediction model is similar to a specific training process of the exposure range prediction model. More descriptions regarding the content herein may be found in the related descriptions above.

In some embodiments of the present disclosure, by determining the exposure interval time based on the risk level of the current puncture needle area, the lesion characteristics, and the adjustment amplitude of the posture of the puncture needle, the actual conditions can be better combined, and the exposure interval time that better satisfies current needs can be determined to display a more accurate annotated image.

By flexibly setting and displaying the exposure range and the exposure interval time of the annotated image, the display of the annotated image can better conform to actual needs of the user, and the user experience can be further improved.

FIG. 4 is an exemplary schematic diagram illustrating a puncture process according to some embodiments of the present disclosure.

As shown in FIG. 4, the puncture process mainly includes S401 to S406. The operations S401 to S406 are described below.

In S401, preoperative image scanning is performed.

In some embodiments, before the puncture surgery starts, a medical imaging device is used to scan a diseased portion of a target object. For example, when the medical imaging device is a CT scanning device, the CT scanning device may be used to perform multi-phase enhanced scanning and plain scanning. An image obtained through scanning is able to clarify lesion characteristics (e.g., a lesion nature, a contour, a range, etc.), and an analysis of important organs may be achieved.

Merely by way of example, in the preoperative scanning stage, three-dimensional reconstruction in transverse, coronal, and sagittal orientations may be performed on an abdominal liver and a chest lung lobe, and virtual reality (VR) display may be adopted.

In the embodiment shown in FIG. 4, a tumor site is used as a target position for puncture. In the preoperative image scanning stage, operations such as target organ segmentation, tumor site extraction, and dangerous tissue identification may be achieved.

Merely by way of example, in the preoperative image scanning stage, a contour extraction may be performed on a lesion, a soft tissue, a target organ, and other important tissues and organs based on a Dicom image scanned by the CT scanning device, and the processor displays the extracted image through the display module.

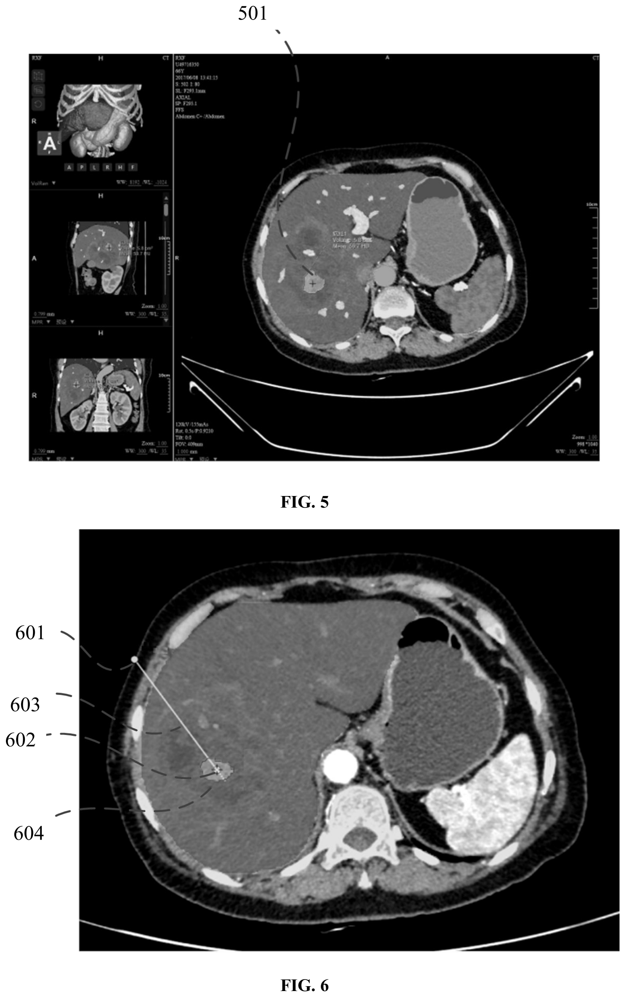

FIG. 5 is an exemplary schematic diagram illustrating a tissue segmentation obtained during a preoperative image scanning stage according to some embodiments of the present disclosure. As shown in FIG. 5, in the embodiment, the CT scanning device is used for a scanning operation, the lesion is located on a liver, and a contour of the lesion 501 is marked in FIG. 5. In the embodiment, in the preoperative image scanning stage, marking the lesion in the image is able assist the doctor in locating the lesion position of the patient and understanding a situation around the lesion.

In S402, initial puncture path planning is performed.

Based on a tissue model obtained in the preoperative image scanning stage, i.e., a tissue segmentation diagram as shown in FIG. 5, an initial puncture path is planned by a plurality of planning manners (e.g., automatic planning, semi-automatic planning, manual planning, etc.) under a premise of avoiding important tissues and organs such as blood vessels, nerves, bones, etc.

The initial puncture path may include: a body surface puncture point of the target object, a puncture target point, a needle insertion angle, and a needle insertion depth. FIG. 6 is an exemplary schematic diagram illustrating an initial puncture path displayed on an image obtained by a scanning device according to some embodiments of the present disclosure. As shown in FIG. 6, in the embodiment, the initial puncture path includes a body surface puncture point 601, a puncture target point 602, and a needle tract 603 connecting the body surface puncture point 601 and the puncture target point 602, and the puncture target point 602 is located within a lesion contour 604.

In some embodiments, the initial puncture path may be displayed on a three-dimensional image, a conversion from a two-dimensional image to the three-dimensional image may be implemented according to a three-dimensional reconstruction manner, which is not described in detail in the present disclosure.

In S403, guiding and positioning are performed.

An end-puncture actuator is connected to a surgical robot slave hand, and the end-puncture actuator is fixed next to a medical imaging device. Then, a registration relationship among a target object space, a mechanical arm space of the surgical robot slave hand, and a medical image space of the medical imaging device is determined by a surgical robot navigation device to obtain a registration matrix. A coordinate transformation among the three spaces may be achieved using the registration matrix, the target object space may also be referred to as a patient space. In some embodiments, the navigation device refers to an NDI stereotactic navigation device, or the like. In some embodiments, without the surgical robot navigation device, the end-puncture actuator at an end of the surgical robot may first be scanned using the medical imaging device to achieve registration between the surgical robot and the medical imaging device. Then, a coordinate transformation relationship between the medical imaging device and the target object is obtained from a preoperative image, thereby enabling determination of the coordinate transformation relationships among the three spatial coordinate systems.

In S404, puncture preparing is performed.

A physician selects a puncture needle of an appropriate length based on a needle insertion depth in the initial puncture path plan and installs the puncture needle on the puncture tip of the surgical robot slave hand. After the puncture needle is installed, the surgical robot slave hand automatically controls a robotic arm to move the puncture needle to a puncture starting point, the puncture starting point is the body surface puncture point in the initial path plan. A needle insertion angle of the puncture needle is adjusted to be consistent with the needle insertion angle in the initial puncture path plan.

S405, intraoperative puncture is performed.

As shown in FIG. 4, the intraoperative puncture phase primarily includes three portions: needle tract posture adjustment, needle tract insertion, and real-time exposure. The needle tract posture adjustment primarily includes adjustment of a posture of the puncture needle. The needle tract insertion primarily includes adjustment of a puncture depth of the puncture needle. The real-time exposure primarily includes scanning the target object with medical imaging during the posture adjustment and a needle insertion process.

In embodiments of the present disclosure, a physician may choose automatic posture adjustment or manual posture adjustment based on clinical circumstances. The automatic posture adjustment refers to the puncture assistance device automatically controlling the surgical robot to adjust the posture of the puncture needle. The manual posture adjustment refers to the physician performing posture adjustment such as translation and rotation on the puncture needle in the surgical robot by operating the surgical robot.

In embodiments of the present disclosure, during the needle tract posture adjustment and needle tract insertion process, the medical imaging device is controlled to perform real-time exposure, and an exposure range is set within a puncture range, i.e., the exposure range is set within the tomographic range where the needle tract is located. This allows real-time observation of the movement direction of the puncture needle inside the body of the patient and the actual position reached by the needle tip point of the puncture needle, thereby enabling assessment of the current puncture situation and avoiding impact of the puncture on the important tissues or organs of the patient.

In S406, puncture is completed.

After the needle tract insertion is completed, the needle tip point of the puncture needle reaches the lesion area, the puncture operation is completed.

It is able to be understood that the lesion area in embodiments of the present disclosure is a broad concept, and the definition of the lesion area may vary depending on the type of surgery. For example, for puncture surgeries such as biopsy and injection, the lesion area refers to an area encompassed by a lesion contour. For puncture surgeries such as ablation and particle implantation, the lesion area includes the area encompassed by the lesion contour and an area within a certain range at a lesion edge or periphery.

It is able to be understood that, in the aforementioned puncture process, the intraoperative puncture phase is the most critical and important phase. The puncture method in embodiments of the present disclosure primarily focuses on improvements to the intraoperative puncture phase. The puncture method in embodiments of the present disclosure is described below with reference to the accompanying drawings.

For example, the processor may display, through a display module, a tomographic image where a center of the needle tip point is located among all annotated tomographic images marked with the needle tip point. The processor may also display, through the display module, a tomographic image where a center of the predicted target point is located among all annotated tomographic images marked with the predicted target point.

It should be noted that the descriptions of the aforementioned processes are merely for illustration and explanation and do not limit the applicable scope of the present disclosure. Those skilled in the art is able to make various modifications and changes to the processes under the guidance of the present disclosure. For example, the sequence of operation S401, operation S402, and operation S403 is merely for illustration and explanation, and operation S403 does not necessarily occur after operation S402. Merely by way of example, registration between the surgical robot and the medical imaging device may occur before operation S401. Operation S401 obtains the preoperative image of the target object, i.e., obtains a coordinate relationship between the target object and the medical imaging device, thereby enabling determination of the coordinate transformation relationships among the three spatial coordinate systems. However, these modifications and changes remain within the scope of the present disclosure.

In some embodiments, position information of a first needle tip point may be determined based on a relative positional relationship between the puncture needle and the surgical robot. More descriptions regarding content herein may be found in FIG. 2 above and the related descriptions.

In some embodiments, the processor may further determine a needle tract contour of the puncture needle based on the target image. The needle tract contour may represent a three-dimensional structure of the puncture needle. In some embodiments, the needle tract contour of the puncture needle may be obtained by performing three-dimensional reconstruction on the puncture needle based on the target image. In some embodiments, the processor may use an image recognition algorithm to obtain the needle tract contour of the puncture needle, the image recognition algorithm may be a machine learning algorithm. Both the three-dimensional reconstruction and the image recognition algorithm may employ common algorithms, which are not limited or elaborated upon in the present disclosure.

Merely by way of example, FIG. 7 is an exemplary schematic diagram illustrating an extracted needle tract contour according to some embodiments of the present disclosure. As shown in FIG. 7, a medical imaging device in the embodiment is the CT scanning device. Diagram a in FIG. 7 is a target image before needle tract contour extraction. Diagram b in FIG. 7 is the target image after needle tract contour extraction. Furthermore, in diagram b of FIG. 7, a target lesion is also annotated, i.e., diagram b in FIG. 7 shows a needle tract contour 701 and a target lesion 702.

In some embodiments, the processor may determine position information of a second needle tip point based on the needle tract contour.

The position information of the second needle tip point represents position information of the needle tip point of a puncture needle in the needle tract contour, e.g., an actual position of the needle tip point. The position information of the second needle tip point may include actual coordinates of the needle tip point. The actual coordinates of the needle tip point may be obtained based on the extracted needle tract contour.

In some embodiments, the processor may determine whether the needle tract contour involves an anomaly based on position information of the first needle tip point and the position information of the second needle tip point.

To facilitate understanding, a process of determining whether the needle tract contour involves an anomaly based on the position information of the first needle tip point and the position information of the second needle tip point is exemplarily described below.

Merely by way of example, the position information of the first needle tip point includes theoretical coordinates of the needle tip point, which are coordinates of the needle tip point reported by a surgical robot. The position information of the second needle tip point includes actual coordinates of the needle tip point, which are obtained based on the extracted needle tract contour. Since the actual coordinates of the needle tip point are in an image coordinate system, when determining whether the needle tract contour involves an anomaly, the actual coordinates of the needle tip point are first converted to a robot coordinate system to obtain converted actual coordinates of the needle tip point. Then, in the robot coordinate system, the converted actual coordinates of the needle tip point are compared with the theoretical coordinates of the needle tip point. If a distance between the converted actual coordinates of the needle tip point and the theoretical coordinates of the needle tip point is greater than a preset error threshold, it is determined that the needle tract contour involves an anomaly. If the distance between the converted actual coordinates of the needle tip point and the theoretical coordinates of the needle tip point is less than or equal to the preset error threshold, it is determined that the needle tract contour does not involve an anomaly.

In some embodiments, when determining whether the needle tract contour involves an anomaly, the theoretical coordinates of the needle tip point in the robot coordinate system may first be converted to the image coordinate system to obtain converted theoretical coordinates of the needle tip point. Then, in the image coordinate system, the converted theoretical coordinates of the needle tip point are compared with the actual coordinates of the needle tip point. If a distance between the converted theoretical coordinates of the needle tip point and the actual coordinates of the needle tip point is greater than a preset error threshold, it is determined that the needle tract contour involves an anomaly. If the distance between the converted theoretical coordinates of the needle tip point and the actual coordinates of the needle tip point is less than or equal to the preset error threshold, it is determined that the needle tract contour does not involve an anomaly.

In some embodiments, in response to the needle tract contour involving an anomaly, the processor may issue a warning regarding the anomaly.

Merely by way of example, the warning regarding the anomaly generated by the processor may be an anomaly warning displayed through the display module. For example, the processor may display needle tract anomaly prompt information on the display module. The needle tract anomaly prompt information may be a prompt window. The needle tract anomaly prompt information may be an anomaly warning issued in other forms, e.g., an alarm module may be installed in the processor to issue an anomaly alarm through the alarm module. The present disclosure does not limit a specific form of the warning regarding the anomaly.

In the embodiment above, the warning regarding the anomaly can promptly alert the user to the anomaly information of the needle tract, thereby avoiding the impact of the needle tract anomaly on puncture accuracy.

In some embodiments, the processor may perform an early warning of a needle tract deviation of the puncture needle during the puncture process.

The needle tract deviation refers to a deviation between an actual puncture path of the needle tract and a target puncture path. More descriptions regarding the actual puncture path and the target puncture path may be found in the related descriptions below.

The early warning is used to remind the user of a potential risk associated with the next puncture in advance. The early warning corresponds to an early warning parameter. In some embodiments, the early warning parameter may include a distance for performing the early warning, a content displayed by the early warning, etc. The distance for performing the early warning refers to a distance between a position of an actual needle tip point of the next puncture and a position of a target needle tip point of the next puncture. The content displayed by the early warning refers to the content related to the next puncture displayed to the user. For example, the content displayed by the early warning may include that the needle tract is about to deviate, the needle tract deviation is too large and requires timely adjustment, etc. In some embodiments, the early warning parameter may further include a preset threshold. More descriptions regarding the content herein may be found in the related descriptions below.

In some embodiments, the processor may determine the early warning parameter based on object information of the next puncture and an importance of a tissue around a puncture position.

The object information of the next puncture is information reflecting related features corresponding to a portion for the next puncture. For example, the object information may include a contour of an organ on a puncture needle trajectory, a contour of a blood vessel, a contour of a bone, etc. In some embodiments, the processor may determine the object information of the next puncture based on the target image or the target puncture path.

The importance of the tissue around the puncture position refers to an importance degree of the tissue around the puncture position to a human body. In some embodiments, a technician or the user may preset a second preset table including importances of various tissues of the human body. The processor may determine the importance of the tissue around the puncture position by querying the second preset table.

In some embodiments, the processor may determine the early warning parameter based on a third preset relationship between the object information of the next puncture, the importance of the tissue around the puncture position, and the early warning parameter. The third preset relationship may be preset based on actual situations and historical experiences. For example, the third preset relationship may include a correlation between the object information of the next puncture and the distance for performing the early warning. The third preset relationship may be preset manually based on experience. As another example, the third preset relationship may further include a correspondence between the distance for performing the early warning, the importance of the tissue around the puncture position, and the content displayed by the early warning. The third preset relationship may be preset manually.

In some embodiments of the present disclosure, by determining the early warning parameter based on the object information of the next puncture and the importance of the tissue around the puncture position, the early warning parameter that better conforms to the actual situation may be determined, so as to provide a more effective early warning and make a reasonable prediction regarding the deviation of the needle tract trajectory.

In some embodiments, the processor may predict an estimated deviation of the next puncture based on the actual puncture path, the target puncture path, and deviation information of the needle tract deviation. The processor may determine the early warning parameter based on the estimated deviation.

The actual puncture path refers to a current actual travel path of a puncture needle tract.

The target puncture path refers to an initial planned puncture path or an ideal travel path of the puncture needle after posture adjustment.

The deviation information of the needle tract deviation refers to information reflecting a deviation degree between the actual puncture path and the target puncture path. For example, the deviation information may include a deviation angle, a deviation distance of the needle tip position of the puncture needle, etc.

In some embodiments, the processor may determine the deviation information based on the actual puncture path and the target puncture path. For example, the processor may determine an angle between a tangent of the actual puncture path and a tangent of the target puncture path as the deviation angle based on the actual puncture path and the target puncture path. The processor may determine a linear distance between a current path endpoint of the actual puncture path and a current path endpoint of the target puncture path as the deviation distance of the needle tip position of the puncture needle.

The estimated deviation refers to a predicted value of the deviation information of the next puncture. For example, the estimated deviation may include an estimated deviation angle, an estimated deviation distance of the puncture needle tip, etc.

In some embodiments, the processor may determine the estimated deviation of the next puncture in a plurality of ways. For example, the processor may determine the deviation information based on the actual puncture path and the target puncture path. The processor may determine the estimated deviation of the next puncture based on the actual puncture path and the deviation information of the needle tract deviation. Merely by way of example, the processor may determine an actual needle tip point position of the next puncture by adding a preset length in a deviation direction of the needle tract deviation based on the deviation angle at a path endpoint of the actual puncture path. The processor may determine the estimated deviation angle and the estimated deviation distance of the puncture needle tip based on the actual needle tip point position of the next puncture and a target needle tip point position of the next puncture. The preset length may be preset manually. The target needle tip point position of the next puncture may be determined based on the target puncture path.

In some embodiments, the processor may determine the early warning parameter based on a fourth preset relationship between the estimated deviation and the early warning parameter. For example, the fourth preset relationship may be that when the estimated deviation exceeds a deviation threshold, the estimated deviation is used as the early warning parameter.

In some embodiments, the processor may determine the early warning parameter based on the actual puncture path, the target puncture path, and the deviation information of the needle tract deviation through a deviation prediction model. In some embodiments, the deviation prediction model may be a machine learning model. For example, the deviation prediction model may be the neural network model, the deep neural network model, etc., or a combination thereof.

In some embodiments, the deviation prediction model may be obtained by training using third training samples and third labels. Each of the third training samples may include a sample actual puncture path, a sample target puncture path, and sample deviation information from historical records. The third label of the third training sample is an actual early warning parameter corresponding to the third training sample from historical surgeries. The third training samples may include a plurality of types of data. For example, during a historical surgery, if corresponding processing was performed according to the early warning after the early warning, and no abnormality occurred subsequently, such data may be constructed as a third training sample and a corresponding third label. The corresponding third label is the actual early warning parameter corresponding to the early warning. As another example, during a historical surgery, if no corresponding processing was performed for the early warning after the early warning, and an abnormality occurred subsequently, such data may be constructed as a third training sample and a corresponding third label. The corresponding third label is the actual early warning parameter corresponding to the early warning.

In some embodiments, the third training samples and the third labels may further be constructed based on whether a deviation occurring in a subsequent puncture in the historical surgery is greater than a preset threshold. For example, if the deviation occurring in the subsequent puncture is greater than the preset threshold, the actual needle tip point position of the puncture needle at a time when the deviation is greater than the preset threshold is recorded. A distance between the actual needle tip point position and the target needle tip point position of the puncture needle at the time when the deviation is greater than the preset threshold is used as a sample distance for performing the early warning in the third label. The preset threshold refers to a threshold used to limit the deviation of the needle tract deviation. The preset threshold may be determined based on experiences.

A specific training process of the deviation prediction model is similar to a specific training process of an exposure range prediction model. More descriptions regarding the content herein may be found in FIG. 3 and the related descriptions.

In some embodiments of the present disclosure, by determining the early warning parameter based on the actual puncture path, the target puncture path, and the deviation information of the needle tract deviation, a possible deviation in a next operation can be reasonably estimated in combination with the actual situation, the user can be promptly reminded, and unnecessary injury during a surgery can be avoided.

In some embodiments of the present disclosure, by providing the early warning for the needle tract deviation of the puncture needle during the puncture process, an occurrence of an excessive needle tract deviation can be effectively avoided, an accuracy of a travel path of the puncture needle during the surgery can be improved, and a surgical efficiency can be enhanced.

In some embodiments, the needle tip point and the predicted target point may be located on a target image of a same slice or on target images of different slices. When the needle tip point and the predicted target point are annotated onto the target image of the same slice, a connection line between the needle tip point and the predicted target point is further displayed on the annotated target image.

In some embodiments, if the needle tip point and the predicted target point are located in a same slice of the target object, the needle tip point and the predicted target point are annotated onto the same target image. In this case, the annotated image displayed by a display module may be the target image annotated with the needle tip point and the predicted target point.

FIG. 8 is a schematic diagram illustrating an interface where a needle tip point and a predicted target point are located in a same target image according to some embodiments of the present disclosure. As shown in FIG. 8, in the embodiment, a medical imaging device is the CT scanning device. Four different scan images are displayed in total, a needle tip point 801 and a predicted target point 802 are located on a same scan image (i.e., a target image).

Merely by way of example, when the needle tip point and the predicted target point are annotated on the same target image, a connection line between the needle tip point and the predicted target point is further displayed on an annotated image (i.e., an annotated target image). For example, in FIG. 8, a connection line 803 between the needle tip point 801 and the predicted target point 802 is further displayed. The connection line 803 is able to represent a direction of a needle tract when the puncture needle performs a puncture in a current puncture state, so that the user is able to more clearly determine a position of the needle tract, and then more accurately determine whether to perform the puncture based on the current puncture state.

It is able to be understood that, if the image shown in FIG. 8 is a second image in a needle insertion state, then the needle tip point 801 and the predicted target point 802 are located on the same second image. Therefore, a needle insertion process is a same-slice needle insertion. In this case, both the needle tip point and the predicted target point are on a -729.125 mm slice. The two annotation points are connected by a connection line 803. The connection line 803 may be a dashed line to represent a subsequent needle insertion path of the needle tip, facilitating the doctor to determine tissue information that will be passed through in a subsequent puncture process. As a needle insertion depth increases, the needle tip point 801 will continuously approach the predicted target point 802 along the dashed line until the puncture is completed.

In some embodiments, if the needle tip point and the predicted target point are located in two different slices of the target object, the needle tip point and the predicted target point are annotated onto two different target images.

FIG. 9 is a schematic diagram illustrating an interface where a needle tip point and a predicted target point are located in different target images according to some embodiments of the present disclosure. As shown in FIG. 9, in the embodiment, a medical imaging device is the CT scanning device. In the embodiment, four different scan images are displayed in total, a needle tip point 901 and a predicted target point 902 are located on two different target images.

It is able to be understood that, if the image shown in FIG. 9 is a second image in the needle insertion state, then the needle tip point 901 and the predicted target point 902 are located on different second images. Therefore, the needle insertion process is a cross-slice needle insertion. In this case, a real-time needle tip point 901 is on a -729.125 mm slice, and the predicted target point 902 is on a -727.875 mm slice. As the needle insertion depth increases, the real-time needle tip point 901 will be punctured from an initial slice (i.e., the -729.125 mm slice) to a target slice (i.e., the -727.875 mm slice) until the puncture is completed.

It is able to be understood that, in FIG. 8 and FIG. 9, to facilitate user differentiation, markers with different colors and shapes may be used to annotate the needle tip point and the predicted target point.

In some embodiments, markers with a same shape but different colors may be used to annotate the needle tip point and the predicted target point, or markers with a same color but different shapes may be used to annotate the needle tip point and the predicted target point. The present disclosure does not limit a specific form of the markers.

In some embodiments, when the needle tip point and the predicted target point are annotated on target images of different slices, a slice number of an objective target point coordinate may be further displayed on an annotated image, and the objective target point is annotated onto the target image of a slice corresponding to the objective target point.

The objective target point refers to a spatial point where the puncture needle enters the needle and ultimately requires the needle tip point of the puncture needle to be reached. The objective target point coordinate refers to a coordinate of the spatial point that the needle tip point of the puncture needle needs to reach ultimately. The slice number of the objective target point coordinate refers to a slice number corresponding to the target image of the slice corresponding to the objective target point.

In some embodiments, when the deviation information of the needle tract deviation is greater than a preset threshold, a processor is able to display a predicted needle tract trajectory on the annotated image in advance. More descriptions regarding the preset threshold may be found in the related descriptions above.

In some embodiments, the processor may determine the predicted needle tract trajectory through the deviation prediction model. In some embodiments, when the processor trains the deviation prediction model, the processor may include an actual needle tract trajectory in the third label corresponding to the third training sample in the historical record. The actual needle tract trajectory may be constructed based on a needle tract trajectory of the puncture needle within a time period after a historical current time point measured in a historical surgery corresponding to the third training sample.

In some embodiments, the preset threshold may include a deviation angle threshold, a deviation distance threshold of a needle tip position of the puncture needle, etc. In some embodiments, the preset threshold may be set manually based on experience.

In some embodiments of the present disclosure, by displaying the predicted needle tract trajectory in advance when the deviation information of the needle tract deviation is greater than the preset threshold, a more intuitive reference may be provided to the doctor through the annotated image, thereby avoiding the excessive deviation of the needle tract deviation and affecting the judgment of the doctor.

In some embodiments, the processor may determine the preset threshold based on a puncture depth, a puncture rate, and an importance of tissue around a puncture position.

More descriptions regarding the puncture depth and the puncture rate may be found in FIG. 2 and the related descriptions. More descriptions regarding the importance of the tissue around the puncture position may be found in the related descriptions above.

In some embodiments, the processor may determine the preset threshold based on a fifth preset relationship between the puncture depth, the puncture rate, and the importance of the tissue around the puncture position and the preset threshold. The fifth preset relationship may be preset based on the actual situation and historical experiences. For example, the fifth preset relationship may be that the deeper the puncture depth, the faster the puncture rate, and the higher the importance of the tissue around the puncture position, the smaller the preset threshold (the deviation angle threshold, the deviation distance threshold).

In some embodiments of the present disclosure, by determining the preset threshold based on the puncture depth, the puncture rate, and the importance of the tissue around the puncture position, an accuracy of the determined preset threshold can be further improved.

In some embodiments, the early warning parameter may further include the preset threshold.

In some embodiments, when the early warning parameter includes the preset threshold, the processor may determine the early warning parameter including the preset threshold through the deviation prediction model. In some embodiments, when the early warning parameter includes the preset threshold and the processor trains the deviation prediction model, the processor may add a sample puncture depth, a sample puncture rate, and an importance of sample tissue around a sample puncture position in the historical record corresponding to the third training sample into the third training sample for training. The third label corresponding to the third training sample includes an actual preset threshold. The actual preset threshold may be constructed based on deviation data measured in the historical surgery corresponding to the third training sample.

In some embodiments of the present disclosure, by including the preset threshold in the early warning parameter, a determination of the preset threshold may be made more flexible and more reasonable, enabling the early warning parameter to more accurately reflect a real situation, thereby providing more reliable prompt information.

In some embodiments of the present disclosure, by displaying the actual needle tract trajectory on the annotated image in advance when the deviation information of the needle tract deviation is greater than the preset threshold, it helps the user judge a current situation when the needle tract deviates, thereby improving the accuracy of the puncture process.

In some embodiments, while displaying the annotated image through the display module, the processor further displays an adjacent scan image of the annotated image through the display module. By displaying the adjacent scan image, a movement of the needle tip point and the predicted target point of the puncture needle in different slices may be seen during the puncture. Meanwhile, when the needle tip point and the predicted target point move onto the adjacent scan image, they can be quickly displayed.

It is able to be understood that the adjacent scan image of the annotated image may be a scan image corresponding to a slice before a slice corresponding to the annotated image, or may be a scan image corresponding to a slice after the slice corresponding to the annotated image. Furthermore, the scan image corresponding to the slice before the slice corresponding to the annotated image may include one or more, and the scan image corresponding to the slice after the slice corresponding to the annotated image may also include one or more. The present disclosure does not limit this.

For example, in the embodiment shown in FIG. 8, in addition to the annotated image annotated with the needle tip point 801 and the predicted target point 802, three scan images adjacent to the annotated image are also displayed in FIG. 8.

As another example, in the embodiment shown in FIG. 9, in addition to two different annotated images annotated with the needle tip point 901 and the predicted target point 902, FIG. 9 also shows two scan images adjacent to the annotated images. Each of the two scan images is adjacent to one annotated image.

It should be understood that when the user determines, based on an image displayed by the display module, that the predicted target point does not coincide with a target lesion, or determines that the predicted target point is located outside a contour of the target lesion, a posture adjustment operation of the puncture needle is required. For ease of understanding, a posture adjustment process in the puncture method in an embodiment of the present disclosure is exemplarily described below with reference to the accompanying drawings.

In some embodiments, the puncture assistance system provides two posture adjustment manners: automatic posture adjustment and manual posture adjustment. The user may select one of the automatic posture adjustment and the manual posture adjustment based on the clinical situation. The automatic posture adjustment and the manual posture adjustment are exemplarily described below, respectively.

When the user selects the automatic posture adjustment, the puncture assistance device provides adjustment data for the posture adjustment.

In some embodiments, in response to receiving a first posture adjustment instruction, the processor may determine lesion position information of a target lesion of a target object based on a plurality of scan images. First posture adjustment data is obtained based on the lesion position information and puncture status information. The first posture adjustment data represents posture change data of the puncture needle when the predicted target point is adjusted to a lesion position corresponding to the lesion position information. A first movement instruction corresponding to the first posture adjustment data is sent to a surgical robot. The first movement instruction is used to control the surgical robot to move the puncture needle to adjust a posture of the puncture needle.

The first posture adjustment instruction indicates the automatic posture adjustment, i.e., the user selects an automatic posture adjustment function. In the embodiment, the processor obtains the lesion position of the target lesion by performing image recognition on the plurality of scan images. The lesion position of the target lesion is the lesion position information. The first posture adjustment data is obtained based on the lesion position information and the puncture status information. The automatic posture adjustment may be implemented through the manner above, thereby saving posture adjustment time of the user.

In some embodiments, before sending the first movement instruction corresponding to the first posture adjustment data to the surgical robot, the processor may be further configured to display the first posture adjustment data. In response to receiving an instruction from the user confirming adoption of the first posture adjustment data, the processor sends the first movement instruction corresponding to the first posture adjustment data to the surgical robot. That is, in the automatic posture adjustment process in the embodiment, specific posture adjustment may only be performed after the user confirmation, which is equivalent to adding an operation for the doctor review and confirmation, thereby improving the reliability of the puncture method and the accuracy of the puncture result.

In some embodiments, during the automatic posture adjustment process, the processor may further determine whether needle withdrawal is required before posture adjustment. In some embodiments, before sending the first movement instruction corresponding to the first posture adjustment data to the surgical robot, the processor is further configured to determine whether at least one of the following needle withdrawal conditions is satisfied. The needle withdrawal conditions include: a puncture depth of the puncture needle is greater than a first threshold, or a distance between the predicted target point and the lesion position is greater than a second threshold, or posture adjustment based on the first posture adjustment data causes the puncture needle to interfere with a target tissue or organ of the target object.

In some embodiments, in response to determining that the at least one of the needle withdrawal conditions is satisfied, the processor sends a second movement instruction to the surgical robot to control the surgical robot to move the puncture needle to withdraw the puncture needle. The second movement instruction is used to control the surgical robot to move the puncture needle to withdraw the puncture needle. After the needle withdrawal is completed, the processor sends the first movement instruction corresponding to the first posture adjustment data to the surgical robot. The first movement instruction is used to control the surgical robot to move the puncture needle to perform posture adjustment.

It is able to be understood that in the case of automatic posture adjustment, if the puncture depth of the puncture needle is greater than the first threshold, it indicates that the puncture needle has been inserted to a relatively deep depth, and direct posture adjustment may cause significant harm to the target object. If the distance between the predicted target point and the lesion position is greater than the second threshold, it indicates that the predicted target point deviates by a large angle. In this case, if posture adjustment is performed directly, the puncture needle requires a large adjustment range, which may cause significant harm to the target object. Interference between the puncture needle and the target tissue or organ of the target object refers to that the puncture needle may contact the target tissue or organ of the target object, thereby potentially harming the contacted target tissue or organ. Therefore, controlling the surgical robot to withdraw the puncture needle first under at least one of the three needle withdrawal conditions and then perform posture adjustment can sufficiently reduce harm to the target object and improve the safety of automatic posture adjustment. The target tissue or organ refers to an important tissue or organ of the target object. For example, the target tissue or organ may be an important tissue or organ, such as a blood vessel, a bone, or a nerve.

In some embodiments, during the automatic posture adjustment, before sending the first movement instruction corresponding to the first posture adjustment data to the surgical robot, the processor is configured to perform the following operations: if the puncture assistance device determines that the puncture depth of the puncture needle is less than or equal to the first threshold, and the distance between the predicted target point and the lesion position is less than or equal to the second threshold, and direct posture adjustment based on the first posture adjustment data will not cause the puncture needle to interfere with the target tissue or organ of the target object, the processor sends the first movement instruction to the surgical robot, the first movement instruction is used to control the surgical robot to move the puncture needle to perform posture adjustment.

It is able to be understood that in the case of automatic posture adjustment, if the puncture depth of the puncture needle is less than or equal to the first threshold, it indicates that the puncture depth of the puncture needle is relatively shallow, and direct posture adjustment causes less harm to the target object. If the distance between the predicted target point and the lesion position is less than or equal to the second threshold, it indicates that the predicted target point deviates by a small angle. In this case, direct posture adjustment requires only a small adjustment range for the puncture needle, causing less damage to the target object. No interference between the puncture needle and the target tissue or organ of the target object refers to the puncture needle not contacting the target tissue or organ of the target object. Therefore, when all three conditions above are satisfied, the surgical robot may be controlled to perform posture adjustment directly without needle withdrawal.

Merely by way of example, after the puncture assistance device determines that needle withdrawal is unnecessary and posture adjustment may be performed directly, before sending the first movement instruction corresponding to the first posture adjustment data to the surgical robot, the puncture assistance device displays the first posture adjustment data in the display module. If an instruction to adopt the first posture adjustment data is received, the first movement instruction corresponding to the first posture adjustment data is sent to the surgical robot. That is, in the automatic posture adjustment process in the embodiment, specific posture adjustment may only be performed after the user confirmation, which is equivalent to adding an operation for the doctor review and confirmation, thereby improving the reliability of the puncture method and the accuracy of the puncture result.

In some embodiments, the processor may determine the first threshold based on a puncture range, a lesion characteristic, and the objective target point coordinate.

More descriptions regarding the puncture range and the lesion characteristic may be found in FIG. 2 and the related descriptions.

In some embodiments, the processor may determine the first threshold based on a sixth preset relationship between the puncture range, the lesion characteristic, the objective target point coordinate, and the first threshold. For example, the sixth preset relationship may be: the larger the puncture range, the farther the organ where the lesion is located from the body surface, and the deeper a coordinate position of the predicted target point, the larger the first threshold.

In some embodiments, the processor may determine the first threshold by performing matching in a fourth vector database based on the puncture range, the lesion characteristic, and the predicted target point coordinate. For example, the processor may construct a fourth feature vector based on the puncture range, the lesion characteristic, and the predicted target point coordinate, perform matching in the fourth vector database, and determine a first threshold corresponding to a fourth reference vector with the highest similarity as a corresponding first threshold.

The fourth vector database includes the fourth reference vectors and the corresponding first thresholds each of which corresponds to one of the fourth reference vectors. A fourth reference vector is composed of an actual historical puncture range, a historical lesion characteristic, and a historical predicted target point coordinate in the historical record. The first threshold corresponding to the fourth reference vector may be determined in a manner similar to determining a fourth label described later. The similarity may be calculated based on a cosine distance, a Euclidean distance, etc.

In some embodiments, the processor may determine the first threshold by using a first threshold prediction model based on the puncture range, the lesion characteristic, and the predicted target point coordinate. In some embodiments, the first threshold prediction model may be a machine learning model. For example, the first threshold prediction model may be the neural network model, the deep neural network model, etc., or a combination thereof.

In some embodiments, the first threshold prediction model may be obtained by training based on fourth training samples and fourth labels. A fourth training sample may be a sample puncture range, a sample lesion characteristic and a sample predicted target point coordinate in the historical records. The fourth label of the fourth training sample may be an actual first threshold corresponding to a historical surgery with the best needle withdrawal compensation effect (e.g., timely needle withdrawal, high surgery completion rate), and the fourth label of the fourth training sample may be constructed based on the fourth training sample. In some embodiments, the processor may construct the fourth label of the fourth sample based on the puncture depth of the puncture needle and a corresponding puncture anomaly occurrence frequency in the historical surgery corresponding to the fourth training sample.

A training manner of the first threshold prediction model is similar to a training manner of an exposure range prediction model. More description regarding the training manner of the first threshold prediction model may be found in FIG. 3 and the related descriptions.

In some embodiments of the present disclosure, by determining the first threshold based on the puncture range, the lesion characteristic, and the predicted target point coordinate, the first threshold may be set more specifically and more conform to the actual situation, thereby enabling a more reasonable judgment on whether to perform a needle withdrawal operation.

In some embodiments, the processor may determine the second threshold based on a seventh preset relationship between the puncture rate, the position information of the first needle tip point, the lesion characteristic, and the second threshold. For example, the seventh preset relationship may be: the faster the puncture rate, the closer a position of the first needle tip point is to the lesion, and the smaller a size of the lesion in the lesion characteristic, the smaller the second threshold may be set to avoid the puncture needle proceeding to an incorrect target point (e.g., a non-lesion portion).

In some embodiments, the processor may determine the second threshold by performing matching through a fifth vector database based on the puncture rate, the position information of the first needle tip point, and the lesion characteristic. For example, the processor may construct a fifth feature vector based on the puncture rate, the position information of the first needle tip point, and the lesion characteristic, perform matching in the fifth vector database, and determine the second threshold corresponding to a fifth reference vector in the fifth vector database with the highest similarity as a corresponding second threshold.

The fifth vector database includes the fifth reference vectors and corresponding second thresholds each of which corresponds to one of the fifth reference vectors. A fifth reference vector is constructed based on the actual historical puncture rate in the historical records, position information of a historical first needle tip point, and a historical lesion characteristic. The corresponding second threshold may be determined in a manner similar to determining a fifth label described later. The similarity may be calculated based on a cosine distance, a Euclidean distance, etc.

In some embodiments, the processor may determine the second threshold based on the puncture rate, the position information of the first needle tip point, and the lesion characteristic through a second threshold prediction model. In some embodiments, the second threshold prediction model may be a machine learning model. For example, the second threshold prediction model may be a neural network model, a deep neural network model, etc., or any combination thereof.

In some embodiments, the second threshold prediction model may be obtained by training based on fifth training samples and fifth labels. A fifth training sample may be a sample puncture rate, sample position information of a first needle tip point, and a sample lesion characteristic in the historical records. The fifth label may be an actual second threshold corresponding to a historical surgery with a best needle withdrawal compensation effect (e.g., a best subsequent posture adjustment effect). The fifth label of the fifth training sample may be constructed based on the fifth training sample. In some embodiments, the processor may construct the fifth label based on a distance between the predicted target point and the lesion position in a historical surgery corresponding to the fifth training sample and a corresponding puncture anomaly occurrence frequency.

A training manner of the second threshold prediction model is similar to a training manner of an exposure range prediction model. More descriptions regarding the training manner of the second threshold prediction model may be found in FIG. 3 and the related description.

In some embodiments of the present disclosure, by determining the second threshold based on the puncture rate, the position information of the first needle tip point, and the lesion characteristic enables the second threshold to be set more specifically and more conform to an actual situation, thereby enabling a more reasonable determination of whether to perform the needle withdrawal operation.

In some embodiments, during the manual posture adjustment, the user controls the surgical robot to drive the puncture needle to perform the posture adjustment. Merely by way of example, the processor may further: in response to receiving a second posture adjustment instruction, send a third movement instruction to the surgical robot to control the surgical robot to adjust a posture of the puncture needle. The second posture adjustment instruction may be generated based on a posture adjustment action performed by the user at a surgical robot master hand. In the embodiment, the provided posture adjustment process may satisfy usage requirements with high user autonomy and facilitate manual posture adjustment by the user.

It is able to be understood that, in a specific posture adjustment process, the posture adjustment may include: the same-slice posture adjustment, the cross-slice posture adjustment, and free-mode posture adjustment. The same-slice posture adjustment, the cross-slice posture adjustment, and the free-mode posture adjustment are exemplarily described below with reference to the accompanying drawings. It is able to be understood that, in the following examples, the medical imaging device is the CT scanning device.

FIG. 10 is a schematic diagram illustrating an interface of same-slice posture adjustment according to some embodiments of the present disclosure.

(1) Same-slice posture adjustment.

The same-slice posture adjustment refers to a scenario where a predicted target point before the posture adjustment and a predicted target point after the posture adjustment are on a same transverse section (i.e., on a same tomographic image). In this case, only a same-slice angle (i.e., a left-right deflection angle) needs to be changed, and a cross-slice angle (i.e., a head-foot deflection angle) does not need to be changed. As shown in FIG. 10, diagram a in FIG. 10 is a tomographic image before the posture adjustment, and diagram b in FIG. 10 is a tomographic image after the posture adjustment. As shown in diagram a in FIG. 10, a predicted target point 1001 before the posture adjustment and a target lesion 1002 are located on a same tomographic image but do not coincide. Therefore, the same-slice posture adjustment is required. After the same-slice angle is changed, a position of the predicted target point correspondingly changes on a Dicom image of the same transverse section (Z-axis coordinate -729.125 mm). Diagram b in FIG. 10 is the tomographic image after the posture adjustment. As shown in diagram b in FIG. 10, a predicted target point 1003 after the posture adjustment coincides with the target lesion 1002, the posture adjustment is completed.

In some embodiments, the same-slice posture adjustment mode may be set. When it is determined that the predicted target point and the target lesion are located on the same tomographic image, during the automatic posture adjustment or the manual posture adjustment, the user selects the same-slice posture adjustment mode. In this case, only the same-slice angle may be changed during the posture adjustment process, and the cross-slice angle is unable to be changed. The same-slice posture adjustment mode, by restricting changes to only the same-slice angle, can avoid the problem of accidental cross-slice during the posture adjustment process.

FIG. 11 is a schematic diagram illustrating an interface for cross-slice posture adjustment according to some embodiments of the present disclosure.

(2) Cross-slice posture adjustment.

The cross-slice posture adjustment refers to a scenario where a predicted target point before the posture adjustment and a predicted target point after the posture adjustment exhibit a cross-slice phenomenon in the transverse section (i.e., they are located on different tomographic images). The same-slice angle (left-right deflection angle) may remain unchanged or change, and the cross-slice angle (head-foot deflection angle) changes. As shown in FIG. 11, diagram a in FIG. 11 is a tomographic image before the posture adjustment, and diagram b in FIG. 11 is a tomographic image after the posture adjustment. As shown in diagram a in FIG. 11, there is no target lesion on the tomographic image where the predicted target point 1101 before the posture adjustment is located. Therefore, the predicted target point needs to be adjusted onto a tomographic image displaying the target lesion. In this case, the cross-slice posture adjustment is required. After the cross-slice angle is changed, a Z-axis coordinate of the predicted target point 1102 is updated from -729.125 mm to - 727.875 mm, and the predicted target point 1102 after the posture adjustment coincides with the target lesion 1103, the posture adjustment is completed.

In some embodiments, the cross-slice posture adjustment mode may be set. When it is determined that the predicted target point and the target lesion are located on different tomographic images, during the automatic posture adjustment or the manual posture adjustment, the user selects the cross-slice posture adjustment mode. In this case, only the cross-slice angle may be changed during the posture adjustment process, and the same-slice angle is unable to be changed. A setting of the cross-slice posture adjustment mode enables rapid adjustment of the predicted target point to the tomographic image where the target lesion is located during the posture adjustment process, thereby improving posture adjustment efficiency.

It is able to be understood that, after adjusting the predicted target point to the tomographic image where the target lesion is located, if the predicted target point and the target lesion are still unable to coincide, the same-slice posture adjustment mode may be selected at this time to complete a final posture adjustment in the same-slice posture adjustment mode.

(3) Free-mode posture adjustment.

The free-mode posture adjustment refers to a scenario where a doctor is able to manipulate a surgical robot master hand to perform both same-slice posture adjustment and cross-slice posture adjustment simultaneously. After a posture of the needle tract is changed, a position of the predicted target point may change either within the same slice or across slices. This is not repeated here.

In some embodiments, before sending the third movement instruction to the surgical robot, the processor may be further configured to: in response to receiving a needle withdrawal action performed by the user at the surgical robot master hand, send a fourth movement instruction to the surgical robot to control the surgical robot to move the puncture needle to withdraw the puncture needle.

The fourth movement instruction refers to an instruction for controlling the surgical robot to perform the needle withdrawal. In some embodiments, the processor may generate the fourth movement instruction based on the needle withdrawal action performed by the user.

After introducing the posture adjustment process, a triggering process of the medical imaging device in the embodiments of the present disclosure is described below.

In some embodiments, the processor is further configured to: in response to receiving a signal that the surgical robot is performing a puncture operation, send a scan instruction to the processor to cause the processor to control the medical imaging device to perform scanning.

In some embodiments, the puncture assistance device automatically controls the medical imaging device to perform a scanning operation.

Merely by way of example, in a scenario where the puncture assistance device automatically controls the medical imaging device to perform the scanning operation, the processor, upon receiving the signal that the surgical robot is performing the puncture operation, the surgical robot sends the scan instruction to the processor to control the medical imaging device to perform the scanning operation. The puncture operation includes a posture adjustment operation or a needle insertion operation. The posture adjustment operation is used to adjust a posture of the puncture needle. The needle insertion operation is used to adjust a puncture depth of the puncture needle. In the embodiment herein, the puncture assistance device may automatically control the medical imaging device to perform scanning, thereby improving synchronization between the medical imaging device and the puncture operation and enhancing the real-time nature of the scanning.

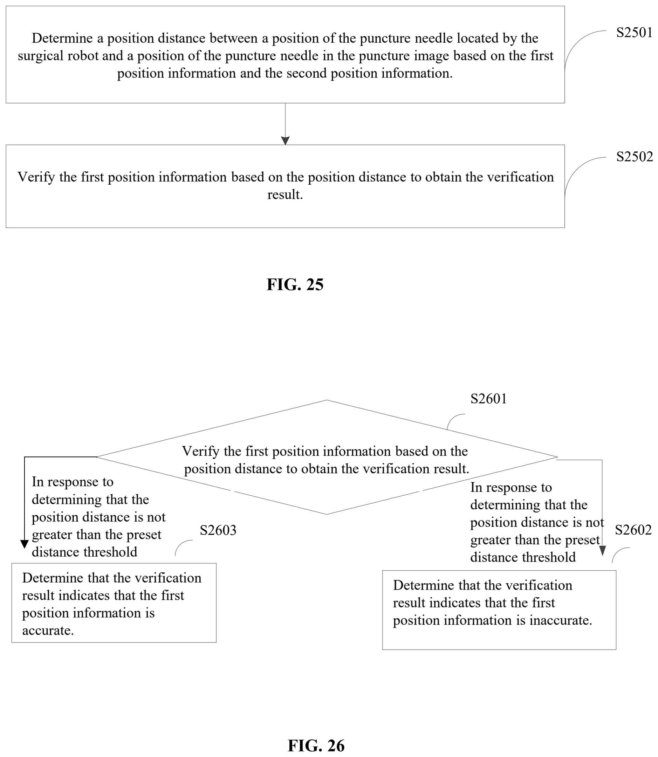

In some embodiments, the puncture operation may further include the needle withdrawal operation. More descriptions regarding the puncture operation may be found in FIG. 26 and the related descriptions.

In some embodiments, when the surgical robot master hand receives a first puncture operation instruction, it prompts the puncture assistance device to control the medical imaging device to perform the scanning operation, thereby achieving automatic control of the medical imaging device for real-time exposure scanning during the puncture operation process.

In some embodiments, a time interval between the surgical robot master hand receiving the puncture operation instruction and the surgical robot slave hand executing the puncture operation instruction is utilized to perform medical image scanning, this enables the medical image scanning to be performed before the puncture operation is executed, thereby avoiding blind puncture caused by the puncture operation having been executed without medical image scanning being performed.

In some embodiments, a time interval between a processor sending a sixth movement instruction corresponding to a second puncture operation instruction to the surgical robot slave hand and the surgical robot slave hand executing the sixth movement instruction is utilized to perform the medical imaging scanning, this enables the medical imaging scanning to be performed before the puncture operation is executed, thereby avoiding blind puncture caused by the puncture operation having been executed without performing the medical image scan.

In some embodiments, the processor may determine whether a puncture action is completed by detecting a motion state of the surgical robot slave hand. For example, the determination may be made based on information reported by robot components. When it is detected that the puncture action is completed, the processor controls the medical imaging device to stop exposure.

In some embodiments, the processor may determine whether the puncture operation is completed by detecting whether a real-time needle tip point position reported by the surgical robot has changed.

More descriptions regarding the automatic scanning, such as the first puncture operation instruction, the second puncture operation instruction, and the sixth movement instruction, may be found in FIG. 1 and the related descriptions in the embodiment of the puncture assistance system, which will not be repeated here.

In some embodiments, the processor may determine a frequency for obtaining the first image based on a lesion characteristic and a patient characteristic. More descriptions regarding the lesion characteristic may be found in FIG. 2 and the related descriptions.

The patient characteristic refers to a relevant feature reflecting vital signs of a patient. For example, the patient characteristic may include a patient heart rate, a patient normal breathing frequency, etc.

In some embodiments, the processor may determine the frequency for obtaining the first image through an eighth preset relationship among the lesion characteristic, the patient characteristic, and the frequency for obtaining the first scan image. The eighth preset relationship may be preset based on actual situations and historical experiences. For example, the eighth preset relationship may include a correlation between the lesion characteristic and the frequency for obtaining the first scan image. Merely by way of example, according to the lesion characteristic, when an organ where the lesion is located is greatly affected by respiration, it indicates that a deformation degree of the lesion is large. At this time, the processor may increase the frequency for obtaining the first image to more accurately reflect an impact of respiration on organ deformation. Conversely, the frequency for obtaining the first image may be reduced.

As another example, the eighth preset relationship may include a correlation between the patient characteristic and the frequency for obtaining the first scan image. Merely by way of example, according to the patient characteristic, when a heart rate and a breathing frequency of the patient are high, it indicates that a frequency of organ deformation is fast. At this time, the processor may increase the frequency for obtaining the first image to more accurately reflect the impact of respiration on organ deformation. Conversely, the frequency for obtaining the first image may be reduced.

In some embodiments, the processor may also determine the frequency for obtaining the first image based on the lesion characteristic and the patient characteristic by performing matching through a sixth vector database. For example, the processor may construct the sixth vector database based on actual historical lesion characteristics, historical patient characteristics, and corresponding frequencies for obtaining the first image in historical records, construct a sixth feature vector based on a current lesion characteristic and a current patient characteristic, perform matching in the sixth vector database, and determine the frequency for obtaining the first image corresponding to a sixth reference vector in the sixth vector database with a highest similarity as a frequency for obtaining the first image corresponding to the current situation.

The sixth vector database includes the sixth reference vectors and corresponding frequencies for obtaining the first image each of which corresponds to one of the sixth reference vectors. A sixth reference vector is constructed based on the actual historical lesion characteristic and the historical patient characteristic in the historical records. The corresponding frequency for obtaining the first image may be determined based on an actual frequency for obtaining the first image in historical surgeries. The similarity may be calculated based on a cosine distance, a Euclidean distance, etc.

In some embodiments, the processor may also determine the frequency for obtaining the first image based on the lesion characteristic and the patient characteristic through a frequency prediction model. In some embodiments, the frequency prediction model may be a machine learning model. For example, the frequency prediction model may be a Neural Networks (NN) model, a Deep Neural Networks (DNN) model, etc., or any combination thereof.

In some embodiments, the frequency prediction model may be obtained by training based on sixth training samples and sixth labels. A sixth training sample may be a sample patient characteristic and sample lesion characteristics in the historical records. The sixth label of the sixth training sample may be constructed based on the sixth training sample. For example, the processor may construct the sixth label of the sixth training sample based on an accuracy of a target guidance image corresponding to the sixth training sample in a historical surgery, e.g., using the frequency for obtaining the first image corresponding to the historical surgery with the highest accuracy of the target guidance image as the sixth label. As another example, the processor may construct the sixth label of the sixth training sample based on an overlap degree between a second image and the first image in the historical surgery corresponding to the sixth training sample, e.g., using the frequency for obtaining the first image from a historical surgery where the second image is entirely encompassed within the first image as the sixth label.

A specific training process of the frequency prediction model is similar to a specific training process of an exposure range prediction model. More descriptions regarding the specific training process of the frequency prediction model may be found in FIG. 3 and the related descriptions.

In some embodiments of the present disclosure, by determining the frequency for obtaining the first image based on the lesion characteristic and the patient characteristic, a more reasonable frequency for obtaining the first image may be determined. This enables the obtained first image to better reflect an actual situation of a target portion during the first time period, which helps in subsequently determining a more accurate target image.

FIG. 12 is an exemplary flowchart illustrating a process of determining a target image according to some embodiments of the present disclosure. In some embodiments, a process 1200 is executed by the processor. As shown in FIG. 12, the process 1200 may include the following operations.

In 1210, one or more deformation parameters may be determined based on a preoperative image and a first image.

More descriptions regarding the preoperative image and the first image may be found in FIG. 1 and the related descriptions.

The deformation parameters refer to parameters related to registration between a target portion in the preoperative image and a target portion in the first image. For example, the deformation parameters may include elastic registration parameters, rigid registration parameters, etc. The registration between the target portion in the preoperative image and the target portion in the first image may include elastic registration, rigid registration, etc. The elastic registration refers to registration of image deformation. The rigid registration refers to registration involving translation and rotation of coordinate axes. Merely by way of example, the rigid registration may involve setting the target portion in the first image as a reference image, and performing spatial transformation (e.g., a series of rotations, translations, etc.) on the target portion in the preoperative image based on the reference image, so that corresponding pixel points in the preoperative image achieve spatial consistency with pixel points in the reference image. Correspondingly, the rigid registration parameter may reflect a spatial transformation relationship (e.g., a rotation angle, a translation distance, etc.) between two images. In some embodiments, the rigid registration parameter may include parameters such as a horizontal displacement, a vertical translation, and a rotation angle during the rigid registration process. In some embodiments, the rigid registration parameter may be represented by a value, a vector, a matrix, etc. The elastic registration may involve performing a spatial transformation on the target portion in the preoperative image, which has irregular internal deformation, according to the target portion in the first image. Correspondingly, the elastic registration parameter may refer to a transformation parameter corresponding to a deformed portion in medical images when performing registration on the medical images (e.g., geometric shape changes of organ tissues or a target area in radiotherapy). The radiotherapy or spontaneous autonomous movement of organs may cause changes in a size, shape, volume, etc., of internal tissues and organs, resulting in some irregular internal deformations in the acquired medical images. For example, as respiration changes, the shape and the size of the target portion of the target object gradually change.

In some embodiments, at a time when the medical imaging device acquires the preoperative image, the processor may also acquire a respiratory amplitude point corresponding to the target object at the time through a respiratory gating device, and determine the respiratory amplitude point as a target respiratory amplitude point.

The respiratory amplitude point refers to a parameter used to reflect a fluctuation degree of the chest wall when the target object breathes. The target respiratory amplitude point refers to the respiratory amplitude point of the target object when the preoperative image is acquired.

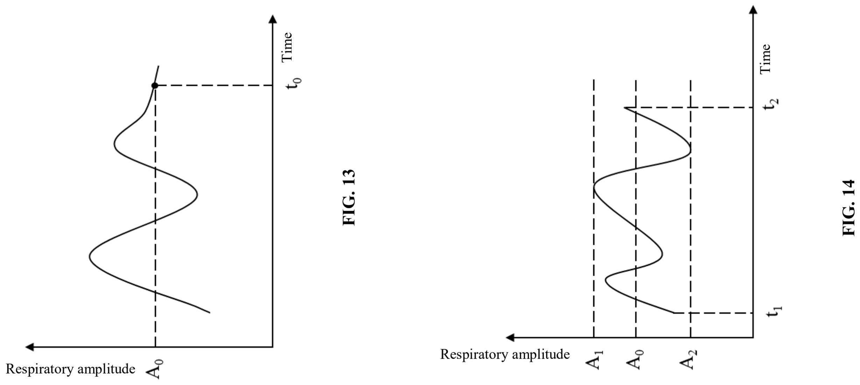

FIG. 13 is an exemplary schematic diagram illustrating a target respiratory amplitude point according to some embodiments of the present disclosure.

As shown in FIG. 13, a horizontal axis represents time, and the vertical axis represents a respiratory amplitude point of a target object collected by the processor. When a preoperative image of the target object is acquired by a medical imaging device at time t0, a respiratory amplitude point A0 corresponding to the target object is the target respiratory amplitude point.

To enable real-time interventional guidance during a subsequent surgery execution process, during a first time period, at a time when the medical imaging device acquires a first image, the processor may also acquire a respiratory amplitude point corresponding to the target object during the first time period through the respiratory gating device. Each first image corresponds to one respiratory amplitude point.

In some embodiments, the processor acquires the first image through the medical imaging device at a preset time during the first time period. For example, the processor acquires a first image at a time specified by the user within the first time period. By acquiring the first image at the preset time, a radiation dose to the target object caused by scanning can be reduced.

In some embodiments, the processor may obtain a plurality of first images through the medical imaging device at preset respiratory amplitude points based on the respiratory amplitude points of the target object. For example, the processor acquires a first image every time a preset respiratory amplitude point variation occurs, until acquisition for a complete respiratory cycle of the target object is completed.

The plurality of first images acquired by the foregoing manner in some embodiments of the present disclosure can show respiratory changes of the target object under different respiratory amplitude points within the complete respiratory cycle, so that deformation parameters determined later can more completely reflect deformation of the target portion of the target object within the respiratory cycle.

In some embodiments, when the processor acquires at least one first image, a respiratory pressure value and/or a respiratory amplitude of the target object needs to satisfy a preset condition. The preset condition refers to a condition preset to trigger the processor to acquire the first image. For example, the preset condition may include that the respiratory pressure value is greater than a first pressure value and less than a second pressure value. The preset condition may also include other conditions. For example, the preset condition may include that the respiratory amplitude is greater than a first amplitude and less than a second amplitude. The first pressure value, the second pressure value, the first amplitude, and the second amplitude may be default values, preset values, etc. Merely by way of example, the preset condition may include that the respiratory pressure value is greater than 1.375 kpa and less than 1.782 kpa.

In some embodiments, when the respiratory pressure value and/or the respiratory amplitude of the target object satisfy the preset condition and the processor acquires at least one first image, the respiratory amplitude point of the target object should be within a target respiratory amplitude range. The target respiratory amplitude range refers to a range that includes the target respiratory amplitude point. An upper bound and a lower bound of the range may be determined based on a maximum value and a minimum value of the respiratory amplitude points of the target object within the first time period during which the processor acquires the first image. For example, the processor may determine the maximum value of the respiratory amplitude points of the target object within the first time period during which the first image is acquired as the upper bound of the target respiratory amplitude range.

FIG. 14 is an exemplary schematic diagram illustrating a target respiratory amplitude range according to some embodiments of the present disclosure.

Merely by way of example, when the respiratory pressure value and/or the respiratory amplitude of the target object satisfy the preset condition and at least one first image is acquired within the first time period t1~t2, the respiratory amplitude points of the target object acquired by the processor may be as shown in FIG. 14. When the preoperative image is acquired, the target respiratory amplitude point of the target object is A0. Within the first time period t1~t2, the maximum value of the respiratory amplitude points of the target object is A1, and the minimum value is A2. The target respiratory amplitude range is [A2, A1]. The target respiratory amplitude point A0 is within the target respiratory amplitude range [A2, A1].

In some embodiments of the present disclosure, by including the target respiratory amplitude point within the target respiratory amplitude range enables registration of the preoperative image and the first image. During a subsequent interventional process, interventional guidance can be completed as long as the respiratory amplitude point of the target object is within the target respiratory amplitude range, thereby avoiding multiple scans of the target object to find a specific respiratory amplitude point during the process, which would result in the target object receiving excessive radiation dose.

In some embodiments, the target respiratory amplitude range includes at least one respiratory amplitude range segment. Each respiratory amplitude range segment includes a corresponding respiratory amplitude point, and within the respiratory amplitude range segment, deformation of the target portion of the target object is less than a preset deformation threshold. At least one respiratory amplitude range segment may entirely cover or partially cover the target respiratory amplitude range. The processor may determine a respiratory amplitude range segment in which a respiratory amplitude point corresponding to a certain first image is located based on the respiratory amplitude point, thereby determining the respiratory amplitude range segment corresponding to the first image.

During the second time period, when the processor acquires a plurality of second images, it is able to prompt that the respiratory amplitude point of the target object should be within the target respiratory amplitude range. The prompting manner may be a plurality, such as a voice prompt, etc.

FIG. 16 is an exemplary schematic diagram illustrating an ideal respiratory amplitude point of a target object during a surgical procedure according to some embodiments of the present disclosure. Merely by way of example, as shown in FIG. 16, during an execution of the interventional procedure within a second time period t3~t4, the target object is prompted that a respiratory amplitude point should be within the target respiratory amplitude range [A2, A1] and as close as possible to the target respiratory amplitude point A0.

In some embodiments of the present disclosure, by having the respiratory amplitude point of the target object within a range close to a range of the target respiratory amplitude point during the second time period, deformation differences of tissues and organs between the preoperative image and the second image caused by respiratory motion can be reduced, which is beneficial for improving subsequent registration accuracy of the preoperative image and the second image.

In some embodiments, during the second time period, the processor may acquire the respiratory amplitude point corresponding to the target object in real time through the respiratory gating device, and acquire the second image under the same respiratory amplitude parameter based on the respiratory amplitude parameter corresponding to each first image. The respiratory amplitude parameter refers to a parameter related to respiratory information of the target object. In some embodiments, the respiratory amplitude parameter refers to a respiratory amplitude point or a respiratory amplitude range segment to which the respiratory amplitude point belongs. Deformation of the target portion of the target object at various respiratory amplitude points within the respiratory amplitude range segment is less than a preset deformation threshold. For example, when only one first image exists, the processor may perform acquisition on the target object based on the respiratory amplitude parameter corresponding to the first image to obtain at least one second image, so as to ensure that a deformation parameter corresponding to the second image exists. More descriptions regarding the deformation parameter may be found in related content below in the present disclosure. It is able to be understood that a count of first images and a count of second images do not need to correspond. For example, the count of the first images may be only one, but the processor may acquire a plurality of second images through the medical imaging device within the respiratory amplitude point or the respiratory amplitude range segment corresponding to the first image.

In some embodiments, the processor may obtain a plurality of first images under different respiratory amplitude parameters, and obtain a plurality of deformation parameters by performing registration between each of the plurality of first images and the preoperative image.

It is able to be understood that, when the plurality of first images exist and the respiratory amplitude parameters (e.g., respiratory amplitude range segments) corresponding to the plurality of first images are sufficient to cover a complete respiratory cycle of the target object, the processor may obtain the second image at any time point within the second time period, and a corresponding deformation parameter exists for the second image obtained at any time point.

The processor may process the preoperative image and the at least one first image based on registration software to determine at least one deformation parameter.

In some embodiments, the processor may process the preoperative image and the at least one first image based on a registration deep learning model to determine the at least one deformation parameter.

The registration deep learning model may be a machine learning model for determining the deformation parameter. For example, the registration deep learning model may be a Deep Neural Network (DNN) model.

It should be understood that, to enable real-time scanning, image reconstruction, and image transmission of the target portion, the registration deep learning model may be a lightweight (e.g., a simplified network structure) model. The processor may also perform quantization, pruning, knowledge distillation, etc., on the registration deep learning model to ensure that the registration deep learning model is able to perform real-time processing of the preoperative image and the at least one first image. To further improve the calculation rate and processing performance of the registration deep learning model, high-performance image processing hardware (graphics processing unit, GPU) may be used when deploying the registration deep learning model.

In some embodiments, an input of the registration deep learning model may include the preoperative image and a certain first image. An output of the registration deep learning model may include a deformation parameter corresponding to the respiratory amplitude point of the first image. When the count of the first images is a plurality, the registration deep learning model may determine a deformation parameter corresponding to the respiratory amplitude point of each first image by processing multiple inputs of the preoperative image and different first images.

In some embodiments, the processor can determine a respiratory amplitude range segment corresponding to each respiratory amplitude point, and determine the deformation parameter corresponding to the respiratory amplitude point of a certain first image as the deformation parameter of the respiratory amplitude range segment corresponding to the respiratory amplitude point. Each respiratory amplitude range segment includes a corresponding respiratory amplitude point, and within the respiratory amplitude range segment, deformation of the target portion of the target object is less than a preset deformation threshold. When a certain respiratory amplitude range segment corresponds to deformation parameters determined based on the plurality of first images, an average value of the plurality of deformation parameters may be determined as the deformation parameter corresponding to the respiratory amplitude range segment.

In some embodiments, the registration deep learning model may be obtained by training a plurality of seventh training samples with seventh labels. The specific training manner is similar to the training manner of the exposure range prediction model. More descriptions may be found in FIG. 3 and the related descriptions.

In some embodiments, the seventh training samples include sample preoperative images and a plurality of sample first images of different sample target objects. A seventh label of a seventh training sample may include a sample deformation parameter corresponding to each sample first image. In some embodiments, the seventh training samples are obtained based on historical data (e.g., historical preoperative images and a plurality of historical first images). The seventh labels are determined based on manual registration processing of the sample preoperative images and the plurality of sample first images.

In some embodiments of the present disclosure, by using a lightweight registration deep learning model and performing quantization, pruning, knowledge distillation, etc., on the lightweight registration deep learning model, real-time processing of the preoperative image and the plurality of first images is achieved, occupying more memory is avoided and calculation rate and processing performance are improved. Furthermore, by considering the influence of various factors, the determination of the deformation parameters is efficient and accurate, thereby avoiding errors from manual determination.

When the processor acquires only one first image during the first time period, the processor determines one deformation parameter based on the first image and the preoperative image, and stores a correspondence between a respiratory amplitude point or a respiratory amplitude range segment of the first image and the deformation parameter in a storage device.

In some embodiments of the present disclosure, by acquiring only one first image to determine a deformation parameter corresponding to a certain respiratory amplitude point or respiratory amplitude range segment through registration, the calculation load during both the preliminary registration and the guidance in the subsequent interventional surgery is reduced, the preparation time before the interventional surgery is shortened, and a quick transition into the interventional surgery is facilitated.

When the processor acquires a plurality of first images under different respiratory amplitude points during the first time period, the processor determines one deformation parameter based on each first image and the preoperative image, and stores a correspondence between the respiratory amplitude point or the respiratory amplitude range segment of each first image and the deformation parameter in the storage device, to facilitate subsequent direct determination of a corresponding deformation parameter based on a respiratory amplitude point or a respiratory amplitude range segment corresponding to a second image. For example, the respiratory amplitude points corresponding to the plurality of first images are C0, D0, and E0, respectively, and the corresponding deformation parameters are XC, XD, and XE, respectively. When the processor acquires the second image and the respiratory amplitude point of the target object is C0, then the deformation parameter corresponding to the second image is XC. As another example, the respiratory amplitude points corresponding to the plurality of first images are C0, D0, and E0, respectively, and the corresponding deformation parameters are XC, XD, and XE, respectively. If the respiratory amplitude range segments corresponding to the plurality of first images are [C1, C2], [D1], D2], and [E1, E2], respectively. Herein, the respiratory amplitude point C0 is within the respiratory amplitude range segment [C1, C2], so the deformation parameter corresponding to the respiratory amplitude range segment [C1, C2] is XC. The respiratory amplitude point D0 is within the respiratory amplitude range segment [D1, D2], so the deformation parameter corresponding to the respiratory amplitude range segment [D1, D2] is XD. The respiratory amplitude point E0 is within the respiratory amplitude range segment [E1, E2], so the deformation parameter corresponding to the respiratory amplitude range segment [E1, E2] is XE. When the processor acquires a certain second image, the respiratory amplitude point of the target object is C3, and the respiratory amplitude point C3 is within the respiratory amplitude range segment [C1, C2], then the deformation parameter corresponding to the second image is XC.

In some embodiments of the present disclosure, by acquiring the plurality of first images for registration enables determination of deformation parameters corresponding to a plurality of different respiratory amplitude points or respiratory amplitude range segments, which is beneficial for increasing the update frequency of the target guidance image during the subsequent surgical procedure and ensuring the accuracy of image registration, thereby making the determined target guidance image more accurate.

In 1220, a target image for the second time period is determined based on the one or more deformation parameters, the preoperative image, and the at least one second image.

More descriptions regarding the second image and the target object may be found in FIG. 2 and the related descriptions.

In some embodiments, the target image for the second time period may include a target guidance image. The target guidance image refers to a real-time image used to guide an interventional surgery. For example, the target guidance image may include the puncture needle, a lesion, a target organ, etc. The target guidance image may be a real-time image used to guide the puncture needle during the interventional procedure in the surgical execution process.

The processor determines at least one target guidance image for the second time period based on at least one deformation parameter, the preoperative image, and at least one second image, using image fusion software or other suitable manners.

In some embodiments, when there is only one deformation parameter X0, the processor determines a respiratory amplitude point A3 or a respiratory amplitude range segment [A4, A5] corresponding to the deformation parameter X0, the respiratory amplitude point A3 is within the respiratory amplitude range segment [A4, A5]. During the surgical execution process, when the respiratory amplitude point of the target object is the respiratory amplitude point A3 or within the respiratory amplitude range segment [A4, A5], the processor acquires the at least one second image and determines the deformation parameter X0 as the deformation parameter corresponding to the at least one second image. The processor performs deformation processing on the preoperative image based on the deformation parameter X0, fuses the preoperative image with the at least one second image, and determines the target guidance image corresponding to the at least one second image. In some embodiments of the present disclosure, by determining the target guidance image for guiding the interventional surgery only within the respiratory amplitude point or respiratory amplitude range segment corresponding to one deformation parameter, it is able to reduce the calculation load during guidance in the interventional surgery.

In some embodiments, when a count of the deformation parameters is a plurality, the processor obtains a respiratory amplitude parameter corresponding to each deformation parameter. During the surgical execution process, when the target object is under the same respiratory amplitude parameter, the processor acquires the at least one second image. For each second image, the processor determines a deformation parameter corresponding to the second image based on the respiratory amplitude parameter corresponding to the second image, performs deformation processing on the preoperative image using the corresponding deformation parameter, and fuses the preoperative image with the second image to determine the target guidance image corresponding to the second image. Merely by way of example, a deformation parameter corresponding to a respiratory amplitude range segment [C1, C2] is XC, a deformation parameter corresponding to a respiratory amplitude range segment [D1, D2] is XD, and a deformation parameter corresponding to a respiratory amplitude range segment [E1, E2] is XE. If respiratory amplitude points corresponding to a plurality of second images F1, F2, F3 acquired by the processor are C3, D3, and E3, respectively, and the respiratory amplitude point C3 is within the respiratory amplitude range segment [C1, C2], the respiratory amplitude point D3 is within the respiratory amplitude range segment [D1, D2], and the respiratory amplitude point E3 is within the respiratory amplitude range segment [E1, E2], then the deformation parameters corresponding to the second images F1, F2, F3 are XC, XD, and XE, respectively. The processor performs deformation processing on a preoperative image G0 based on the deformation parameters XC, XD, and XE, respectively, to obtain deformed preoperative images G1, G2, and G3, respectively. The processor fuses the deformed preoperative images G1, G2, and G3 with the corresponding second images F1, F2, and F3, respectively, to obtain target guidance images H1, H2, and H3. It is able to be understood that when the plurality of deformation parameters cover a complete respiratory cycle of the target object, a user adjusts an acquisition frequency of the second images (e.g., 30 Hz) as needed, thereby enabling continuous output of the target guidance images for real-time guidance of the interventional surgery. In some embodiments of the present disclosure, by using the plurality of deformation parameters, an output frequency of the target guidance images is ensured, thereby guaranteeing the real-time nature and accuracy of the interventional guidance.

In some embodiments, the target guidance image includes a first guidance image and/or a second guidance image.

The first guidance image is represented by a scan image of at least one slice related to the at least one second image and the preoperative image. For example, the first guidance image is represented by a two-dimensional image of at least one slice of a target portion of the target object. The first guidance image is represented by two-dimensional images of a plurality of slices of the target portion of the target object. The user may adjust the display to show two-dimensional images of different slices of the target portion as needed.

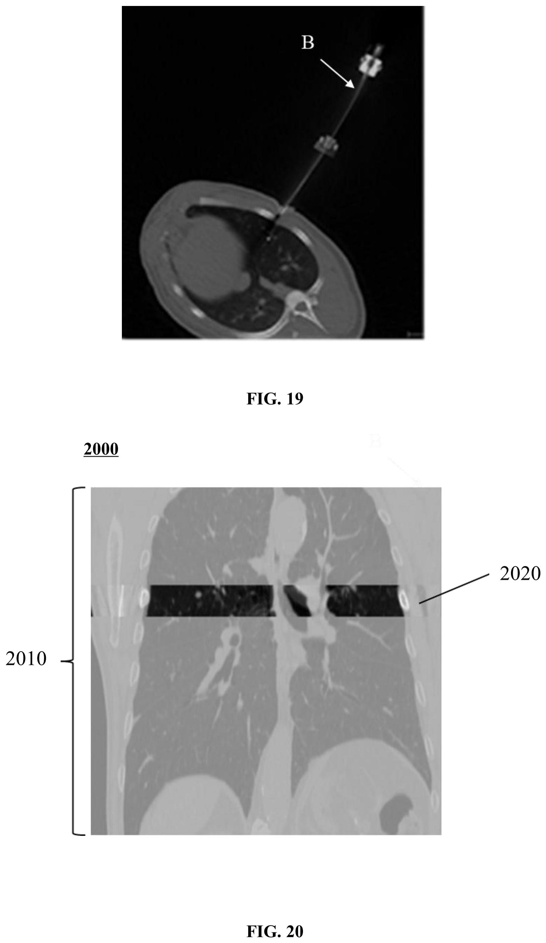

FIG. 20 is an exemplary schematic diagram illustrating a first guidance image according to some embodiments of the present disclosure. Merely by way of example, as shown in FIG. 20, a first guidance image 2000 is a two-dimensional image of a certain slice of the target portion. The first guidance image 2000 is a two-dimensional image obtained by fusing a deformed preoperative image 2010 of the slice with a second image 2020 of the slice.

The second guidance image is represented by a three-dimensional model related to at least one second image and the preoperative image. For example, the second guidance image is represented by a three-dimensional model including a puncture needle and related portions. The related portions include the target portion of the target object and surrounding tissues or organs.

FIG. 21 is an exemplary schematic diagram illustrating a second guidance image according to some embodiments of the present disclosure. Merely by way of example, as shown in FIG. 21, a second guidance image 2100 is represented by a three-dimensional model of a puncture needle 2120 and related portions 2110.

In some embodiments, the display module is further configured to display the first guidance image and/or the second guidance image.

In some embodiments of the present disclosure, by dividing the target guidance image into the first guidance image and the second guidance image, one or more types of images are displayed according to user requirements, thereby facilitating adaptation to different user operation habits. It should be understood that the target guidance image determined in some embodiments of the present disclosure is only used to provide guidance for the operations of a user or a doctor during the interventional surgery.

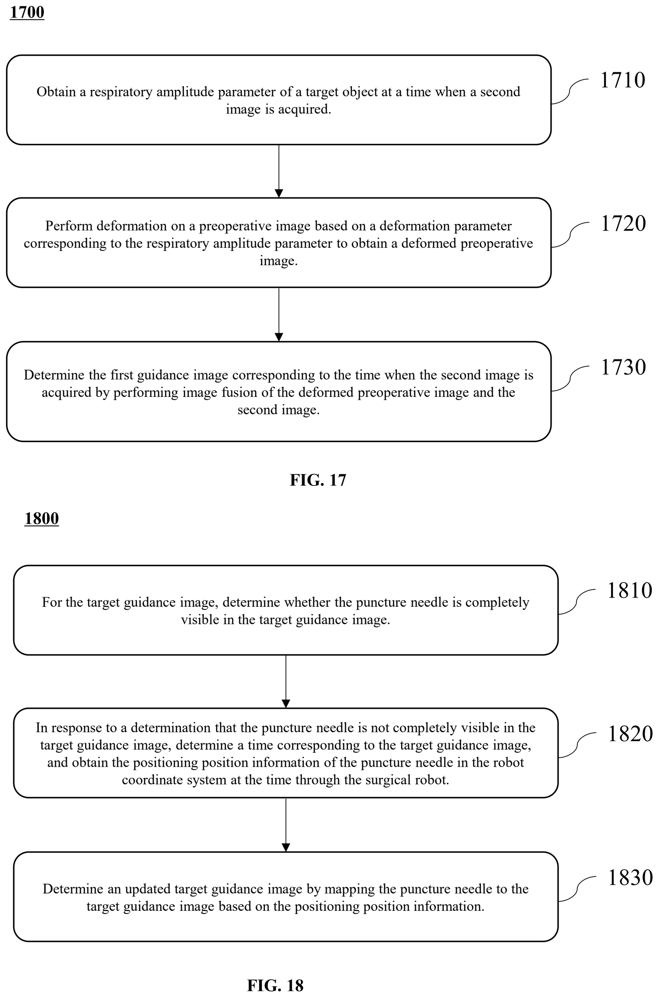

In some embodiments, for each second image, the processor may obtain a respiratory amplitude parameter of a target object at a time when a second image is acquired; performs deformation processing on the preoperative image based on a deformation parameter corresponding to the respiratory amplitude parameter to obtain a deformed preoperative image, and determines the first guidance image corresponding to the time when the second image is acquired by performing image fusion of the deformed preoperative image and the second image. More descriptions regarding the above embodiments may be found in FIG. 17 and the related descriptions.

In some embodiments, for each second image, the processor performs segmentation processing on the second image to determine a puncture needle image in the second image, obtains a respiratory amplitude parameter of the target object at the time when the second image is acquired, performs segmentation and three-dimensional reconstruction on the preoperative image to obtain a three-dimensionally reconstructed result, performs deformation on the three-dimensionally reconstructed result based on the deformation parameter corresponding to the respiratory amplitude parameter to obtain an initial model corresponding to a deformed preoperative image, and determines the second guidance image corresponding to the time of the second image based on the puncture needle image and the initial model. More descriptions regarding the above embodiments may be found in FIG. 17 and the related descriptions.

In some embodiments of the present disclosure, the preparation time before the execution of the interventional surgery is utilized to complete image registration. Real-time mapping from the preoperative image to the second image is performed during the surgery. The preoperative image obtained from a largerange scan before the surgery is fused into the second image obtained from a narrow-range scan during the surgery, with dynamic updates performed simultaneously. This achieves real-time tracking of organ deformation while providing a large field of view for surgical guidance, the spatial sense during the intervention is enhanced, and the radiation dose is controlled, thereby improving the safety of the surgery.

FIG. 17 is an exemplary flowchart illustrating a process of determining a first guidance image according to some embodiments of the present disclosure. In some embodiments, a process 1700 may be executed by the processor. As shown in FIG. 17, the process 1700 may include the following operations.

In some embodiments, the count of one or more deformation parameters exceeds 1, each of the deformation parameters corresponds to a respiratory amplitude parameter.

In 1710, a respiratory amplitude parameter of a target object at a time when a second image is acquired may be obtained.

More descriptions regarding the respiratory amplitude parameter may be found in FIG. 12 and the related descriptions.

In some embodiments, when a medical imaging device acquires a plurality of second images, the processor may further acquire a respiratory amplitude point corresponding to the target object during a second time period through a respiratory gating device, and each second image corresponding to a respiratory amplitude point. The processor may determine a respiratory amplitude range segment in which the respiratory amplitude point is located based on the respiratory amplitude point corresponding to a certain second image, thereby determining the respiratory amplitude range segment corresponding to the second image.

In 1720, deformation on a preoperative image may be performed based on a deformation parameter corresponding to the respiratory amplitude parameter to obtain a deformed preoperative image.

In some embodiments, for each second image, the processor may determine a deformation parameter corresponding to the second image by accessing a storage device based on a respiratory amplitude parameter corresponding to the second image and a correspondence between the respiratory amplitude parameter and the deformation parameter, elastic deformation processing and/or rigid deformation processing on a target portion in the preoperative image is performed, and the deformed preoperative image is obtained.

In some embodiments, when the processor determines the deformation parameter, the processor may further consider other factors affecting organ deformation.

In some embodiments, the other factors affecting organ deformation may include: an influence of other organ activities on organ deformation (e.g., an influence of heartbeat on organ deformation), and an influence of fat and muscle content of a patient on organ deformation.

In some embodiments, the processor may construct a third preset table relating other factors affecting organ deformation to variation conditions of the deformation parameter, and determine the deformation parameter by querying the third preset table. The third preset table includes a correspondence between other factors affecting organ deformation and variation conditions of the deformation parameter, and may be preset manually based on experience.

In some embodiments of the present disclosure, by considering the influence of other factors affecting organ deformation on organ shape, the finally determined deformation parameter can better conform to the actual situation, thereby making the deformed image more accurate and more referential.

In 1730, the first guidance image corresponding to the time when the second image is acquired may be determined by performing image fusion of the deformed preoperative image and the second image.

More descriptions regarding the first guidance image may be found in FIG. 12 and the related descriptions.

In some embodiments, the processor may perform image fusion on a target portion in the deformed preoperative image and a target portion in the second image to determine the first guidance image when the second image is acquired during the second time period.

During an interventional procedure, image registration is completed using preparation time before the interventional procedure is performed, and deformation parameters corresponding to various respiratory amplitude parameters are saved according to respiratory amplitude points of the target object. During the procedure execution, the deformation parameter corresponding to the respiratory amplitude parameter of the respiratory amplitude point of the target object at the time when the second image is acquired is directly used to perform deformation processing on the preoperative image to obtain the deformed preoperative image, and the image fusion of the deformed preoperative image and the second image is performed, then a guidance image at the time when the second image is acquired is determined, thereby reducing computational load for image registration and image fusion and ensuring real-time performance of the guidance image.

In some embodiments, for each second image, the processor may perform segmentation processing on the second image to determine a puncture needle image in the second image; obtain a respiratory amplitude parameter of the target object at a time when the second image is acquired; perform segmentation and three-dimensional reconstruction on the preoperative image to obtain a three-dimensionally reconstructed result; perform deformation on the three-dimensionally reconstructed result based on the deformation parameter corresponding to the respiratory amplitude parameter to obtain an initial model corresponding to a deformed preoperative image; and determine a second guidance image corresponding to the time of the second image based on the puncture needle image and the initial model. More descriptions regarding the second guidance image may be found in FIG. 12 and the related descriptions. More descriptions regarding obtaining the respiratory amplitude parameter of the target object when the second image is acquired may be found in FIG. 17 and the related descriptions.

In some embodiments, for each second image, the processor may process the second image using an image segmentation software to obtain the puncture needle image.

In some embodiments, for each second image, the processor may process the second image based on a segmentation deep learning model to determine the puncture needle image in the second image.

FIG. 19 is an exemplary schematic diagram illustrating a puncture needle in a second image according to some embodiments of the present disclosure.

As shown in FIG. 19, the processor may process the second image shown in FIG. 19 based on the segmentation deep learning model to segment and obtain an image of a puncture needle B.

The segmentation deep learning model may be a machine learning model for determining the puncture needle. For example, the segmentation deep learning model may be a Deep Neural Network (DNN) model.

It should be understood that, to enable real-time segmentation of the second image to obtain a puncture needle image, the segmentation deep learning model may be a lightweight (e.g., a simplified network structure) model. The processor may further perform quantization, pruning, knowledge distillation, etc., on the segmentation deep learning model to ensure that the segmentation deep learning model may perform real-time processing on the plurality of second images. To further improve the computational rate and processing performance of the segmentation deep learning model, the high-performance image processing hardware may be used when deploying the segmentation deep learning model.

In some embodiments, an input of the segmentation deep learning model may include the second image; an output may include the puncture needle image in the second image

It should be understood that the processor may process second images of different slices respectively through the segmentation deep learning model to obtain puncture needle images in the second images of the different slices, and perform three-dimensional reconstruction on the puncture needle images to obtain a three-dimensional puncture needle image.

In some embodiments, the segmentation deep learning model may be obtained by training based on a plurality of eighth training samples with eighth labels. A training manner of the segmentation deep learning model is similar to a training manner of an exposure range prediction model. More descriptions regarding the training manner of the exposure range prediction model may be found in FIG. 3 and the related descriptions.

In some embodiments, a eighth training sample may include a sample second image of a sample target object; a eighth label of the eighth training sample may include a sample puncture needle image. In some embodiments, the eighth training sample may be obtained based on historical data (e.g., a historical second image); the eighth label may be determined based on manual segmentation processing of the sample second image.

In some embodiments of the present disclosure, by using a lightweight segmentation deep learning model and performing quantization, pruning, knowledge distillation, etc., real-time processing of the plurality of second images can be achieved, occupying more memory is avoided and computational rate and processing performance are improved. Furthermore, by considering the influence of various factors, the segmented puncture needle image is more efficient and accurate, thereby avoiding errors from manual determination.

In some embodiments, the processor may segment organ tissues of the preoperative images of different slices based on the segmentation deep learning model, and perform three-dimensional reconstruction on the organ tissues segmented from all slices to obtain the initial model corresponding to the preoperative image.

In some embodiments, the processor may perform deformation processing on the initial model corresponding to the preoperative image based on the deformation parameter corresponding to the second image to obtain a deformed preoperative image, and obtain an initial model corresponding to the deformed preoperative image.

It should be understood that the order of segmentation, three-dimensional reconstruction, and deformation processing performed by the processor on the preoperative image may be different. For example, the processor may perform deformation on the preoperative images of different slices based on the deformation parameter corresponding to the second image to obtain deformed preoperative images of the different slices. Further, the processor may segment organ or tissues of the deformed preoperative images of the different slices based on the segmentation deep learning model, and perform three-dimensional reconstruction on the organ or tissues segmented from all slices to obtain an initial model corresponding to the deformed preoperative image.

In some embodiments, the processor may determine the second guidance image corresponding to the time when the second image is acquired based on the puncture needle image and the initial model corresponding to the deformed preoperative image through a formula. The second guidance image may be determined by the following formula (4):

$$V_{Curent} = F_{Curent} + DVF_{A'}(F_{Before}) \quad (4)$$

$V_{Curent}$ denotes the second guidance image corresponding to the time when the second image is acquired, $F_{Curent}$ denotes the puncture needle image in the second image, $F_{Before}$ denotes the initial model corresponding to the preoperative image, and $DVF_{A'}(F_{Before})$ denotes the initial model corresponding to the deformed preoperative image. That is, for each second image, the corresponding deformation parameter is determined based on the corresponding respiratory amplitude parameter, the initial model $F_{Before}$ corresponding to the preoperative image is deformed using the deformation parameter to obtain the initial model $DVF_{A'}(F_{Before})$ corresponding to the deformed preoperative image, and the e initial model $DVF_{A'}(F_{Before})$ corresponding to the deformed preoperative image is fused with the puncture needle image $F_{Curent}$ in the second image to obtain the second guidance image $V_{Curent}$ at the time when the second image is acquired.

In some embodiments, by performing segmentation processing on the second image to obtain a real-time image of the puncture needle during the interventional procedure, deformation of the puncture needle can be effectively reflected. By performing segmentation and three-dimensional reconstruction on the organ tissues of the preoperative images of different slices to obtain the initial model corresponding to the preoperative image, deforming the preoperative image to obtain a deformed preoperative image, and performing image fusion of the deformed preoperative image and the segmented puncture needle image not only makes the determined guidance image more accurate and better reflect the actual situation of the target portion during the procedure, but also presents the second guidance image in a three-dimensional form, thereby providing a greater sense of three-dimensionality and spatial perception.

FIG. 18 is an exemplary flowchart illustrating a process of determining an updated target guidance image according to some embodiments of the present disclosure. In some embodiments, a process 1800 may be executed by the processor. As shown in FIG. 18, the process 1800 may include the following operations.

In 1810, for the target guidance image, whether the puncture needle is completely visible in the target guidance image is determined.

It should be understood that, due to the limited scanning range used to acquire the second image, the puncture needle may not be fully visible in the second image, or the puncture needle may even be absent the second image.

In some embodiments, the processor may process a second image corresponding to the target guidance image using the image recognition software to determine whether the puncture needle is completely visible in the target guidance image.

In some embodiments, for target guidance image, the processor may determine a puncture needle image in the second image corresponding to the target guidance image based on the second image corresponding to the target guidance image; and determine whether the puncture needle is completely visible in the target guidance image based on the puncture needle image and a standard puncture needle image.

In some embodiments, the processor may process the second image corresponding to the target guidance image based on the segmentation deep learning model to determine the puncture needle image in the second image. More descriptions regarding the segmentation deep learning model may be found in FIG. 17 and the related descriptions.

The standard puncture needle image refers to a complete image of the puncture needle used as a reference. The standard puncture needle image may be pre-stored in the storage device.

In some embodiments, the processor may obtain the standard puncture needle image by accessing the storage device based on the puncture needle image obtained by segmenting the second image corresponding to the guidance image, the puncture needle image obtained by segmenting the second image is a segmented puncture needle image, perform an image comparison between the segmented puncture needle image and the standard puncture needle image to determine whether the segmented puncture needle image is complete, thereby determining whether the puncture needle is completely visible in the target guidance image.

In some embodiments of the present disclosure, by comparing the puncture needle image obtained by segmenting the second image during the aforementioned image fusion with the pre-stored standard puncture needle image, to not only to determine whether the puncture needle is completely visible in the target guidance image but also to reduce the computational load of the processor and shorten the update time of the target guidance image.

In some embodiments, for the target guidance image, the processor may determine first position information of the puncture needle in an image coordinate system based on positioning position information of the puncture needle in a robot coordinate system at a time corresponding to the target guidance image; obtain third position information of the second image corresponding to the target guidance image in the image coordinate system; and determine whether the puncture needle is completely visible in the target guidance image based on the first position information and the third position information.

The positioning position information refers to parameters related to the position of the puncture needle in the robot coordinate system. For example, the positioning position information may include three-dimensional coordinates of a start point and an end point of the puncture needle in the robot coordinate system.

In some embodiments, the processor may obtain the positioning position information of the puncture needle in the robot coordinate system at the time corresponding to the target guidance image by accessing the storage device of a surgical robot.

The first position information refers to parameters related to the position of the puncture needle in the image coordinate system.

In some embodiments, the processor may convert the positioning position information of the puncture needle in the robot coordinate system into the first position information in the image coordinate system based on a mapping relationship between the robot coordinate system and the image coordinate system.

The third position information refers to parameters related to the position of the second image in the image coordinate system. For example, the third position information may include three-dimensional coordinates of a boundary contour of the second image in the image coordinate system.

In some embodiments, the processor may determine whether the puncture needle is completely visible in the target guidance image by determining whether the first position information of the puncture needle is completely within a coverage range of the third position information of the second image. When the first position information of the puncture needle is completely within the coverage range of the third position information of the second image, the processor determines that the puncture needle is completely visible in the target guidance image; when a portion of the first position information of the puncture needle is within the coverage range of the third position information of the second image, the processor determines that the puncture needle is partially visible in the target guidance image; when the first position information of the puncture needle is not within the coverage range of the third position information of the second image, the processor determines that the puncture needle is absent from the target guidance image.

In some embodiments of the present disclosure, by mapping the positioning position information of the puncture needle in the robot coordinate system to obtain the second position information of the puncture needle in the image coordinate system, and determining the display status of the puncture needle in the target guidance image based on the third position information of the second image in the image coordinate system, it is applicable not only to situations where the puncture needle is incompletely visible in the target guidance image but also to situations where the puncture needle is absent from the target guidance image.

In some embodiments, if the puncture needle image obtained by segmenting the second image completely displays the entire puncture needle, the processor may directly fuse the segmented puncture needle image with the deformed preoperative image to determine the target guidance image at the time when the second image is acquired.

In 1820, in response to a determination that the puncture needle is not completely visible in the target guidance image, a time corresponding to the target guidance image is determined, and the positioning position information of the puncture needle in the robot coordinate system at the time is obtained through the surgical robot.

In some embodiments, the processor may obtain the positioning position information of the puncture needle in the robot coordinate system at the time corresponding to the target guidance image by accessing the storage device of the surgical robot.

In 1830, an updated target guidance image is determined by mapping the puncture needle to the target guidance image based on the positioning position information.

In some embodiments, the processor may convert the positioning position information of the puncture needle in the robot coordinate system into the first position information in the image coordinate system based on the mapping relationship between the robot coordinate system and the image coordinate system, correct the first position information based on second position information of the puncture needle, and fuse the pre-stored standard puncture needle image into the second image. It should be understood that, during the surgical procedure, the puncture needle may deform, causing the real-time image of the puncture needle to differ from the standard puncture needle image pre-stored in the storage device. Therefore, after determining the first position information of the puncture needle in the image coordinate system, if the puncture needle is absent from the target guidance image, the processor may directly correct the first position information or the positioning position information based on the second position information, map the pre-stored standard puncture needle image to the target guidance image, and determine the updated target guidance image. If the puncture needle is partially displayed but not completely visible in the target guidance image, the pre-stored standard puncture needle image is unable to be directly used to replace the puncture needle image obtained by segmenting the second image; the processor needs to supplement a missing portion of the puncture needle image obtained by segmenting the second image based on the standard puncture needle image to determine the updated target guidance image. More descriptions regarding image fusion may be found in FIG. 17 and the related descriptions.

It is worth noting that, during the interventional procedure, the puncture needle image may deform; if a pre-stored complete puncture needle image is used directly, the target guidance image may fail to reflect a deformation of the puncture needle. In contrast, some embodiments of the present disclosure perform real-time segmentation on the second image, obtain the puncture needle image corresponding to each second image and fuse the puncture needle image with the deformed preoperative image, consider the deformation of the puncture needle, and obtain a more accurate target guidance image. When the segmented puncture needle image is incompletely visible or absent from the target guidance image, some embodiments of the present disclosure fuse the pre-stored standard puncture needle image into the second image based on the positioning position information of the puncture needle in the robot coordinate system to obtain the target guidance image, thereby ensuring the completeness of the target guidance image during the interventional procedure.

FIG. 22 is an exemplary flowchart illustrating a process of a puncture needle position correction according to some embodiments of the present disclosure. In some embodiments, a process 2200 is executed by a puncture needle position correction system. As shown in FIG. 22, the process 2200 includes the following operations:

In some embodiments, the puncture needle position correction system further includes a positioning correction module, and the positioning correction module may be configured to perform the following operations:

In S2201, first position information of a puncture needle in an image coordinate system may be determined based on positioning position information of the puncture needle in a robot coordinate system.

More descriptions regarding the positioning position information, the first position information, and the second position information may be found in FIG. 18 and the related descriptions. In some embodiments, each of the positioning position information, the first position information, and the second position information may include information of a needle tip position and a needle tail position of the puncture needle.

A puncture image refers to an image obtained by performing image scanning on the puncture needle during the puncture process. In some embodiments, the puncture image includes a second image and/or a target guidance image. More descriptions regarding the second image may be found in FIG. 1 and the related descriptions. More descriptions regarding the target guidance image may be found in FIG. 12 and the related descriptions.

In embodiments of the present disclosure, during the puncture process performed by a surgical robot using the puncture needle, the positioning correction module may determine the needle tip position and/or the needle tail position of the puncture needle in real time, i.e., calculate the first position information. The surgical robot may also be connected to a medical imaging device, and the medical imaging device performs image scanning on the puncture needle during the puncture process of the surgical robot to obtain a puncture image including the puncture needle. After the medical imaging device scans and obtains each frame of the puncture image, each frame of the puncture image may be transmitted to the positioning correction module in real time, so that the positioning correction module is able to subsequently verify the position of the puncture needle in the puncture image. In some embodiments, when the first position information is position information in the robot coordinate system, after the surgical robot calculates and obtains the first position information, the surgical robot may further convert the first position information in the robot coordinate system into the first position information in the image coordinate system based on coordinate system registration information registered by the surgical robot, to feed back the first position information to the puncture image for subsequent processing operations.

In S2202, the needle tip position in a puncture image is identified based on the first position information to obtain second position information of the puncture needle in the puncture image.

In some embodiments, the second position information includes needle tip information and needle tail information of the puncture needle in the puncture image.

In the embodiments of the present disclosure, when the positioning correction module obtains the first position information and the puncture image based on the foregoing operations, the positioning correction module identifies the needle tip position of the puncture needle in the puncture image with reference to the needle tip information and the needle tail information in the first position information to obtain the second position information including the needle tip position information. The identification manner of the needle tip position of the puncture needle in the puncture image includes the following. A first manner includes inputting the first position information and the puncture image into a needle tip identification network for needle tip identification to directly obtain the second position information. The needle tip identification network is obtained by training performed by the positioning correction module in advance based on a sample set of puncture images and a corresponding annotated image set. The annotated image set is obtained by annotating each puncture image in the sample set corresponding to the needle tip position of the puncture needle provided by the surgical robot. A second manner includes processing the puncture image based on the first position information to obtain a processed puncture image that is easy to identify, and inputting the processed puncture image into the needle tip identification network for needle tip identification to obtain the second position information.

In S2203, the first position information is verified based on the second position information to obtain a verification result, and determine target position information based on the verification result.

In some embodiments, the verification result includes that the first position information is accurate and that the first position information is inaccurate.

In the embodiments of the present disclosure, when the surgical robot obtains the first position information of the puncture needle calculated by the surgical robot and the second position information of the puncture needle in the puncture image, the surgical robot uses the second position information as reference information to assist in verifying the accuracy of the first position information. Optionally, the surgical robot performs verification by checking consistency between the first position information and the second position information to obtain the verification result. Optionally, the surgical robot performs an operation or processing on the first position information and the second position information to obtain difference information between the first position information and the second position information, and then performs verification based on the difference information to obtain the verification result. When the surgical robot obtains the verification result and the verification result indicates that the first position information is accurate, it indicates that the second position information obtained based on the first position information is also accurate. In this case, the second position information is determined as the target position information, so as to provide guidance information for subsequent positioning of the puncture needle based on the target position information.

In the foregoing correction method, the needle tip positioning of the puncture image is verified by using the puncture needle information provided by the surgical robot, thereby implementing a combination of needle tip positioning of an interventional robot hardware system and needle tip positioning of an image algorithm, and greatly improving the precision and accuracy of the needle tip positioning. In addition, since the surgical robot is able to provide needle tip positioning in real time, real-time verification of the needle tip position in the puncture image is implemented, which can satisfy clinical scenarios with real-time requirements and improve the efficiency of a puncture surgery.

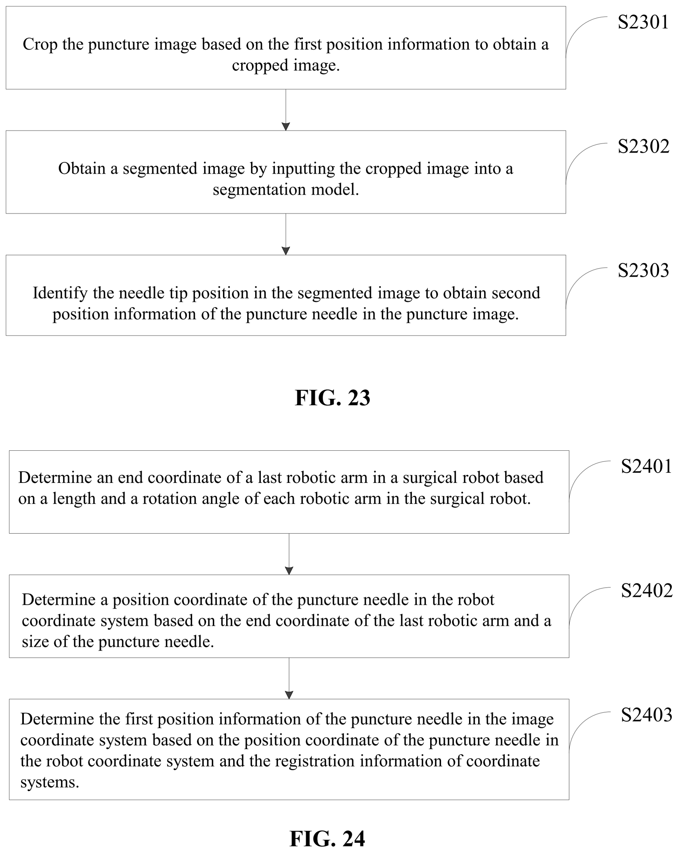

FIG. 23 is an exemplary flowchart illustrating a process of a puncture needle position correction according to another embodiment of the present disclosure.

In an embodiment, a method for positioning the puncture needle in a puncture image is further provided. As shown in FIG. 23, the method is performed by a positioning correction module and includes the following.

In S2301, the puncture image is cropped based on the first position information to obtain a cropped image.

In the embodiments of the present disclosure, when a surgical robot calculates and obtains the first position information, if the first position information is position information in a robot coordinate system, the first position information in the robot coordinate system is converted into first position information in an image coordinate system based on registration information of coordinate systems. Then, the model size of the puncture needle is determined based on the needle tip information and the needle tail information in the first position information. Then, the puncture image is cropped based on the model size of the puncture needle to obtain the cropped image, so that the cropped image includes a complete puncture needle and rich information of the puncture needle.

In S2302, a segmented image is obtained by inputting the cropped image into a segmentation model.

The segmentation model is a machine learning model. For example, the segmentation model is a pretrained neural network model configured to segment a target tissue in an input image to obtain a corresponding mask image.

In the embodiments of the present disclosure, the surgical robot inputs the cropped image into the pretrained segmentation model for image segmentation to segment a structure of the puncture needle to obtain the mask image, i.e., the segmented image. It should be noted that the segmentation model is a lightweight neural network model or a compressed model, so that the segmentation model is able to complete the image segmentation in a very short time to achieve real-time segmentation, thereby improving the real-time performance of subsequent verification.

In S2303, a needle tip position in the segmented image is identified to obtain second position information of the puncture needle in the puncture image.

In the embodiments of the present disclosure, when the surgical robot obtains the segmented image, the surgical robot first restores the size of the segmented image to the size of the puncture image, and identifies the needle tip position in the restored segmented image to obtain the second position information of the puncture needle in the puncture image. Optionally, the surgical robot uses a pre-trained positioning network or segmentation model to obtain needle tip position information of the puncture needle, i.e., the second position information of the puncture needle. Optionally, the surgical robot uses a high-dimensional data dimensionality reduction algorithm (Principal Component Analysis, PCA) to identify a needle tip direction in the restored segmented image, thereby obtaining a main puncture direction of the puncture needle in the segmented image to assist in positioning the puncture needle.

In the positioning method according to the embodiments of the present disclosure, the puncture image is first cropped based on the first position information of the puncture needle provided by the surgical robot, and then image segmentation is performed based on the cropped image, which greatly reduces the calculation amount required for the image segmentation, improves calculation efficiency, and thereby improves the real-time performance of puncture needle positioning.

FIG. 24 is an exemplary flowchart illustrating a process of a puncture needle position correction according to another embodiment of the present disclosure.

In an embodiment, the foregoing first position information is calculated by the robot. Therefore, the present disclosure further provides a method for obtaining the first position information. As shown in FIG. 24, the method for obtaining the first position information includes the following.

In S2401, an end coordinate of a last robotic arm in a surgical robot is determined based on a length and a rotation angle of each robotic arm in the surgical robot.

The surgical robot is a multi-degree-of-freedom robotic arm robot, for example, a five-degree-of-freedom robotic arm robot or a six-degree-of-freedom robotic arm robot.

In the embodiments of the present disclosure, the surgical robot first calculates a position coordinate of a first robotic arm based on a length and a rotation angle of the first robotic arm. Then, based on the position coordinate of the first mechanical arm and a length and a rotation angle of a second robotic arm, the surgical robot calculates a position coordinate of the second robotic arm. The calculation of a position coordinate of a next robotic arm continues in this way until the end coordinate of the last robotic arm is obtained.

In S2402, a position coordinate of the puncture needle in the robot coordinate system is determined based on the end coordinate of the last robotic arm and a size of the puncture needle.

The size of the puncture needle includes a needle tip position and a needle tail position of a real puncture needle. The position coordinate of the puncture needle in the robot coordinate system includes a needle tip coordinate of the puncture needle in the robot coordinate system and a needle tail coordinate of the puncture needle in the robot coordinate system.

In the embodiments of the present disclosure, when the surgical robot obtains the end coordinate of the last robotic arm, the surgical robot determines the needle tip coordinate of the puncture needle in the robot coordinate system in combination with the needle tip position of the real puncture needle, and determines the needle tail coordinate of the puncture needle in the robot coordinate system in combination with the needle tail position of the real puncture needle.

In S2403, the first position information of the puncture needle in the image coordinate system is determined based on the position coordinate of the puncture needle in the robot coordinate system and the registration information of coordinate systems.

The first position information includes the needle tip position and the needle tail position of the puncture needle in the image coordinate system, the registration information of coordinate systems includes a coordinate system registration matrix. The coordinate system registration matrix represents a conversion relationship between the robot coordinate system and the image coordinate system.

In the embodiments of the present disclosure, when the robot calculates and obtains the position coordinate of the puncture needle in the robot coordinate system, i.e., obtains the needle tip coordinate and the needle tail coordinate of the puncture needle in the robot coordinate system, the robot may extract the coordinate system registration matrix from the registration information of coordinate systems, and performs coordinate conversion on the needle tip coordinate of the puncture needle in the robot coordinate system based on the coordinate system registration matrix, and performs coordinate conversion on the needle tail coordinate of the puncture needle in the robot coordinate system, specifically converting the needle tip coordinate and the needle tail coordinate in the robot coordinate system into the first position information including the needle tip information and the needle tail information in the image coordinate system.

FIG. 25 is an exemplary flowchart illustrating a process of a puncture needle position correction according to another embodiment of the present disclosure.

In an embodiment, a method for correcting the position of the puncture needle in a puncture image based on position information provided by a surgical robot is provided. That is, as shown in FIG. 25, the foregoing S2203 "verify the first position information based on the second position information to obtain a verification result" includes the following operations.

In S2501, a position distance between a position of the puncture needle located by the surgical robot and a position of the puncture needle in the puncture image is determined based on the first position information and the second position information.

In the embodiments of the present disclosure, when the surgical robot verifies the first position information, the surgical robot first extracts a needle tip position from the first position information and a needle tip position from the second position information, and then determines a distance between the two needle tip positions, i.e., determines a distance between the needle tip position provided by the surgical robot and a needle tip position in the image algorithm, so as to subsequently verify the needle tip position provided by the surgical robot based on the distance.

In S2502, the first position information is verified based on the position distance to obtain the verification result.

In the embodiments of the present disclosure, after the surgical robot obtains the distance between the first position information and the second position information based on the foregoing operations, the surgical robot directly verifies the first position information based on a distance value of the distance, or evaluates the distance and then verifies the first position information based on an evaluation result to obtain the verification result.

FIG. 26 is an exemplary flowchart illustrating a process of a puncture needle position correction according to another embodiment of the present disclosure.

As shown in FIG. 26, the correction method is performed by a positioning correction module. The foregoing S2502 "the first position information is verified based on the position distance to obtain the verification result" includes the following operation.

In S2601, the first position information is verified based on the position distance to obtain the verification result.

In response to determining that the position distance is greater than a preset distance threshold, operation S2602 is performed. In response to determining that the position distance is not greater than the preset distance threshold, operation S2603 is performed.

In S2602, the verification result indicating that the first position information is inaccurate is determined.

In S2603, the verification result indicating that the first position information is accurate is determined.

The preset distance threshold is determined in advance based on positioning precision for positioning the puncture needle, and is a reference parameter for evaluating whether the first position information is accurate.

In some embodiments, the preset distance threshold may be adjusted based on different puncture needles, different medical imaging devices, and different robot systems.

The robot system refers to a system corresponding to a different surgical robot.

More descriptions regarding the puncture needle may be found in FIG. 18 and the related descriptions. More descriptions regarding the medical imaging device may be found in FIG. 1 and the related descriptions.

In some embodiments, the positioning correction module may determine a corresponding preset distance threshold by querying a fourth preset table based on a corresponding puncture needle, a medical imaging device, and a robot system. The preset distance threshold may correspond to different puncture needles, different medical imaging devices, and different robot systems in the fourth preset table.

In some embodiments, the positioning correction module may determine the preset distance threshold based on device parameters of the medical imaging device and an average value of imaging quality of one or more CT images.

In some embodiments, the device parameter of the medical imaging device may include a scan time, a slice thickness, a scan algorithm, a scan mode, etc.

In some embodiments, the imaging quality of the CT image may be obtained by automatically performing a comprehensive evaluation by the medical imaging device after obtaining the CT image through CT scanning.

In some embodiments, the positioning correction module may determine the preset distance threshold based on a ninth preset relationship between the device parameters of the medical imaging device, an average value of imaging quality of one or more CT images, and the preset distance threshold. For example, the ninth preset relationship may include a relationship between the scan time, the slice thickness, and the preset distance threshold. A longer scan time and a larger slice thickness may allow the preset distance threshold to be set larger. As another example, the ninth preset relationship may include a relationship between the average value of the imaging quality and the preset distance threshold. When the average value of the imaging quality of the one or more CT images is higher, it indicates that the CT image is able to more accurately reflect the needle tip position. In this case, the preset distance threshold may be appropriately set larger.

In some embodiments, the positioning correction module may determine the preset distance threshold based on a type of the puncture needle and system parameters of the robot system.

The system parameters of the robot system refer to related parameters of the robot system corresponding to the surgical robot. For example, the system parameters may include master-slave control accuracy, theoretical cumulative error, device stability, etc.

In some embodiments, the positioning correction module may determine the preset distance threshold based on a tenth preset relationship between the type of the puncture needle, the system parameters of the robot system, and the preset distance threshold. For example, the tenth preset relationship may include a relationship between the master-slave control accuracy, the theoretical cumulative error, the device stability, and the preset distance threshold. For example, higher master-slave control accuracy, a smaller theoretical cumulative error, and higher device stability may allow the preset distance threshold to be set larger.

As another example, the tenth preset relationship may include a relationship between the type of the puncture needle and the preset distance threshold.

In some embodiments of the present disclosure, by determining the preset distance threshold based on the device parameters of the medical imaging device and the average value of the imaging quality of the one or more CT images, the impact of the medical imaging device on the accuracy of the scanned image may be considered to determine a more reasonable preset distance threshold.

In some embodiments of the present disclosure, by determining the preset distance threshold based on the type of the puncture needle and the system parameters of the robot system, the impact of the type of the puncture needle and the robot system on the fault tolerance degree of the surgery may be considered to set a more reasonable preset distance threshold.

Embodiments of the present disclosure relate to a method for verifying the first position information based on a distance value of the position distance. In response to determining that the position distance is greater than the preset distance threshold, it indicates a large difference between the first position information and the second position information. In this case, the position information of the puncture needle provided by the surgical robot is considered inaccurate. In response to determining that the position distance is not greater than the preset distance threshold, it indicates a small difference between the first position information and the second position information. In this case, the position information of the puncture needle provided by the surgical robot is considered accurate. In response to determining that the first position information is accurate, the positioning correction module may determine the second position information as the target position information.

In one embodiment, when the verification result indicates that the first position information is inaccurate, the method shown in FIG. 26 further includes the following operations: a cause for inaccuracy of the first position information is determined, and a next action of the surgical robot is determined based on the cause.

The cause for the inaccuracy of the first position information includes a cumulative error of a positioning system of the surgical robot, a deformation of the puncture needle, a mismatch of a puncture needle positioning algorithm, environmental factor interference, etc.

In embodiments of the present disclosure, when it is determined through verification that the first position information is inaccurate, the surgical robot may further analyze the cause for the inaccuracy of the first position information, thereby determining, based on the cause, whether to continue the needle puncture, whether to re-correct an image positioning algorithm, or whether to correct parameters of the surgical robot, i.e., determining the next action of the surgical robot. For example, when the surgical robot determines that the first position information is inaccurate, the surgical robot may self-check its robotic parameters or self-check its positioning system to determine whether the cumulative error of the positioning system of the surgical robot causes a positioning problem of the surgical robot, thereby affecting the accuracy of the first position information provided by the surgical robot. Optionally, the surgical robot may further detect the size of a real puncture needle to determine whether the puncture needle is deformed. If deformation occurs, it is determined that the deformation of the puncture needle causes the inaccuracy of the first position information provided by the surgical robot. Optionally, if the surgical robot excludes the inaccuracy of the first position information caused by the cumulative error of the positioning system of the surgical robot and the deformation of the puncture needle, it may be determined that the inaccuracy of the first position information is caused by other reasons.

In one embodiment, in response to the surgical robot analyzes that the cause for the inaccuracy of the first position information includes the cumulative error of the positioning system of the surgical robot, the positioning correction module may perform the following operations: the surgical robot is controlled to perform recalibration to obtain updated registration information of coordinate systems, the surgical robot is controlled to obtain updated first position information based on the updated registration information of the coordinate systems.

In embodiments of the present disclosure, in response to determining that the cause for the inaccuracy of the first position information includes the cumulative error of the positioning system of the surgical robot, it indicates that the robotic state of the surgical robot has changed after prolonged use, i.e., hardware damage may have occurred, inaccuracy of the positioning system of the surgical robot. In this case, the surgical robot may be recalibrated based on a current robotic state, thereby obtaining new coordinate system registration information for registration, so that when the surgical robot calculates position information of a next puncture needle in real time, the surgical robot can perform the calculation based on the new coordinate system registration information, thereby obtaining accurate positioning information of the puncture needle. It should be noted that the recalibration process includes: reacquiring a first coordinate of the puncture needle in the new robot coordinate system, and reacquiring a second coordinate of the puncture needle in an image coordinate system; constructing a new coordinate system registration matrix based on a conversion relationship between the first coordinate and the second coordinate; and thereby obtaining the new coordinate system registration information from the newly constructed coordinate system registration matrix.

In some embodiments, the positioning correction module may adjust scan parameters of the medical imaging device based on the verification result and the cumulative error of the positioning system of the surgical robot.

In some embodiments, the scan parameter of the medical imaging device may include a scan field of view, an exposure dose, a scan type, a slice thickness, a count of acquisition channels used, etc.

In some embodiments, the positioning correction module may determine the scan parameters of the medical imaging device based on an eleventh preset relationship between the verification result, the cumulative error of the positioning system of the surgical robot, and the scan parameters. For example, the eleventh preset relationship may be that when the verification result is inaccurate, the scan field of view and the exposure dose are adjusted based on the cumulative error of the positioning system of the surgical robot, so that the needle tip is at a center position of the scan field of view.

In some embodiments of the present disclosure, by adjusting the scan parameters of the medical imaging device based on the verification result and the cumulative error of the positioning system of the surgical robot, the medical imaging device can subsequently obtain a more accurate scan image based on the scan parameters.

In one embodiment, when the surgical robot analyzes that the cause for the inaccuracy of the first position information includes the deformation of the puncture needle, the positioning correction module may perform the following operations: the surgical robot is controlled to perform a needle withdrawal operation.

In embodiments of the present disclosure, in response to determining that the cause for the inaccuracy of the first position information includes the deformation of the puncture needle, it indicates that the puncture needle is deformed after prolonged use, i.e., damage may have occurred on the puncture needle, a deviation is caused in positioning the puncture needle in the image. Therefore, in this case, the puncture is unable to be continued, and the surgical robot is controlled to perform the needle withdrawal operation to avoid affecting the puncture. The surgical robot may further display warning information to inform a user of a puncture risk and to stop the puncture operation. The surgical robot may further replace the puncture needle after performing the needle withdrawal operation, so as to subsequently perform the puncture operation again with a new puncture needle.

In some embodiments, the positioning correction module may determine a problem of the puncture needle, a cause of the problem, and a next action of the surgical robot through a puncture needle monitoring model based on the first position information and the second position information.

In some embodiments, the puncture needle monitoring model may be a machine learning model. For example, the puncture needle monitoring model may be a neural network model, a deep neural network model, etc., or any combination thereof. In some embodiments, an input of the puncture needle monitoring model may include the first position information and the second position information, and an output may be the problem of the puncture needle, the cause of the problem, and the next action of the surgical robot.

In some embodiments, the puncture needle monitoring model may be obtained by training based on a large number of ninth training samples with ninth labels. A ninth training sample may be sample first position information and sample second position information in the historical records. The ninth label of the ninth training sample may be an actual historical problem of the puncture needle, an actual historical cause of the problem, and an actual historical next action of the surgical robot corresponding to the ninth training sample when the puncture needle had the problem due to the cause. The ninth label may be automatically annotated by the system based on the historical records.

In some embodiments, the actual historical problem of the puncture needle and the cause of the problem corresponding to the ninth training sample in the ninth label may be obtained based on a post-analysis or detection performed in a historical surgery corresponding to the ninth training sample. For example, the post analysis or detection may includes performing abnormal positioning after subsequently removing the puncture needle, or performing more precise abnormal positioning using another device.

In some embodiments, the actual historical next action of the surgical robot corresponding to the ninth training sample in the ninth label may be determined based on an actual execution action that had a good subsequent execution effect (obtained based on a subsequent evaluation) in the historical surgery corresponding to the ninth training sample.

In some embodiments, the actual historical next action of the surgical robot corresponding to the ninth training sample in the ninth label may also be determined based on a next action manually selected by a doctor in a historical surgery corresponding to the sample.

A training manner of the puncture needle monitoring model is similar to a training manner of an exposure range prediction model. More descriptions regarding the training manner of the puncture needle monitoring model may be found in FIG. 3 and the related descriptions.

In some embodiments of the present disclosure, the puncture needle monitoring model is obtained through training. The puncture needle monitoring model is configured to determine the problem of the puncture needle, the cause of the problem, and the next action of the surgical robot, which can better monitor the condition of the puncture needle during a surgery, so as to promptly identify problems and provide adjustment ideas, thereby improving surgical efficiency.

In some embodiments, the output of the puncture needle monitoring model may further include: whether to send a reminder to a user and corresponding reminder information.

In some embodiments, reminder information corresponding to different situations may be preset in advance.

In some embodiments, when the output of the puncture needle monitoring model includes whether to send the reminder to the user and corresponding reminder information, the processor may add whether to send the reminder to the user and corresponding reminder information corresponding to the ninth training sample into a corresponding ninth label when training the puncture needle monitoring model. Whether to send the reminder to the user and corresponding reminder information corresponding to the ninth training sample may be determined based on an actual situation of a historical surgery corresponding to the ninth training sample. For example, if the puncture needle was deformed after a certain time in the historical surgery, whether to send the reminder to the user and corresponding reminder information may be: determining that a reminder needs to be sent to the user, and sending corresponding needle withdrawal reminder information to the user.

In some embodiments of the present disclosure, by including whether to send the reminder to the user and corresponding reminder information as a portion of the output of the puncture needle monitoring model, the monitoring process of the puncture needle can be made more accurate and effective, which helps to promptly identify problems and provide reminders.

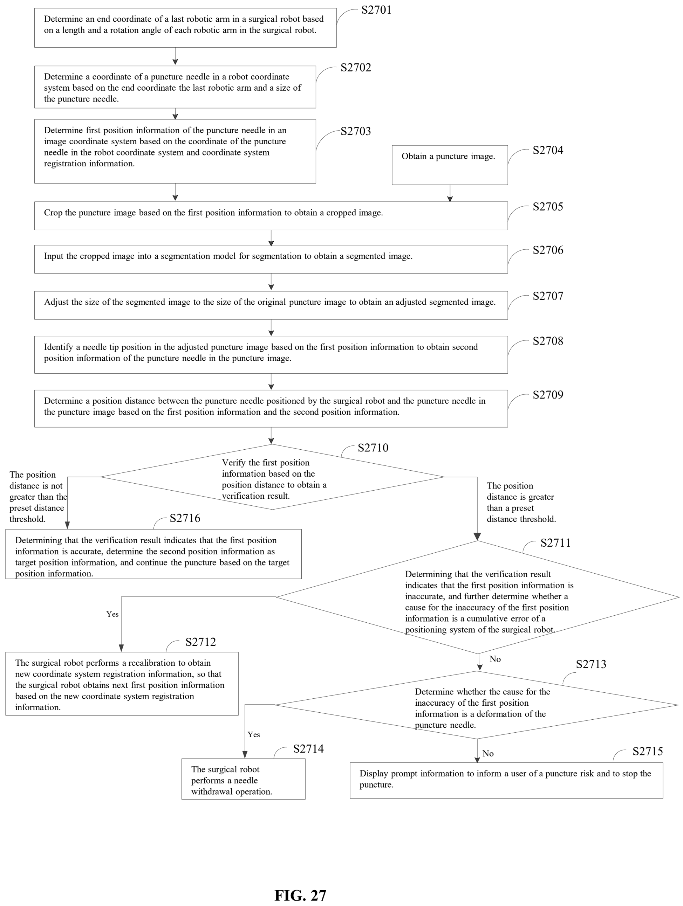

FIG. 27 is an exemplary flowchart illustrating a process of a puncture needle position correction according to another embodiment of the present disclosure.

Based on all the above embodiments, as shown in FIG. 27, the puncture needle position correction method is further provided. The method includes the following operations.

In S2701, an end coordinate of a last robotic arm in a surgical robot are determined based on a length and a rotation angle of each robotic arm in the surgical robot.

In S2702, a coordinate of a puncture needle in a robot coordinate system is determined based on the end coordinate of the last robotic arm and a size of the puncture needle.

In S2703, first position information of the puncture needle in an image coordinate system is determined based on the coordinate of the puncture needle in the robot coordinate system and coordinate system registration information. The first position information includes a needle tip position and a needle tail position of the puncture needle.

In S2704, a puncture image is obtained.

In S2705, the puncture image is cropped based on the first position information to obtain a cropped image.

In S2706, the cropped image is input into a segmentation model for segmentation to obtain a segmented image.

In S2707, the size of the segmented image is adjusted to the size of the original puncture image to obtain an adjusted segmented image.

In S2708, a needle tip position in the adjusted puncture image is identified based on the first position information to obtain second position information of the puncture needle in the puncture image.

In S2709, a position distance between the puncture needle positioned by the surgical robot and the puncture needle in the puncture image is determined based on the first position information and the second position information.

In S2710, the first position information is verified based on the position distance to obtain a verification result.

In response to determining that the position distance is greater than a preset distance threshold, operations S2711 to S2715 are performed.

In response to determining that the position distance is not greater than the preset distance threshold, operation S2716 is performed.

In S2711, the verification result indicates that the first position information is inaccurate is determined, and whether a cause for the inaccuracy of the first position information is a cumulative error of a positioning system of the surgical robot is further determined. In response to determining that the cause is the cumulative error of the positioning system of the surgical robot, operation S2712 is performed. In response to determining that the cause is not the cumulative error of the positioning system of the surgical robot, operation S2713 is performed.

In S2712, the surgical robot performs a recalibration to obtain new coordinate system registration information, so that the surgical robot obtains next first position information based on the new coordinate system registration information.

In S2713, whether the cause for the inaccuracy of the first position information is a deformation of the puncture needle is determined. In response to determining that the cause includes the deformation of the puncture needle, operation S2714 is performed. In response to determining that the cause does not include the deformation of the puncture needle, operation S2715 is performed.

In S2714, the surgical robot performs a needle withdrawal operation.

In S2715, prompt information is displayed to inform a user of a puncture risk and to stop the puncture.

In S2716, the verification result indicates that the first position information is accurate is determined, the second position information is determined as target position information, and the puncture is continued based on the target position information.

The manners described in the above operations have been explained in the foregoing content. More descriptions regarding the operations above may be found in the foregoing descriptions, which is not repeated here.

The method according to the embodiments of the present disclosure effectively verifies the position of the puncture needle in the puncture image. Furthermore, by combining the needle tip position based on the image algorithm and the needle tip position calculated by an interventional robot hardware system, the needle tip position of the image algorithm is accurately verified. Moreover, when the positioning of the image algorithm is verified to be inaccurate, the positioning system of the surgical robot can be corrected in time, thereby achieving an effect of timely guiding the surgical robot to perform a correct puncture, so as to improve efficiency and accuracy of the puncture surgery.

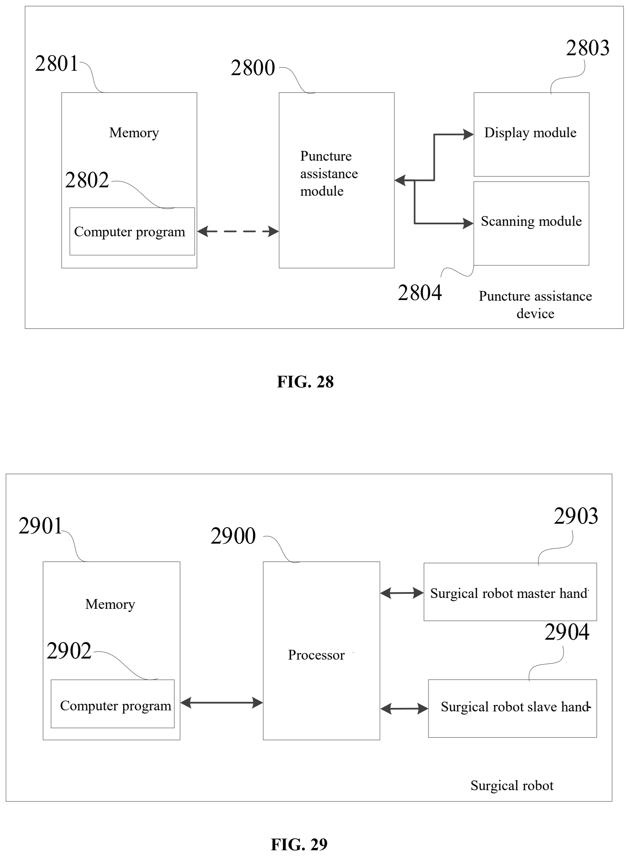

FIG. 28 is a schematic diagram illustrating another exemplary puncture assistance device according to some other embodiments of the present disclosure.

As shown in FIG. 28, the puncture assistance device in the embodiments of the present disclosure includes a display module 2803, a scanning module 2804, and a puncture assistance module 2800. The puncture assistance module 2800 is configured to perform operations executed by the puncture assistance device in any of the foregoing puncture method embodiments.

In some embodiments, the puncture assistance module 2800 is configured to perform the following operations: obtaining a plurality of scan images acquired by a medical imaging device performing a scanning operation on a target object through the scanning module 2804; obtaining puncture status information of a puncture needle in a surgical robot; determining position information of a needle tip point of the puncture needle and position information of a predicted target point based on the puncture status information; annotating the needle tip point and the predicted target point onto a target image based on the position information of the needle tip point and the position information of the predicted target point to generate an annotated image; and displaying the annotated image through the display module 2803. More descriptions regarding the content herein may be found in FIG. 2 and the related descriptions.

In some embodiments, the puncture assistance module 2800 may directly obtain the plurality of scan images from the medical imaging device through the scanning module, or may indirectly obtain the plurality of scan images from the medical imaging device. The present disclosure does not limit this.

In some embodiments, as shown in FIG. 28, the puncture assistance device in some other embodiments further includes a memory 2801 and a computer program 2802 stored in the memory 2801 and executable on the puncture assistance module 2800. The puncture assistance module 2800 implements steps executed by the puncture assistance device in any of the foregoing puncture method embodiments when executing the computer program 2802.

In some embodiments, the puncture assistance module 2800 executes a plurality of operations when executing the computer program 2802. More descriptions regarding the content herein may be found in FIG. 2 and the related descriptions.

FIG. 28 is merely an example of the puncture assistance device and does not limit the puncture assistance device. The puncture assistance device may include more or fewer components than those shown, or combine some components, or have different components.

FIG. 29 is a schematic diagram illustrating an exemplary surgical robot according to some embodiments of the present disclosure.

As shown in FIG. 29, the surgical robot in the embodiments of the present disclosure includes a surgical robot master hand 2903, a surgical robot slave hand 2904, at least one processor 2900 (only one processor is shown in FIG. 29), a memory 2901, and a computer program 2902 stored in the memory 2901 and executable on the at least one processor 2900. The processor 2900 implements operations executed by the processor in any of the foregoing puncture assistance system embodiments when executing the computer program 2902.

FIG. 29 is merely an example of the surgical robot and does not limit the surgical robot. The surgical robot may include more or fewer components than those shown, or combine some components, or have different components.

In some embodiments of the present disclosure, the plurality of first images acquired by the foregoing method can show respiratory changes of the target object at different respiratory amplitude points within a complete respiratory range, so that deformation parameters determined subsequently can more completely reflect deformation of a target portion of the target object within the respiratory range.

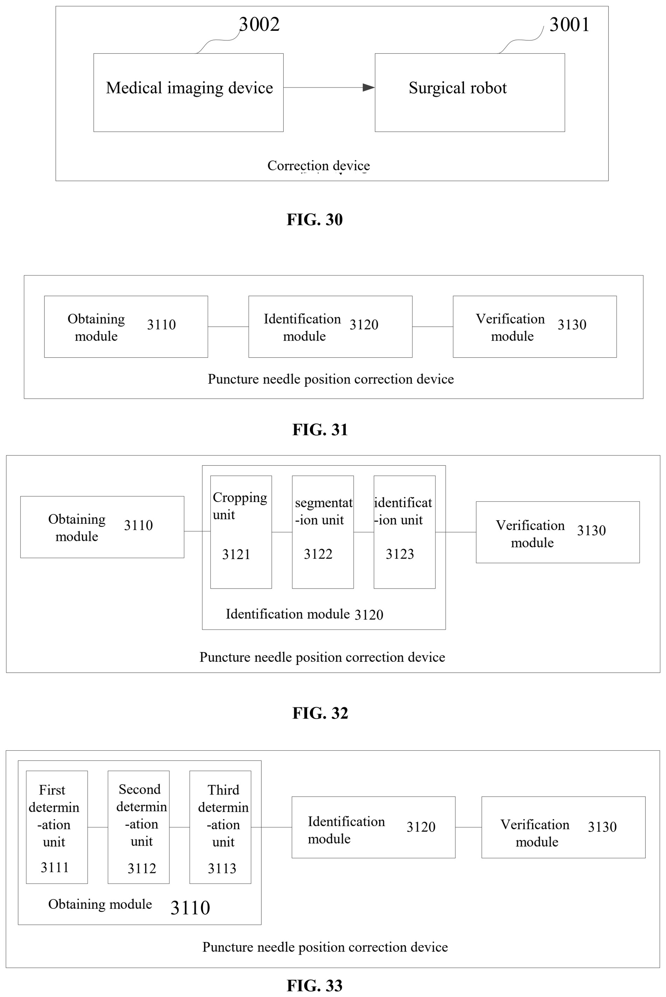

FIG. 30 is a schematic diagram illustrating another exemplary puncture needle position correction system according to some embodiments of the present disclosure.

The puncture needle position correction method provided by the embodiments of the present disclosure may be applied to a verification system as shown in FIG. 30. A surgical robot 3001 is connected to a medical imaging device 3002 through a wired or wireless communication connection. The surgical robot 3001 may obtain a puncture image from the medical imaging device 3002, and verify a position of the puncture needle in the puncture image based on positioning position information of the puncture needle provided by the surgical robot 3001. The surgical robot 3001 may perform a puncture operation, and may perform real-time positioning of the puncture needle during the puncture process to obtain positioning information of the real-time positioning. The surgical robot 3001 includes a processing device. The processing device may include, but is not limited to, various personal computers, laptops, smartphones, tablets, servers, etc. The medical imaging device 3002 may be various image scanning devices, e.g., a computed tomography (CT) device, a magnetic resonance imaging (MRI) device, etc.

FIG. 31 is a schematic diagram illustrating an exemplary puncture needle position correction device according to some embodiments of the present disclosure.

In one embodiment, as shown in FIG. 31, a puncture needle position correction device is provided, the device includes a positioning correction module. The positioning correction module includes:

an obtaining module 3110 configured to determine first position information of the puncture needle in an image coordinate system based on positioning position information and obtain a puncture image; an identification module 3120 configured to identify a needle tip position in the puncture image based on the first position information to obtain second position information of the puncture needle in the puncture image;
a verification module 3130 configured to verify the first position information based on the second position information to obtain a verification result, and determine target position information based on the verification result.

FIG. 32 is a schematic diagram illustrating another exemplary puncture needle position correction device according to some other embodiments of the present disclosure.
In one embodiment, as shown in FIG. 32, the identification module 3120 includes:

a cropping unit 3121 configured to crop a puncture image based on first position information to obtain a cropped image;
a segmentation unit 3122 configured to obtain a segmented image from the cropped image through a segmentation model;
an identification unit 3123 configured to identify a needle tip position in the segmented image to obtain second position information in the puncture image.

FIG. 33 is a schematic diagram illustrating another exemplary puncture needle position correction device according to some other embodiments of the present disclosure.
In one embodiment, as shown in FIG. 33, the obtaining module 3110 includes:

a first determination unit 3111 configured to determine an end coordinate of a last robotic arm in the surgical robot based on a length and a rotation angle of each robotic arm in the surgical robot;
a second determination unit 3112 configured to determine a position coordinate of the puncture needle in a robot coordinate system based on the end coordinate of the last robotic arm and a size of the puncture needle;
a third determination unit 3113 configured to determine first position information of the puncture needle in an image coordinate system based on the position coordinate in the robot coordinate system and coordinate system registration information.

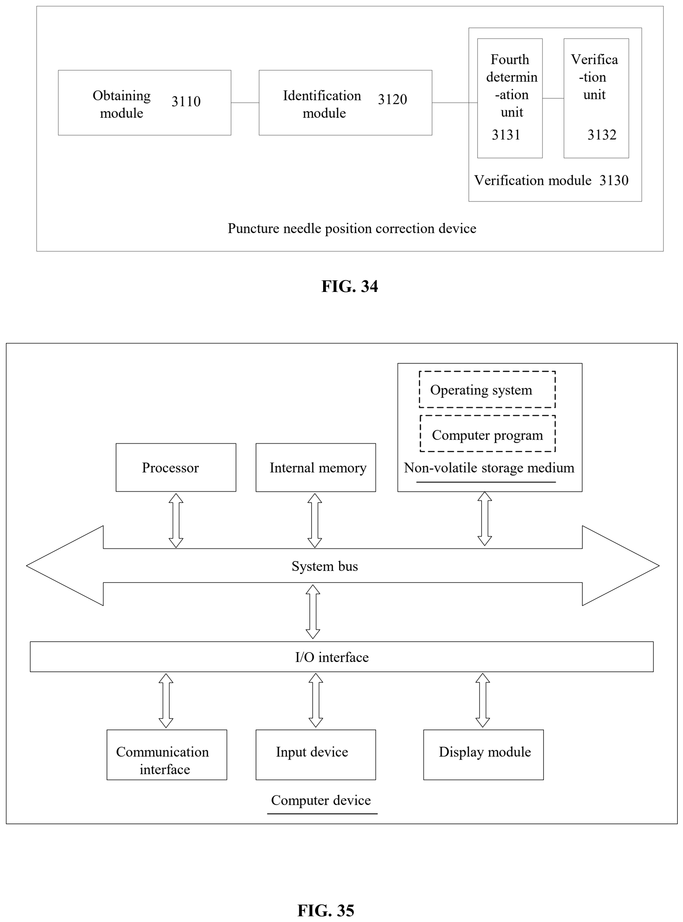

FIG. 34 is a schematic diagram illustrating another exemplary puncture needle position correction device according to some other embodiments of the present disclosure.
In one embodiment, as shown in FIG. 34, the verification module 3130 includes:
a fourth determination unit 3131 configured to determine a position distance between a position of the puncture needle located by a surgical robot and a position of the puncture needle in the puncture image based on first position information and second position information;
A verification unit 3132 is configured to verify the first position information based on the position distance to obtain the verification result.
Each module in the puncture needle position correction device may be implemented entirely or partially by software, hardware, or a combination thereof. Each module may be embedded in a hardware form in or independent of a processor of a computer device, or may be stored in a software form in a memory of the computer device, so that the processor is able to call and execute operations corresponding to each module.
FIG. 35 is a schematic diagram illustrating an exemplary computer device according to some embodiments of the present disclosure.
In one embodiment, the computer device is provided. The computer device may be a terminal, and a diagram of an internal structure may be as shown in FIG. 35. The computer device includes a processor, a memory, a communication interface, a display module, and an input device connected through a system bus and an I/O interface. The processor of the computer device is configured to provide computing and control capabilities. The memory of the computer device includes a non-volatile storage medium and an internal memory. The non-volatile storage medium stores an operating system and a computer program. The internal memory provides an environment for running the operating system and the computer program in the non-volatile storage medium. The communication interface of the computer device is configured to communicate with an external terminal in a wired or wireless manner. The wireless manner may be implemented via WIFI, a mobile cellular network, NFC (Near Field Communication), or other technologies. The computer program implements a puncture needle position correction method when executed by the processor. The display module of the computer device may be a liquid crystal display screen or an electronic ink display screen. The input device of the computer device may be a touch layer covering the display screen, or may be a key, a trackball, or a touchpad disposed on a housing of the computer device, or may be an external keyboard, touchpad, or mouse, etc.
Those skilled in the art is able to understand that the structure shown in FIG. 35 is only a block diagram of a portion of the structure related to the solution of the present disclosure, and does not constitute a limitation on the computer device to which the solution of the present disclosure is applied. A specific computer device may include more or fewer components than those shown in the figure, or combine some components, or have different component arrangements.
The implementation principle and technical effect of a computer program product provided in the above embodiment are similar to those of the above method embodiment, and are not repeated here.
Those of ordinary skill in the art is able to understand that all or portion of the processes in the methods of the above embodiments may be implemented by instructing relevant hardware through a computer program. The computer program may be stored in a non-volatile computer-readable storage medium. When the computer program is executed, the processes of the embodiments of the above methods may be included. Any reference to a memory, a database, or other media used in the embodiments provided in the present disclosure may include at least one of a non-volatile memory and a volatile memory. The non-volatile memory may include a read-only memory (ROM), a magnetic tape, a floppy disk, a flash memory, an optical memory, a high-density embedded non-volatile memory, a resistive random access memory (ReRAM), a magneto resistive random access memory (MRAM), a ferroelectric random access memory (FRAM), a phase change memory (PCM), a graphene memory, etc. The volatile memory may include a random access memory (RAM), an external cache memory, etc. By way of illustration and not limitation, the RAM may be in various forms, such as a static random access memory (SRAM), a dynamic random access memory (DRAM), etc.

The database involved in the embodiments provided in the present disclosure may include at least one of a relational database and a non-relational database. The non-relational database may include a blockchain-based distributed database, etc., without limitation thereto.

An embodiment of the present disclosure further provides a chip located in an electronic device. The chip includes: a processing unit and a communication unit. The processing unit may be a processor, etc. The communication unit may be an input/output interface, a pin, a circuit, etc. The processing unit may execute computer instructions to cause the electronic device to perform the operations executed by the puncture assistance device in any puncture method provided in the embodiments of the present disclosure, or the operations executed by the processor in the embodiment of the puncture assistance device.

In some embodiments, the computer instructions are stored in a storage unit.

In some embodiments, the storage unit is a storage unit inside the chip, such as a register, a cache, etc. The storage unit may also be a storage unit located outside the chip in the terminal, such as a ROM, or other types of static storage devices capable of storing static information and instructions, a random RAM.

The puncture assistance device, the computer-readable storage medium, the computer program product, and the chip provided in this embodiment are all used to execute the corresponding methods provided above. Therefore, the beneficial effects that can be achieved can refer to the beneficial effects in the corresponding methods provided above, and are not repeated here. The basic concepts have been described above. It is apparent to those skilled in the art that the above detailed disclosure is merely by way of example and does not constitute a limitation of the present disclosure. Although not explicitly stated herein, those skilled in the art may make various modifications, improvements, and amendments to the present disclosure. Such modifications, improvements, and amendments are suggested in the present disclosure. Therefore, such modifications, improvements, and amendments still fall within the spirit and scope of the exemplary embodiments of the present disclosure.

Meanwhile, the present disclosure uses specific words to describe the embodiments of the present disclosure. For example, "an embodiment," "one embodiment," and/or "some embodiments" mean that a certain feature, structure, or characteristic is related to at least one embodiment of the present disclosure. Therefore, it should be emphasized and noted that "an embodiment" or "one embodiment" or "an alternative embodiment" mentioned two or more times in different places in the present disclosure does not necessarily refer to the same embodiment. In addition, certain features, structures, or characteristics in one or more embodiments of the present disclosure can be appropriately combined.

Similarly, it should be noted that, in order to simplify the expression disclosed in the present disclosure and thereby help the understanding of one or more inventive embodiments, in the foregoing description of the embodiments of the present disclosure, multiple features are sometimes grouped into one embodiment, drawing, or description thereof. However, this disclosure method does not mean that the object of the present disclosure requires more features than those mentioned in the claims. Rather, claimed subject matter may lie in less than all features of a single foregoing disclosed embodiment.

In some embodiments, numbers describing the quantity of ingredients or attributes are used. It should be understood that such numbers used to describe the embodiments are modified by the modifiers "about," "approximately," or "substantially" in some examples. Unless otherwise stated, "about," "approximately," or "substantially" indicates that the stated number allows a variation of $\pm 20\%$. Accordingly, in some embodiments, the numerical parameters used in the specification and claims are approximate values. These approximate values can vary according to the characteristics required by individual embodiments. In some embodiments, numerical parameters should consider the specified number of significant digits and adopt the method of general digit retention. Although the numerical ranges and parameters used to confirm the breadth of their scope in some embodiments of the present disclosure are approximate values, in specific embodiments, the setting of such numerical values is as precise as possible within the feasible range.

Finally, it should be understood that the embodiments described in the present disclosure are only used to illustrate the principles of the embodiments of the present disclosure. Other variations may also fall within the scope of the present disclosure. Therefore, by way of example and not limitation, alternative configurations of the embodiments of the present disclosure can be considered consistent with the teachings of the present disclosure. Accordingly, the embodiments of the present disclosure are not limited to the embodiments explicitly introduced and described in the present disclosure.

**Claims**

1. A puncture assistance system, comprising a processor configured to:

   obtain a scan image of a target object; the scan image including at least one of a preoperative image, a first image from a first time period, and a second image from a second time period; wherein the first time period is earlier than the second time period;
   generate puncture guidance information based on puncture status information and the scan image; guide a puncture operation based on the puncture guidance information;
   and display the puncture guidance information.

2. The puncture assistance system according to claim 1, wherein the processor is further configured to:

   obtain the puncture status information through a surgical robot;
   determine position information of a first needle tip point of a puncture needle and position information of a predicted target point based on the puncture status information, the predicted target point being a spatial point reached by a needle tip point of the puncture needle after completing needle insertion according to a posture in the puncture status information;
   generate an annotated image based on the position information of the first needle tip point and the position information of the predicted target point, the annotated image being obtained by annotating the first needle tip point and the predicted target point onto a target image; the target image being determined based on the scan image;
   display the annotated image.

3. The puncture assistance system according to claim 2, wherein the position information of the first needle tip point is determined based on a relative positional relationship between the puncture needle and the surgical robot, and the processor is further configured to:

   determine a needle tract contour of the puncture needle based on the target image;
   determine position information of a second needle tip point based on the needle tract contour;
   determine whether the needle tract contour involves an anomaly based on the position information of the first needle tip point and the position information of the second needle tip point; and
   provide a warning regarding the anomaly in response to determining that the needle tract contour involves the anomaly.

4. The puncture assistance system according to claim 2, wherein the processor is further configured to:

   in response to receiving a first posture adjustment instruction, determine lesion position information of a target lesion based on the scan image;
   determine first posture adjustment data based on the puncture status information and the lesion position information, the first posture adjustment data representing posture change data of the puncture needle when adjusting the predicted target point to a lesion position corresponding to the lesion position information;
   send a first movement instruction to the surgical robot based on the first posture adjustment data to control the surgical robot to adjust a posture of the puncture needle.

5. The puncture assistance system according to claim 4, wherein before sending the first movement instruction to the surgical robot, the processor is further configured to:
   determine whether at least one of needle withdrawal conditions is satisfied, the needle withdrawal conditions including:

   a puncture depth of the puncture needle being greater than a first threshold; or
   a distance between the predicted target point and the lesion position being greater than a second threshold;
   or
   posture adjustment based on the first posture adjustment data causing the puncture needle to interfere with a target tissue or organ of the target object;
   in response to determining that the at least one of the needle withdrawal conditions is satisfied, send a second movement instruction to the surgical robot to control the surgical robot to move the puncture needle to withdraw the puncture needle.

6. The puncture assistance system according to claim 2, wherein the processor is further configured to:
   in response to receiving a second posture adjustment instruction, send a third movement instruction to the surgical robot to control the surgical robot to adjust a posture of the puncture needle, the second posture adjustment instruction being generated based on a posture adjustment action performed by a user at a surgical robot master hand.

7. The puncture assistance system according to claim 6, wherein, before sending the third movement instruction to the surgical robot, the processor is further configured to:
in response to receiving a needle withdrawal action performed by the user at the surgical robot master hand, send a fourth movement instruction to the surgical robot to control the surgical robot to move the puncture needle to withdraw the puncture needle, the fourth movement instruction being generated based on the needle withdrawal action.

8. The puncture assistance system according to claim 2, wherein the scan image is generated by the processor automatically controlling a medical imaging device to scan the target object; the processor is further configured to:

in response to receiving a signal that the surgical robot is performing the puncture operation, send a scan instruction to the processor to cause the processor to control the medical imaging device to perform scanning;
wherein the puncture operation includes a posture adjustment operation and a needle insertion operation, the posture adjustment operation includes adjusting a posture of the puncture needle, and the needle insertion operation includes adjusting a puncture depth of the puncture needle.

9. The puncture assistance system according to claim 1, wherein the processor is further configured to:

determine one or more deformation parameters based on the preoperative image and the first image; and
determine a target image for the second time period based on the one or more deformation parameters, the preoperative image, and the second image, the target image for the second time period including a target guidance image.

10. The puncture assistance system according to claim 9, wherein the target guidance image includes a first guidance image and/or a second guidance image,

the first guidance image is represented by a scan image of at least one slice related to the second image and the preoperative image, and the second guidance image is represented by a three-dimensional model related to the second image and the preoperative image.

11. The puncture assistance system according to claim 9, wherein the one or more deformation parameters includes a plurality of parameters, each of the deformation parameters corresponds to a respiratory amplitude parameter, the processor is further configured to:

obtain a respiratory amplitude parameter of the target object at a time when the second image is acquired;
perform deformation on the preoperative image based on the deformation parameter corresponding to the respiratory amplitude parameter to obtain a deformed preoperative image; and
determine the first guidance image corresponding to the time when the second image is acquired by performing image fusion of the deformed preoperative image and the second image.

12. The puncture assistance system according to claim 11, wherein the processor is further configured to:

obtain a plurality of first images under different respiratory amplitude parameters;
obtain a plurality of the deformation parameters by performing registration between each of the plurality of first images and the preoperative image.

13. The puncture assistance system according to claim 9, wherein the processor is further configured to:

perform segmentation on the second image to determine a puncture needle image in the second image;
obtain a respiratory amplitude parameter of the target object at a time when the second image is acquired;
perform segmentation and three-dimensional reconstruction on the preoperative image to obtain a three-dimensionally reconstructed result;
perform deformation on the three-dimensionally reconstructed result based on the deformation parameter corresponding to the respiratory amplitude parameter to obtain an initial model corresponding to a deformed preoperative image; and
determine the second guidance image corresponding to the time of the second image based on the puncture needle image and the initial model.

14. The puncture assistance system according to claim 9, wherein the processor is further configured to:
when a respiratory pressure value and/or a respiratory amplitude of the target object satisfies a preset condition, obtain the first image, wherein when the first image is acquired, a respiratory amplitude point of the target object is within a target respiratory amplitude range.

15. A puncture needle position correction system, comprising a positioning correction module configured to:

determine first position information of a puncture needle in an image coordinate system based on positioning position information of the puncture needle in a robot coordinate system, the first position information including a needle tip position and a needle tail position of the puncture needle;

identify the needle tip position in a puncture image based on the first position information to obtain second position information of the puncture needle in the puncture image, the puncture image including a second image and/or a target guidance image;

verify the first position information based on the second position information to obtain a verification result; and determine target position information based on the verification result, the target position information including position information of a first needle tip point.

16. The correction system according to claim 15, wherein the positioning correction module is further configured to:

crop the puncture image based on the first position information to obtain a cropped image;

obtain a segmented image by inputting the cropped image into a segmentation model, the segmentation model being a machine learning model; and

identify the needle tip position in the segmented image to obtain the second position information of the puncture needle in the puncture image.

17. The correction system according to claim 15, wherein the positioning correction module is further configured to:

determine a position distance between a position of the puncture needle located by the surgical robot and a position of the puncture needle in the puncture image based on the first position information and the second position information; and

verify the first position information based on the position distance to obtain the verification result.

18. The correction system according to claim 17, wherein the positioning correction module is further configured to:

in response to determining that the position distance is greater than a preset distance threshold, determine that the verification result indicates that the first position information is inaccurate; and

in response to determining that the position distance is not greater than the preset distance threshold, determine that the verification result indicates that the first position information is accurate.

19. The correction system according to claim 18, wherein the positioning correction module is further configured to:

in response to determining that the first position information is accurate, determine the second position information as the target position information; and

in response to determining that the first position information is inaccurate, determine a cause for inaccuracy of the first position information, and determine a next action of the surgical robot based on the cause.

20. The correction system according to claim 19, wherein the positioning correction module is further configured to:

in response to determining that the cause is a cumulative error of a positioning system of the surgical robot, control the surgical robot to perform recalibration to obtain registration information of an updated robot coordinate system; and control the surgical robot to obtain updated first position information based on the registration information of the updated robot coordinate system.

21. The correction system according to claim 19, wherein the positioning correction module is further configured to:

in response to determining that the cause includes a deformation of the puncture needle, control the surgical robot to perform a needle withdrawal operation.

22. The correction system according to claim 15, wherein the positioning correction module is further configured to:

for the target guidance image, determine whether the puncture needle is completely visible in the target guidance image;

in response to determining that the puncture needle is not completely visible in the target guidance image, determine a time corresponding to the target guidance image, obtain positioning position information of the puncture needle in the robot coordinate system at that time through the surgical robot; and

determine an updated target guidance image by mapping the puncture needle to the target guidance image based on the positioning position information.

23. The correction system according to claim 22, wherein the positioning correction module is further configured to:

for the target guidance image, determine a puncture needle image in the second image corresponding to the target guidance image based on the second image corresponding to the target guidance image; and

determine whether the puncture needle is completely visible in the target guidance image based on the puncture needle image in the second image and a standard puncture needle image.

24. The correction system according to claim 22, wherein the positioning correction module is further configured to:

for the target guidance image, determine the first position information of the puncture needle in the image coordinate system based on the positioning position information of the puncture needle in the robot coordinate system at the time corresponding to the target guidance image;

obtain third position information of the second image corresponding to the target guidance image in the image coordinate system; and

determine whether the puncture needle is completely visible in the target guidance image based on the first position information and the third position information.

25. A puncture assistance device, comprising: a scanning module, a puncture assistance module, and a display module, wherein

the scanning module is configured to obtain a scan image of a target object, the scan image including at least one of a preoperative image, a first image from a first time period, and a second image from a second time period, and the first time period is earlier than the second time period;

the puncture assistance module is configured to generate puncture guidance information based on puncture status information and the scan image, and guide a puncture operation based on the puncture guidance information; and

the display module is configured to display the puncture guidance information.

26. A surgical robot, comprising: a surgical robot master hand, a first processor, a surgical robot slave hand, a puncture needle being disposed on the surgical robot slave hand, the surgical robot master hand being communicatively connected to a puncture assistance device; wherein:

the surgical robot master hand is configured to, after receiving a first puncture operation instruction, send the first puncture operation instruction to the first processor, and send a movement instruction for performing a puncture operation to the puncture assistance device;

the first processor is configured to, after receiving the first puncture operation instruction, send a fifth movement instruction corresponding to the first puncture operation instruction to the surgical robot slave hand;

the puncture assistance device is configured to, after receiving the movement instruction, send a scan instruction to a medical imaging device to cause the medical imaging device to perform medical imaging scanning on a target object; and

the surgical robot slave hand is configured to move the puncture needle according to the fifth movement instruction to perform a puncture operation corresponding to the first puncture operation instruction, the puncture operation including a posture adjustment operation or a needle insertion operation, the posture adjustment operation being used to adjust a posture of the puncture needle, the needle insertion operation being used to adjust a puncture depth of the puncture needle.

27. The surgical robot according to claim 26, wherein:

the surgical robot master hand is configured to, after receiving a second puncture operation instruction, send the second puncture operation instruction to the first processor;

the first processor is configured to determine a sixth movement instruction corresponding to the second puncture operation instruction;

the first processor is configured to send the sixth movement instruction to the surgical robot slave hand, and send a movement instruction for performing a puncture operation to the puncture assistance device;

the puncture assistance device is configured to, after receiving the movement instruction, send a scan instruction to the medical imaging device to cause the medical imaging device to perform medical imaging scanning on the target object;

the surgical robot slave hand is configured to move the puncture needle according to the sixth movement instruction to perform a puncture operation corresponding to the second puncture operation instruction;

the medical imaging scanning is performed before the puncture operation is executed during a time interval between sending the sixth movement instruction to the surgical robot slave hand using the first processor and the surgical robot slave hand executing the sixth movement instruction.

**FIG. 1**

Scanning module210

Puncture assistance module220

Display module230

**FIG. 2**

```
┌─────────────────────┐      ┌─────────────────────┐      ┌─────────────────────┐
│                     │      │  Puncture assistance│      │                     │
│Medical imaging device│      │       device        │      │   Surgical robot    │
│                     │      │                     │      │                     │
└──────────┬──────────┘      └──────────┬──────────┘      └──────────┬──────────┘
           │                            │                            │
┌──────────┴──────────────┐            │                            │
│ S301, a scan image may be │            │                            │
│ acquired by a medical     │            │                            │
│ imaging device performing │            │                            │
│ a scanning operation on a │            │                            │
│ target object.            │            │                            │
└───────────────────────────┘            │                            │
           │                            │                            │
     S302, the medical imaging          │                            │
     device sends the scan image        │                            │
     to a puncture assistance           │                            │
     device.                            │                            │
           │──────────────────────────▶│                            │
           │                            │   S303, the surgical robot │
           │                            │   obtains puncture status  │
           │                            │   information and sends the │
           │                            │   puncture status info to   │
           │                            │   the puncture assistance   │
           │                            │   device.                   │
           │                            │◀───────────────────────────│
```

S301, a scan image may be acquired by a medical imaging device performing a scanning operation on a target object.

S302, the medical imaging device sends the scan image to a puncture assistance device.

S303, the surgical robot obtains puncture status information and sends the puncture status information to the puncture assistance device.

S304, determine position information of a first needle tip point of a puncture needle and position information of a predicted target point based on the puncture status information.

S305, generate an annotated image based on the position information of the first needle tip point and the position information of the predicted target point.

S306, display the annotated image.

**FIG. 3**

S401

| Target organ segmentation | Tumor site extraction | Dangerous tissue identification |

Perform preoperative image scanning.

S402

Perform initial puncture path planning.

S403

Perform guiding and positioning.

S404

Perform puncture preparing.

S405

| Needle tract posture adjustment | Needle tract insertion | Real-time exposure |

Perform intraoperative puncture.

S406

Complete puncture.

**FIG. 4**

501

**FIG. 5**

601

603

602

604

**FIG. 6**

**FIG. 7**

**FIG. 8**

FIG. 9

FIG. 10

**FIG. 11**

**1200**

Determine one or more deformation parameters based on a preoperative image and a first image. ⟶ 1210

Determine a target image for the second time period based on the one or more deformation parameters, the preoperative image, and the at least one second image. ⟶ 1220

**FIG. 12**

**FIG. 13**

**FIG. 14**

| Scanning | Reconstruction | Image registration | Image transmission | | |

FIG. 15

FIG. 16

1700

Obtain a respiratory amplitude parameter of a target object at a time when a second image is acquired. — 1710

Perform deformation on a preoperative image based on a deformation parameter corresponding to the respiratory amplitude parameter to obtain a deformed preoperative image. — 1720

Determine the first guidance image corresponding to the time when the second image is acquired by performing image fusion of the deformed preoperative image and the second image. — 1730

**FIG. 17**

1800

For the target guidance image, determine whether the puncture needle is completely visible in the target guidance image. — 1810

In response to a determination that the puncture needle is not completely visible in the target guidance image, determine a time corresponding to the target guidance image, and obtain the positioning position information of the puncture needle in the robot coordinate system at the time through the surgical robot. — 1820

Determine an updated target guidance image by mapping the puncture needle to the target guidance image based on the positioning position information. — 1830

**FIG. 18**

**FIG. 19**

**2000**

2010

2020

**FIG. 20**

**2100**

**FIG. 21**

**2200**

| Determine first position information of a puncture needle in an image coordinate system based on positioning position information of the puncture needle in a robot coordinate system. | S2201 |

| Identify the needle tip position in a puncture image based on the first position information to obtain second position information of the puncture needle in the puncture image. | S2202 |

| Verify the first position information based on the second position information to obtain a verification result, and determine target position information based on the verification result. | S2203 |

**FIG. 22**

Crop the puncture image based on the first position information to obtain a cropped image. — S2301

Obtain a segmented image by inputting the cropped image into a segmentation model. — S2302

Identify the needle tip position in the segmented image to obtain second position information of the puncture needle in the puncture image. — S2303

**FIG. 23**

Determine an end coordinate of a last robotic arm in a surgical robot based on a length and a rotation angle of each robotic arm in the surgical robot. — S2401

Determine a position coordinate of the puncture needle in the robot coordinate system based on the end coordinate of the last robotic arm and a size of the puncture needle. — S2402

Determine the first position information of the puncture needle in the image coordinate system based on the position coordinate of the puncture needle in the robot coordinate system and the registration information of coordinate systems. — S2403

**FIG. 24**

Determine a position distance between a position of the puncture needle located by the surgical robot and a position of the puncture needle in the puncture image based on the first position information and the second position information.　　S2501

Verify the first position information based on the position distance to obtain the verification result.　　S2502

**FIG. 25**

S2601

Verify the first position information based on the position distance to obtain the verification result.

In response to determining that the position distance is not greater than the preset distance threshold

In response to determining that the position distance is not greater than the preset distance threshold　　S2602

S2603

Determine that the verification result indicates that the first position information is accurate.

Determine that the verification result indicates that the first position information is inaccurate.

**FIG. 26**

Determine an end coordinate of a last robotic arm in a surgical robot based on a length and a rotation angle of each robotic arm in the surgical robot. — S2701

Determine a coordinate of a puncture needle in a robot coordinate system based on the end coordinate the last robotic arm and a size of the puncture needle. — S2702

Determine first position information of the puncture needle in an image coordinate system based on the coordinate of the puncture needle in the robot coordinate system and coordinate system registration information. — S2703

Obtain a puncture image. — S2704

Crop the puncture image based on the first position information to obtain a cropped image. — S2705

Input the cropped image into a segmentation model for segmentation to obtain a segmented image. — S2706

Adjust the size of the segmented image to the size of the original puncture image to obtain an adjusted segmented image. — S2707

Identify a needle tip position in the adjusted puncture image based on the first position information to obtain second position information of the puncture needle in the puncture image. — S2708

Determine a position distance between the puncture needle positioned by the surgical robot and the puncture needle in the puncture image based on the first position information and the second position information. — S2709

Verify the first position information based on the position distance to obtain a verification result. — S2710

The position distance is not greater than the preset distance threshold.

The position distance is greater than a preset distance threshold.

Determining that the verification result indicates that the first position information is accurate, determine the second position information as target position information, and continue the puncture based on the target position information. — S2716

Determining that the verification result indicates that the first position information is inaccurate, and further determine whether a cause for the inaccuracy of the first position information is a cumulative error of a positioning system of the surgical robot. — S2711

Yes

No

The surgical robot performs a recalibration to obtain new coordinate system registration information, so that the surgical robot obtains next first position information based on the new coordinate system registration information. — S2712

Determine whether the cause for the inaccuracy of the first position information is a deformation of the puncture needle. — S2713

Yes

No

The surgical robot performs a needle withdrawal operation. — S2714

Display prompt information to inform a user of a puncture risk and to stop the puncture. — S2715

**FIG. 27**

2801

2800

2803

**Memory**

2802

**Puncture assistance module**

**Display module**

**Computer program**

**Scanning module**

2804

**Puncture assistance device**

**FIG. 28**

2901

2900

2903

**Memory**

2902

**Processor**

**Surgical robot master hand**

2904

**Computer program**

**Surgical robot slave hand**

**Surgical robot**

**FIG. 29**

```
                    3002                              3001
    ┌──────────────────────┐        ┌──────────────────────┐
    │                      │        │                      │
    │ Medical imaging device│──────▶│   Surgical robot     │
    │                      │        │                      │
    └──────────────────────┘        └──────────────────────┘

                      Correction device
```

**FIG. 30**

```
    ┌──────────────────┐    ┌──────────────────┐    ┌──────────────────┐
    │ Obtaining    3110│    │ Identification 3120│   │ Verification 3130│
    │ module           │────│ module           │────│ module           │
    └──────────────────┘    └──────────────────┘    └──────────────────┘

              Puncture needle position correction device
```

**FIG. 31**

```
    ┌──────────┐   ┌──────┐┌──────────┐┌──────────┐   ┌──────────────┐
    │Obtaining │   │Cropping││segmentat-││identificat-│ │ Verification │
    │module 3110│──│unit    ││ion unit  ││ion unit   │─│ module   3130│
    │          │   │        ││          ││           │ │              │
    │          │   │ 3121   ││ 3122     ││ 3123      │ │              │
    └──────────┘   └──────┘└──────────┘└──────────┘   └──────────────┘
                        Identification module 3120

              Puncture needle position correction device
```

**FIG. 32**

```
    ┌──────┐┌──────┐┌──────┐   ┌──────────────┐   ┌──────────────┐
    │First ││Second││Third │   │Identification │   │ Verification │
    │determin││determin││determin│ │module     3120│   │ module   3130│
    │-ation ││-ation ││-ation │   │              │   │              │
    │unit   ││unit   ││unit   │   │              │   │              │
    │3111   ││3112   ││3113   │   │              │   │              │
    └──────┘└──────┘└──────┘   └──────────────┘   └──────────────┘
      Obtaining module    3110

              Puncture needle position correction device
```

**FIG. 33**

| Obtaining module | 3110 | | Identification module | 3120 | | Fourth determin-ation unit | Verifica-tion unit |
|---|---|---|---|---|---|---|---|

Fourth determin-ation unit 3131

Verifica-tion unit 3132

Verification module 3130

Puncture needle position correction device

**FIG. 34**

Operating system

Computer program

Non-volatile storage medium

Processor

Internal memory

System bus

I/O interface

Communication interface

Input device

Display module

Computer device

**FIG. 35**

## INTERNATIONAL SEARCH REPORT

| International application No. |
| --- |
| **PCT/CN2024/099054** |

**A. CLASSIFICATION OF SUBJECT MATTER**

A61B 34/20(2016.01)i; A61B 17/34(2006.01)i

According to International Patent Classification (IPC) or to both national classification and IPC

**B. FIELDS SEARCHED**

Minimum documentation searched (classification system followed by classification symbols)

IPC: A61B 34/-; A61B 17/-

Documentation searched other than minimum documentation to the extent that such documents are included in the fields searched

Electronic data base consulted during the international search (name of data base and, where practicable, search terms used)

CNABS, CNTXT, DWPI, WPABSC, ENTXT, ENTXTC: 武汉联影, 手术, 介入, 穿刺, 针, 影像, 图像, 扫描, 引导, 路径, 规划, 轨迹, 针路, 针道, 轮廓, 报警, 警告, 提示, 机器人, 机械手, 机器臂, 机器手, 定位, 位置, 坐标系, 校正, 校准, 校验, 标定, 配准, 主从, 远程, 调整, 调姿, tomography, CT, image, master, slave, puncture, path, guiding, planning, robot+, manipulat+, adjust+

**C. DOCUMENTS CONSIDERED TO BE RELEVANT**

| Category* | Citation of document, with indication, where appropriate, of the relevant passages | Relevant to claim No. |
| --- | --- | --- |
| X | WO 2022206407 A1 (JEDICARE MEDICAL CO., LTD.) 06 October 2022 (2022-10-06) description, pages 5-15 | 1, 25 |
| Y | WO 2022206407 A1 (JEDICARE MEDICAL CO., LTD.) 06 October 2022 (2022-10-06) description, pages 5-15 | 2, 4-10, 14 |
| X | CN 112336432 A (YISHENGXIN TECHNOLOGY (BEIJING) CO., LTD.) 09 February 2021 (2021-02-09) description, paragraphs 39-63, and figure 1 | 26-27 |
| Y | CN 112336432 A (YISHENGXIN TECHNOLOGY (BEIJING) CO., LTD.) 09 February 2021 (2021-02-09) description, paragraphs 39-63, and figure 1 | 2, 4-8 |
| Y | CN 106236258 A (BEIJING BAIHUI WEIKANG MEDICAL ROBOT TECHNOLOGY CO., LTD.) 21 December 2016 (2016-12-21) description, paragraphs 51-55 | 9-10, 14 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 August 2024** | **21 August 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**EP 4 710 885 A1**

## INTERNATIONAL SEARCH REPORT

| International application No. |
| --- |
| **PCT/CN2024/099054** |

### C. DOCUMENTS CONSIDERED TO BE RELEVANT

| Category* | Citation of document, with indication, where appropriate, of the relevant passages | Relevant to claim No. |
| --- | --- | --- |
| X | CN 113057734 A (SHANGHAI MICROPORT MEDICAL ROBOT (GROUP) CO., LTD.) 02 July 2021 (2021-07-02)<br>description, paragraphs 106-178 | 1,25 |
| A | CN 111012506 A (HARBIN INSTITUTE OF TECHNOLOGY) 17 April 2020 (2020-04-17)<br>description, paragraphs 51-90 | 15-24 |
| A | CN 112163987 A (SUZHOU INSTITUTE OF BIOMEDICAL ENGINEERING AND TECHNOLOGY, CHINESE ACADEMY OF SCIENCES et al.) 01 January 2021 (2021-01-01)<br>entire document | 1-27 |
| A | US 2022378524 A1 (THE REGENTS OF THE UNIVERSITY OF CALIFORNIA) 01 December 2022 (2022-12-01)<br>entire document | 1-27 |

Form PCT/ISA/210 (second sheet) (July 2022)

72

**INTERNATIONAL SEARCH REPORT**
Information on patent family members

International application No.

**PCT/CN2024/099054**

| Patent document cited in search report | | | Publication date (day/month/year) | Patent family member(s) | Publication date (day/month/year) |
|---|---|---|---|---|---|
| WO | 2022206407 | A1 | 06 October 2022 | None | |
| CN | 112336432 | A | 09 February 2021 | None | |
| CN | 106236258 | A | 21 December 2016 | None | |
| CN | 113057734 | A | 02 July 2021 | None | |
| CN | 111012506 | A | 17 April 2020 | None | |
| CN | 112163987 | A | 01 January 2021 | None | |
| US | 2022378524 | A1 | 01 December 2022 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310964477
- CN 202310707754X

- CN 202311421935